# EUROPEAN PATENT SPECIFICATION

(11) **EP 1 625 112 B1**
(45) Date of publication and mention of the grant of the patent: **22.07.2009**
(21) Application number: 04731133.7
(22) Date of filing: 05.05.2004
(51) Int. Cl.: C07C 317/46, C07D 213/70, C07D 277/32, C07D 295/26, C07D 307/28, C07D 307/38, C07D 311/16, C07D 333/34, A61K 31/10, A61P 3/04

(54) **NOVEL COMPOUNDS FOR TREATMENT OF OBESITY**
VERBINDUNGEN ZUR BEHANDLUNG VON OBESITAS
NOUVEAUX COMPOSES POUR LE TRAITEMENT DE L'OBESITE

(30) Priority: 14.05.2003 DK 200300734; 31.10.2003 US 516588 P; 31.10.2003 DK 200301618
(43) Date of publication of application: 15.02.2006
(73) Proprietor: High Point Pharmaceuticals, LLC, High Point, NC 27265 (US)
(72) Inventor: TAGMOSE, Tina, Moeller, DK-2750 Ballerup (DK); OLESEN, Preben, Houlberg, DK-2400 Copenhagen NV (DK); HANSEN, Thomas, Kruse, DK-2730 Herlev (DK)
(74) Representative: Bailey, Lindsey
(86) International application number: PCT/DK2004/000307
(87) International publication number: WO 2004/101505

(56) References cited:
- WO-A-96/40629
- US-A- 3 984 357
- US-A- 5 240 962
- US-A- 5 789 427
- I. KATSUMI, ET AL.: "Studies on styrene derivatives. II. Synthesis and antiinflammatory activity of 3,5-di-tert-butyl-4-hydroxystyrenes" CHEMICAL AND PHARMACEUTICAL BULLETIN., vol. 34, no. 4, 1986, pages 1619-1627, XP002051043 PHARMACEUTICAL SOCIETY OF JAPAN, TOKYO, JP ISSN: 0009-2363
- J. TRÖGER, ET AL.: "Weitere Beitrage zur Kentnis der arylsulfonierten Acetonitrile. Über einige aus arylsulfonierten Acetonitrilen und aromatischen Aldehyden gebildete Kondensationsprodukte" ARCHIV DER PHARMAZIE, vol. 247, 1909, pages 613-617, XP008032991 VCH VERLAGSGESELLSCHAFT, WEINHEIM, DE
- B.R. BAKER, ET AL.: "Sulphones. III. 4-Aminophenyl alkyl sulphones" JOURNAL OF ORGANIC CHEMISTRY, vol. 15, no. 2, March 1950 (1950-03), pages 417-424, XP002288830 AMERICAN CHEMICAL SOCIETY, WASHINGTON, DC, US
- PATENT ABSTRACTS OF JAPAN vol. 0110, no. 60 (C-405), 24 February 1987 (1987-02-24) & JP 61 218558 A (KANEGAFUCHI CHEMICAL INDUSTRIES), 29 September 1986 (1986-09-29) & JP 61 218558 A (KANEGAFUCHI CHEMICAL INDUSTRIES) 29 September 1986 (1986-09-29)
- H. MIYOSHI, ET AL.: "Quantitative analyses of uncoupling activity of SF6847 (2,6-di-t-butyl-4-(2,2-dicyanovinyl)phenol ) and its analogues with spinach chloroplasts" BIOCHIMICA ET BIOPHYSICA ACTA, BIOENERGETICS, vol. 894, 1987, pages 339-345, XP002291974 ELSEVIER SCIENCE PUBLISHERS, AMSTERDAM, NL

## Description

### FIELD OF THE INVENTION

The invention relates to novel hydroxyl styrene sulfonyl derivatives, to their use in therapy, to pharmaceutical compositions comprising said derivatives, to the use of said derivatives in the manufacture of medicaments, and to therapeutic methods comprising the administration of said derivatives to a patient The compounds exert an uncoupling activity and are useful in the treatment of obesity.

### BACKGROUND OF THE INVENTION

Obesity is a well-known risk factor for the development of many very common diseases such as atherosclerosis, hypertension, type 2 diabetes (non-insulin dependent diabetes mellitus (NIDDM)), dyslipidemia, coronary heart disease, and osteoarthritis and various malignancies. It also causes considerable problems through reduced motility and decreased quality of life. The incidence of obese people and thereby also these diseases is increasing throughout the entire industrialised world.

The term obesity implies an excess of adipose tissue. In this context obesity is best viewed as any degree of excess adiposity that imparts a health risk. The cut off between normal and obese individuals can only be approximated, but the health risk imparted by the obesity is probably a continuum with increasing adiposity. In the context of the present invention, individuals with a body mass index (BMI = body weight in kilograms divided by the square of the height in meters) above 25 are to be regarded as obese

Even mild obesity increases the risk for premature death and conditions such as diabetes, dyslipidemia, hypertension, atherosclerosis, gallbladder disease and certain types of cancer. In the industrialised western world the prevalence of obesity has increased significantly in the past few decades. Because of the high prevalence of obesity and its health consequences, its prevention and treatment should be a high public health priority.

Except for exercise, diet and food restriction, which is not feasible for a vast number of patients, no convincing treatment for reducing body weight effectively and acceptably currently exist. However, not only in view of the considerable problems directly related to obesity as described above, but also due to the important effect of obesity as a risk factor in serious and even mortal and common diseases, it is important to find pharmaceutical compounds which are useful in prevention and/or treatment of obesity.

When energy intake exceeds expenditure, the excess calories are stored predominately in adipose tissue, and if this net positive balance is prolonged, obesity results, i.e. there are two components to weight balance, and an abnormality on either side (intake or expenditure) can lead to obesity. This process may be counteracted by increasing the energy expenditure (for instance via exercise) or decreasing the energy intake (for instance by dieting). Pharmacological treatment available up to date only consists of Sibutramine (acting via serotonergic mechanisms, Abbott) and Orlistal (reducing fat uptake from the gut, Roche Pharm) neither reducing body weight effectively nor acceptably. There is therefore a need for pharmaceutical compounds which may be useful in prevention and/or treatment of obesity, for instance by increasing the energy expenditure or decreasing the energy intake.

One way of increasing energy expenditure is by increasing the metabolic rate. Oxidative phosphorylation in mitochondria, the energy from glucose metabolism and free fatty acids oxidation is used to drive the phosphorylation of ADP to ATP. When NADH and FADH₂ formed in the TCA cycle are oxidised back to NAD⁺ and FAD respectively, protons are pumped out of the mitochondrial matrix. The resulting pH gradient (matrix pH-8 and outside pH-7) and potential (∼-170 mV, inside negative) across the inner mitochondrial membrane constitute the electrochemical proton gradient. As the effect of a one-unit pH difference corresponds to a potential of 61.5mV, the electrochemical proton gradient exerts a proton-motive force of roughly -230 mV, which is the driving force for the mitochondrial ATP synthesis.

When the ATP consumption thus increases, the cells respond by increasing the ATP synthesis and consequently the inward flux of protons through the ATP synthase, the enzyme responsible for ATP synthesis and thereby the metabolic rate is increased. Chemical uncouplers are compounds, which can transport protons across membranes, and when protons are transported across the inner mitochondrial membrane, the ATP synthase is by-passed. At the (alkaline) matrix side the proton is released and the deprotonated uncoupler returns to the inter-membrane space where it picks up another proton. The cycling of the uncoupler (or ATP synthesis) and the resulting proton transport leads to an increased outward pumping of protons through an increased oxidation of NADH and FADH₂ by the respiration chain. The NADH concentration in the matrix will consequently drop. Since NADH feed-back inhibits three steps in the TCA cycle (NADH is the main regulator of the TCA cycle), the flux through the TCA cycle will increase. Hence, the metabolic rate will increase.

Compounds, such as chemical uncouplers, which act by increasing the metabolic rate may thus be useful for treating obesity, but also for treating other conditions such as atherosclerosis, hypertension, diabetes, especially type 2 diabetes (NIDDM (non-insulin dependent diabetes mellitus)), dyslipidemia, coronary heart disease, gallbladder disease, osteoarthritis and various types of cancer such as endometrial, breast, prostate and colon cancers and the risk for premature death as well as other conditions, such as diseases and disorders, which conditions are improved by a reduced mitochondrial potential.

Furthermore, chemical uncouplers may reduce reactive oxygen species (ROS) that are assumed (De Grey, Eur J. Biochem 269, 1995 ff (2002)) to be involved in the aging process, in damage of heart tissue as well as neuronai tissue. It is therefore also possible that conditions affected by ROS may be reversed or halted by intervention by chemical uncouplers. Examples of such conditions include diabetic microvascular diseases in the retina, renal glomerulus and peripheral nerves cell.

The best known chemical uncoupler is 2,4-dinitrophenol (DNP), which has been shown to increase energy expenditure in humans as well as animals. The side effects at higher doses include increased perspiration, vasodilatation, skin rashes, cataracts, neuritis and death! Two fatalities amongst the first 100.000 persons treated with DNP, and the fact that the lowest dose, which could be lethal, was only twice the average dose giving a desired 50% increase in basal metabolic rate giving a very narrow safety window combined with other factors led to the removal of DNP from the market. Since then nobody have attempted to develop or market uncouplers for the treatment of obesity.

DNP is the best known chemical uncoupler; but many other compounds are known to induce uncoupling. DNP derivatives such as 4,6-dinitro-o-cresol (Victoria Yellow) and 2,4-dinitro-1-naphtol (Martius Yellow) as well as structurally unrelated compounds such as 2,6-di*t*-butyl-4-(2',2'-dicyanovinyl)phenol) (SF6847) (also known as 2-(3,5-di-tert-butyl-4-hydroxybenzylidene)-malononitrile), carbonylcyanide m-chlorophenylhydrazone (CCCP) and carbonylcyanide ptrifluoromethoxy-phenylhydrazone (FCCP) (Miyoshi H et al. Quantitative relationship between protonophoric and uncoupling activities of analogs of SF6847 (2,6-di-t-butyl-4-(2',2'-dicyanovinyl)phenol), Biochimica et Biophysica Acta 891, 293-299 (1987)) are uncouplers.

Another class of chemical uncouplers is the salicylanilides of which S-13 is the most potent compound discovered so far (Terada H et al. Structural Requirements of Salicylanilides for Uncoupling Activity in Mitochondria Quantitative Analysis of Structure- Uncoupling Relationships, Biochimica et Biophysica Acta 936, 504-512 (1988)).

WO00/06143 to Texas Pharmaceuticals Inc. relates to a method for inducing intracellular hyperthermia comprising a step of administering a mitochondrial uncoupling agent, such as 2,4-dinitrophenol.

US 4,673,691 to Bachynsky relates to the use of 2,4-dinitrophenol for treating obesity.

Hydroxy styrene sulfonyl derivatives have been reported to exert a variety of activities. As examples, US 5,792,771 and WO 95/24190 disclose hydroxy styrene sulfonyl derivatives as modulators of tyrosine kinase signal transduction, and WO 86/40629 and US 5,789,427 disclose hydroxy styrene sulfonyl derivatives as useful in the treatment of cell proliferative disorders.

US 5,240,962 A relates to the use of certain substituted phenol derivatives for the treatment of obesity.

### SUMMARY OF THE INVENTION

The present inventors have surprisingly found that compounds of formula I are potent chemical uncouplers. Accordingly, the invention relates to use of compounds of formula I 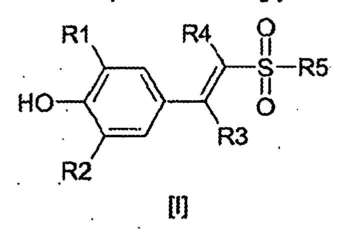
wherein R1 and R2 independently represent hydrogen, nitro, cyano, halogen, alkyl, alkenyl, alkynyl, aryl, heteroaryl, haloalkyl, alkoxy, alkylamino, -C(O)OR6, -S(O)₂OR6,-S(O)ₙR6, -OC(O)R6, -NHC(O)R6 or -N(C(O)R6)₂;
R3 represents hydrogen, nitro, cyano, halogen, alkyl, alkenyl, alkynyl, alkoxy ,alkylamino, -C(O)OR6, -S(O)₂OR6, -S(O)ₙR6, -OC(O)R6, -NHC(O)R6 or -N(C(O)R6)₂;
R4 represents nitro, cyano, halogen, haloalkyl, -C(O)R6, -C(O)OR6, -C(O)N(R6)₂, -S(O)₂OR6, S(O)ₙR6, S(O)₂N(R6)₂, -P(O)(OR6)₂ or -B(OR6)₂;
R6 represents hydrogen or alkyl, aryl or heteroaryl, all of which may be substituted with one or more substituent selected from amongst hydroxyl, halogen, nitro and cyano;
n represents 0, 1 or 2;
R5 represents alkyl, alkenyl, alkynyl, cycloalkyl, aryl, heteroaryl, heterocyclyl, all of which are optionally substituted with one or more substituents selected from the list consisting of alkyl, alkenyl, alkynyl, phenyl, heteroaryl, heterocyclyl, halogen, hydroxyl, cyano, nitro, carboxyl, haloalkyl, -O-R7, -S(O)ₙR7, -O-C(O)R7, -C(O)-O-R7, -C(O)-R7, -C(O)-N(R7)(R8), - N(R7)(R8), -(CH₂)ₚ-N(R8)-C(O)-R7, -B(OR7)(OR8), -(CH₂)ₚ-O-R7, -NR7-C(O)R7, NR7-S(O)ₙR7 and -(CH₂)ₚ-N(R7)(R8), said alkyl, alkenyl, alkynyl, phenyl, heteroaryl, heterocyclyl being optionally substituted with one or more substituents selected from the list consisting of alkyl, halogen, haloalkyl, hydroxyalkyl, cyano, nitro, O-R13, -S(O)ₙR11, -O-C(O)R11, -C(O)-O-R11, -C(O)-R11 -C(O)-N(R11)(R12), -N(R11)(R12), -(CH₂)ₚ-N(R11)-C(O)-R12,-B(OR11)(OR12), -(CH₂)ₚ-O-R11, -(CH₂)ₚ-N(R11)(R12);
R7 and R8 independently represent hydrogen, haloalkyl, hydroxyalkyl, alkyl, alkenyl, alkynyl, dialkylether radical, cycloalkyl, heterocyclyl or phenyl, wherein said phenyl and heterocyclyl are optionally substituted with one or more substituents selected from the list consisting of alkyl, halogen, haloalkyl, hydroxyl, hydroxyalkyl, cyano, nitro, -N(R9)(R10) and -(CH₂)ₚ-N(R9)(R10); or R7 and R8 when bound to a nitrogen together with said nitrogen form a 5-8 membered heterocycle which may be further substituted by alkyl;
R9 and R10 independently represent hydrogen, alkyl, alkenyl, alkynyl, haloalkyl, hydroxyalkyl or cycloalkyl;
or R4 and R5 together with the atoms to which they are attached constitute a 5, 6, 7 or 8 membered ring, which may be saturated, either partly or fully or unsaturated, and wherein said ring is optionally substituted with one or more substituents selected from the list consisting of alkyl, halogen, hydroxyl, cyano and nitro;
each R11 and R12 independently represent hydrogen, haloalkyl, hydroxyalkyl, alkyl, alkenyl, alkynyl, cycloalkyl or phenyl;
R13 represents haloalkyl, hydroxyalkyl, alkyl, alkenyl, alkynyl, cycloalkyl;
p represents 0, 1 or 2;
and pharmaceutically acceptable salts, solvates, and hydrates thereof; optionally in combination with another therapeutically active compound; in the manufacture of a medicament for: treatment of obesity, type 2 diabetes, dyslipidemia; hypertension or gallbladder diseases; prevention of weight gain; or maintenance of weight loss.

### DEFINITIONS

In the present context, the term "alkyl" is intended to indicate a straight or branched chain saturated monovalent hydrocarbon radical having from one to twelve carbon atoms, also denoted as C₁₋₁₂-alkyl. Typical alkyl groups are alkyl groups with from one to eight or from one to six carbon atoms, also denoted as C₁₋₈-alkyl and C₁₋₆-alkyl respectively. Typical C₁₋₆-alkyl groups include, but are not limited to e.g. methyl, ethyl, n-propyl, isopropyl, n-butyl, sec-butyl, isobutyl, tert-butyl, n-pentyl, 2-methylbutyl, 3-methylbutyl, 4-methylpentyl, n-pentyl, n-hexyl, 1,1-dimethylpropyl, 1,2-dimethylpropyl, 2,2-dimethylpropyl (neopentyl), 1,2,2-trimethylpropyl and the like, while typical C₁₋₈-alkyl groups include the same groups as well as alkyl groups having seven or eight carbon atoms, such as heptyl, octyl, 2,2-dimethylhexyl and the like. The term "C₁₋₆-alkyl" as used herein also includes secondary C₃₋₆-alkyl and tertiary C₄₋₆-alkyl. The term "C₁₋₈-alkyl" as used herein also includes secondary C₃₋₈-alkyl and tertiary C₄₋₈-alkyl. The term "C₁₋₁₂-alkyl" as used herein also includes secondary C₃₋₁₂-alkyl and tertiary C₄₋₁₂-alkyl.

In the present context, the term "alkenyl" is intended to indicate a straight or branched chain monovalent hydrocarbon radical having from two to six carbon atoms and at least one carbon-carbon double bond, for example C₃₋₅-alkenyl. Typical C₃₋₅-alkenyl groups include vinyl, allyl, 1-propenyl, 1,3 butadiene-1-yl, and the like. The term "conjugated alkenyl" as used herein, alone or in combination, refers to an alkenyl having consecutive double bonds, such as for instance 1,3 butadiene-1-yl.

In the present context, the term "alkynyl" is intended to indicate a straight or branched chain monovalent hydrocarbon radical having from two to six carbon atoms and at least one carbon-carbon triple bond and optionally one or more carbon-carbon double bonds. Examples include ethynyl, propynyl and 3,4-pentadiene-1-ynyl.

The term "halogen" is intended to indicate members of the seventh main group of the periodic system, i.e. fluoro, chloro, bromo and iodo.

In the present context, the term "dialkylether" is intended to indicate a compound of the formula R'-O-R', wherein each R' independently represent alkyl as indicated above. Examples of dialkylether include dimethyl-ether, methylethyl-ether and diethylether.

In the present context, the term "aryl" is intended to indicate a carbocyclic aromatic ring radical or a fused aromatic ring system radical wherein at least one of the rings are aromatic. Typical aryl groups include phenyl, biphenylyl, indenyl, fluorene, naphthyl (1-naphthyl, 2-naphthyl), anthracenyl (1-anthracenyl, 2-anthracenyl, 3-anthracenyl).

The term "heteroaryl", as used herein, alone or in combination, refers to an aromatic ring radical with for instance 5 to 7 member atoms, or to a fused aromatic ring system radical with for instance from 7 to 18 member atoms, wherein at least on ring is aromatic, containing one or more heteroatoms selected from nitrogen, oxygen, or sulfur heteroatoms, wherein N-oxides and sulfur monoxides and sulfur dioxides are permissible heteroaromatic substitutions. Examples include furanyl, thienyl, thiophenyl, pyrrolyl, imidazolyl, pyrazolyl, triazolyl, tetrazolyl, thiazolyl, oxazolyl, isoxazolyl, oxadiazolyl, thiadiazolyl, isothiazolyl, pyridinyl, pyridazinyl, pyrazinyl, pyrimidinyl, quinolinyl, isoquinolinyl, benzofuranyl, benzothiophenyl, indolyl, indazolyl, thienyl (2-thienyl, 3-thienyl), furanyl (2-furanyl, 3-furanyl), indolyl, oxadiazolyl, isoxazolyl, thiadiazolyl, oxatriazolyl, thiatriazolyl, quinazolinyl, fluorenyl, xanthenyl, isoindanyl, benzhydryl, acridinyl, thiazolyl, pyrrolyl (1-pyrrolyl, 2-pyrrolyl, 3-pyrrolyl), pyrazolyl (1-pyrazolyl, 3-pyrazolyl, 4-pyrazolyl, 5-pyrazolyl), imidazolyl (1-imidazolyl, 2-imidazolyl, 4-imidazolyl, 5-imidazolyl), triazolyl (1,2,3-triazol-1-yl, 1,2,3-triazol-4-yl 1,2,3-triazol-5-yl, 1,2,4-triazol-3-yl, 1,2,4-triazol-5-yl), oxazolyl (2-oxazolyl, 4-oxazolyl, 5-oxazolyl), isoxazolyl (isoxazo-3-yl, isoxazo-4-yl, isoxaz-5-yl), isothiazolyl (isothiazo-3-yl, isothiazo-4-yl, isothiaz-5-yl) thiazolyl (2-thiazolyl, 4-thiazolyl, 5-thiazolyl), pyridinyl (2-pyridinyl, 3-pyridinyl, 4-pyridinyl), pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolinyl (2-quinolinyl, 3-quinolinyl, 4-quinolinyl, 5-quinolinyl, 6-quinolinyl, 7-quinolinyl, 8-quinolinyl), isoquinolinyl (1-isoquinolinyl, 3-isoquinolinyl, 4-isoquinolinyl, 5-isoquinolinyl, 6-isoquinolinyl, 7-isoquinolinyl, 8-isoquinolinyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydrobenzo[b]furanyl (2-(2,3-dihydrobenzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydro-benzo[b]furanyl)), benzo[b]thiophenyl (benzo[b]thiophen-2-yl, benzo[b]thiophen-3-yl, benzo[b]thiophen-4-yl, benzo[b]thiophen-5-yl, benzo[b]thiophen-6-yl, benzo[b]thiophen-7-yl), 2,3-dihydrobenzo[b]thiophenyl (2,3-dihydro-benzo[b]thiophen-2-yl, 2,3-dihydrobenzo[b]thiophen-3-yl, 2,3-dihydro-benzo[b]thiophen-4-yl, 2,3-dihydro-benzo[b]thiophen-5-yl, 2,3-dihydrobenzo[b]thiophen-6-yl, 2,3-dihydro-benzo[b]thiophen-7-yl), indolyl (1-indolyl, 2-indolyl, 3-indolyl, 4-indolyl, 5-indolyl, 6-indolyl, 7-indolyl), indazolyl (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (2-benzoxazolyl, 3-benzoxazolyl, 4-benzoxazolyl, 5-benzoxazolyl, 6-benzoxazolyl, 7-benzoxazolyl), benzothiazolyl (2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepinyl (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepinyl (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz[b,f]azepine-4-yl, 10,11-dihydro-5H-dibenz[b,f]azepine-5-yl), benzo[1,3]dioxole (2-benzo[1,3]dioxole, 4-benzo[1,3]dioxole, 5-benzo[1,3]dioxole, 6-benzo[1,3]dioxole, 7-benzo[1,3]dioxole), and tetrazolyl (5-tetrazolyl, N-tetrazolyl).

The term "fused aromatic ring system" as used herein, alone or in combination, refers to a carbocyclic aromatic ring radical fused to another carbocyclic or heterocyclic ring radical, the two rings having two atoms in common. Typical fused aromatic ring systems include, but are not limited to napthalene, quinoline, isoquinoline, indole, and isoindole.

In the present context the term "cycloalkyl" is intended to indicate a cyclic saturated monovalent hydrocarbon radical having 3, 4, 5, 6, 7 or 8 ring carbon atoms.

The term "hetorocyclyl" is intended to indicate a cyclic non-aromatic radical having 5, 6, 7 or 8 ring atoms, wherein at least one ring atom is selected from the group consisting of nitrogen, oxygen and sulfur heteroatoms, wherein N-oxides and sulfur monoxides and sulfur dioxides are permissible heterocyclic substitutions. Examples of heterocycles include tetrahydrofuran, 1,4-dioxane, 1,3-dioxane, piperidine, pyrrolidine, morpholine and piperazine.

In the present context, the term "alkoxy" is intended to indicate a radical of the formula -OR', wherein R' represents alkyl as indicated above.

In the present context, the term "alkylamino" is intended to indicate a radical of the formula -NH-R' or -N(R')₂, wherein each R' represents alkyl as indicated above.

The term "nitro" shall mean the radical -NO₂.

The term "cyano" shall mean the radical -CN.

In the present context, the term "haloalkyl" is intended to indicate an alkyl, as defined above, substituted with one or more halogens, as defined above. Examples include trihalomethyl, such as trifluoromethyl and trichloromethyl, and 2,2,2-trichloro-1-ethyl.

In the present context, the term "hydroxyalkyl" is intended to indicate an alkyl, as defined above, substituted with one or more hydroxyl groups. Examples include hydroxymethyl, 1-hydoxy-1-ethyl and 2-hydroxy-1-ethyl.

As used herein, the term "solvate" is a complex of defined stoichiometry formed by a solute (in casu, a compound according to the present invention) and a solvent. Solvents may be, by way of example, water, ethanol, or acetic add.

As used herein, the term "prodrug" includes biohydrolyzable amides and biohydrolyzable esters and also encompasses a) compounds in which the biohydrolyzable functionality in such a prodrug is encompassed in the compound according to the present invention, and b) compounds which may be oxidized or reduced biologically at a given functional group to yield drug substances according to the present invention. Examples of these functional groups include 1,4-dihydropyridine, N-alkylcarbonyl-1,4-dihydropyridine, 1,4-cyclohexadiene, tert-butyl, and the like.

As used herein, the term "biohydrolyzable ester" is an ester of a drug substance (in casu, a compound according to the invention) which either a) does not interfere with the biological activity of the parent substance but confers on that substance advantageous properties *in vivo* such as duration of action, onset of action, and the like, or b) is biologically inactive but is readily converted in vivo by the subject to the biologically active principle. The advantage is, for example increased solubility or that the biohydrolyzable ester is orally absorbed from the gut and is transformed to a compound according to the present invention in plasma. Many examples of such are known in the art and include by way of example lower alkyl esters (e.g., C₁-C₄), lower acyloxyalkyl esters, lower alkoxyacyloxyalkyl esters, alkoxyacyloxy esters, alkyl acylamino alkyl esters, and choline esters.

As used herein, the term "biohydrolyzable amide" is an amide of a drug substance (in casu, a compound according to the present invention) which either a) does not interfere with the biological activity of the parent substance but confers on that substance advantageous properties in vivo such as duration of action, onset of action, and the like, or b) is biologically inactive but is readily converted in vivo by the subject to the biologically active principle. The advantage is, for example increased solubility or that the biohydrolyzable amide is orally absorbed from the gut and is transformed to a compound according to the present invention in plasma. Many examples of such are known in the art and include by way of example lower alkyl amides, α-amino acid amides, alkoxyacyl amides, and alkylaminoalkylcarbonyl amides.

In the present context, the term "pharmaceutically acceptable salt" is intended to indicate salts which are not harmful to the patient. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like.

A "therapeutically effective amount" of a compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease disorder or condition as well as to prevent the condition, wherein prevention is to be understood as the management and care of a patient for the purpose of combating the disease, condition, or disorder and includes the administration of the active compounds to prevent the onset of the symptoms or complications. The patient to be treated is preferably a mammal, in particular a human being, but it may also include animals, such as dogs, cats, cows, sheep and pigs.

### DESCRIPTION OF THE INVENTION

The present invention relates to the use of compounds of formula I.

In one embodiment, each R1 and R2 are independently selected from the list consisting of hydrogen, alkyl, aryl, heteroaryl, halogen, nitro, alkoxy, -C(O)OR6, -S(O)₂OR6, in particular R1 and R2 independently represents alkyl, halogen or nitro. Specific examples of R1 and R2 include hydrogen, C₁₋₆alkyl, such as tert.-butyl, butyl, isopropyl or methyl; nitro, chloro, bromo, iodo, and C₁₋₆alkoxy, such as methoxy.

In one embodiment, R1 and R2 both represent methyl, isopropyl, sec.butyl, tert.butyl, methoxy or bromo.

In one embodiment, R1 represents methyl and R2 represents tert.butyl.

In one embodiment, R1 represents iodo and R2 represents methoxy.
In one embodiment, R3 represents hydrogen, alkyl, alkenyl, alkynyl, alkoxy or alkylamino. In particular, R3 may represent C₁₋₄alkyl, such as methyl.

In one embodiment, R4 represents nitro, cyano, -C(O)R6, -C(O)OR6, -S(O)₂OR6,-C(O)N(R6)₂, -S(O)ₙR6 or S(O)₂N(R6)₂ wherein n represents 1 or 2. In particular, R4 represents cyano.

In one embodiment of the invention, R4 and R5 together with the atoms to which they are attached constitute a 5 or 6 membered ring, which may be saturated, either partly or fully, or unsaturated, and wherein said ring is optionally substituted with one or more substituents selected from the list consisting of alkyl, halogen, hydroxyl, cyano and nitro. Examples of said rings are [1,3]Dithiolane 1,1,3,3-tetraoxide, 1,1-Dioxo-tetrahydro-1-thiophen-3-one, 1,1-Dioxo-thiazolidine-4-one, 1,1-Dioxo-thiomorpholine-3-one tetrahydrothiopyran-1,1-dioxide and tetrahydrothiophen-1,1-dioxide.

In one embodiment, R5 represents alkyl, cycloalkyl, alkenyl, aryl, heteroaryl, heterocyclyl, all of which are optionally substituted with one or more substituents selected from the list consisting of alkyl, halogen, hydroxyl, cyano, nitro, haloalkyl, -O-R7, -S(O)ₙR7, -O-C(O)R7, -C(O)O-R7, -C(O)-R7, -C(O)-N(R7)(R8), -N(R7)(R8), -(CH₂)ₚ-N(R8)-C(O)-R7,-B(OR7)(OR8), -(CH₂)ₚ-O-R7, -(CH₂)ₚ-N(R7)(R8), -NR7-C(O)R7 and -NR7-S(O)ₙ-R7.

In one embodiment, R5 represents C₁₋₁₂alkyl, such as methyl, propyl, 2-propyl, butyl, pentyl, hexyl, octyl, nonyl, wherein said alkyl may optionally be substituted with OR7, C(O)R7 or cycloalkyl, such as cyclopropane, cyclobutane and cyclohexane. In this embodiment, R7 may in particular represent hydrogen, C₁₋₄dialkylether radical and ethyl.

In one embodiment, R5 represents C₂₋₈alkenyl, such as pent-4-enyl and hex-5-enyl. In one embodiment, R5 represents cycloalkyl, which may be further substituted by C₁₋₆alkyl. Particular examples include cyclopentyl, cyclohexyl, cycloheptyl, cyclooctyl and tricyclo[2.2.1.0^{2,6}]heptyl.

In one embodiment, R5 represents aryl, and in particular phenyl or naphthyl, optionally substituted with one or more substituents selected from the list consisting of alkyl, alkenyl, alkynyl, heteroaryl, heterocyclyl, halogen, hydroxyl, cyano, nitro, haloalkyl, -O-R7, - S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7, -NR7-S(O)ₙ-R7. In a particular embodiment, said substituents are selected amongst, chloro, hydroxyl, cyano, carboxyl, nitro, NR7R8, -O-R7, C(O)-R7, -C(O)-O-R7, -C(O)-N(R7)(R8).

In one embodiment, R5 represents phenyl substituted with halogen, such as chloro, bromo and iodo.

In one embodiment, R5 represents phenyl substituted with C₁₋₆alkyl, C₂₋₆alkenyl or C₂₋₆alkynyl, wherein the substituents may be further substituted with -C(O)R7, -OR7 and phenyl. In this embodiment, R7 may in particular represent hydrogen and C₁₋₆alkyl, such as methyl, 2-methyl-propyl and butyl.

In one embodiment, R 5 represents phenyl substituted with a heterocycle, such as 2,3-dihydro-furan-2-yl.

In one embodiment, R5 represents phenyl substituted with -OR7, -C(O)OR7, or-S(O₂)R7. In this embodiment, R7 may in particular represent hydrogen, C₁₋₄alkyl, such as methyl; or C₁₋₄haloalkyl, such as -CF₃.

In one embodiment, R5 represents phenyl substituted with -C(O)NR7R8. in this embodiment, R7 and R8 may independently represent C₁₋₄dialkylether radical, such as methylethyl ether radical; C₁₋₄alkyl, such as methyl; or C₁₋₄hydroxyalkyl, such as 2-hydroxyethyl and 2-hydroypropyl. In this embodiment, R7 and R8 may also together with the nitrogen, to which they are bound form a 6-membered heterocyclo comprising N and optionally O, such as morpholinyl and piperazinyl, wherein said heterocycle may be further substituted with C₁₋₄alkyl, such as methyl.

In one embodiemnt, R5 represents a heteroaryl selected from the list consisting of pyridyl fuanyl and imidazolyl, optionally substituted with a substituent selected from the list consisting of alkyl, halogen, hydroxyl, cyano, nitro, haloalkyl, -O-R7, -S(O)ₙR7, -O-C(O)R7,-C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7, -NR7-S(O)ₙ-R7. In particular, said substituents are selected from the list consisting of fluoro, chloro, methyl, hydroxyl, cyano, nitro, -C(O)-O-R7, -C(O)-N(R7)(R8).

In one embodiment, R5 represents a heterocyclyl selected from the list consisting of piperidinyl, morpholinyl, pyrrolidinyl and azepanyl, optionally substituted with a substituent selected from the list consisting of alkyl, halogen, hydroxyl, cyano, nitro, haloalkyl, -O-R7,-S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7, -NR7-S(O)ₙ-R7. In particular, said substituents are selected from the list consisting of of fluoro, chloro, hydroxyl, cyano, nitro, -C(O)-O-R7, -C(O)-N(R7)(R8).

In one embodiment, R7 and R8 independently represent hydrogen, alkyl, haloalkyl, hydroxyalkyl or phenyl.

In one embodiment, R9 and R10 independently represent hydrogen, alkyl, haloalkyl or hydroxyalkyl.

In a still further embodiment, R11 and R12 independently represent hydrogen, C₁₋₆alkyl, C₁₋₆haloalkyl or C₁₋₆hydoxyalkyl, such as hydrogen, methyl, ethyl, trifluoromethyl, hydroxymethyl or 2-hydroxyethyl.

In a still further embodiment, R13 represents C₁₋₆alkyl, C₁₋₆haloalkyl or C₁₋₆hydroxyalkyl, such as methyl, ethyl, trifluoromethyl, hydroxymethyl or 2-hydroxyethyl.

In a still further embodiment, n is 2.

In a still further embodiment, p is 1.

Other embodiments of the invention can be seen from the claims.

Particular examples of the compounds of the present invention are
(E)-3-(3,5-Di-tert-butyl-4-hydroxy-phenyl)-2-methanesulfonyl-acrylonitrile;
(E)-2-(4-Chloro-benzenesulfonyl)-3-(3,5-di-tert-butyl-4-hydroxy-phenyl)-acrylonitrile;
(E)-2-(4-Chloro-benzenesulfonyl)-3-(4-hydroxy-3-nitro-phenyl)-acrylonitrile;
(E)-3-(4-Hydroxy-3-nitro-phenyl)-2-methanesulfonyl-acrylonitrile;
(E)-3-(3,5-Di-tert-butyl-4-hydroxy-phenyl)-2-(1-methyl-1H-imidazole-2-sulfonyl)-acrylonitrile;
(E/Z)-2-(4-Chloro-benzenesulfonyl)-3-(3,5-di-tert-butyl-4-hydroxy-phenyl)-but-2-enenitrile;
(E)-4-[3-Cyano-1-(3,5-di-tert-butyl-4-hydroxy-phenyl)-prop-1-ene-2-sulfonyl]-benzoic acid;
(E)-3-(3,5-Di-tert-butyl-4-hydroxy-phenyl)-2-(4-nitro-benzenesulfonyl)-acrylonitrile;
(E)-3-(3,5-Di-tert-butyl-4-hydroxy-phenyl)-2-(4-trifluoromethoxy-benzenesulfonyl)-acrylonitrile;
(E)-3-(3-tert-Butyl-4-hydroxy-5-nitro-phenyl)-2-(4-chloro-benzenesulfonyl)-acrylonitrile;
(E)-4-[3-Cyano-1-(3,5-di-tert-butyl-4-hydroxy-phenyl)-prop-1-ene-2-sulfonyl]-N,N-bis-(2-methoxy-ethyl)-benzamide;
(E)-4-[3-Cyano-1-(3,5-di-tert-butyl-4-hydroxy-phenyl)-prop-1-ene-2-sulfonyl]-N,N-dimethylbenzamide;
(E)-4-[3-Cyano-1-(3,5-di-tert-butyl-4-hydroxy-phenyl)-prop-1-ene-2-sulfonyl]-N,N-bis-(2-hydroxy-propyl)-benzamide;
(E)-3-(3,5-Di-tert-butyl-4-hydroxy-phenyl)-2-[4-(4-methyl-piperazine-1-carbonyl)-benzenesulfonyl]-acrylonitrile;
(E)-4-[3-Cyano-1-(3,5-di-tert-butyl-4-hydroxy-phenyl)-prop-1-ene-2-sulfonyl]-N-(2-hydroxyethyl)-benzamide;
(E)-3-(3,5-Di-tert-butyl-4-hydroxy-phenyl)-2-[4-(2,6-dimethyl-morpholine-4-carbonyl)-benzenesulfonyl]-acrylonitrile;
(E)-3-(3,5-Di-tert-butyl-4-hydroxy-phenyl)-2-[4-(morpholine-4-carbonyl)-benzenesulfonyl]-acrylonitrile;
(E)-2-(4-Amino-benzenesulfonyl)-3-(3,5-di-tert-butyl-4-hydroxy-phenyl)-acrylonitrile;
(E)-2-[2-Cyano-1-(3,5-di-tert-butyl-4-hydroxy-phenyl)-ethenesulfonyl]-(3,5-di-tert-butyl-4-hydroxy-phenyl)-acrylonitrile;
(E)-2-(4-Chloro-phenylmethanesulfonyl)-3-(3,5-di-tert-butyl-4-hydroxy-phenyl)-acrylonitrile;
(E)-3-(3,5-Di-tert-butyl-4-hydroxy-phenyl)-2-(propane-2-sulfonyl)-acrylonitrile;
4-[3-Cyano-1-(3,5-di-tert-butyl-4-hydroxy-phenyl)-prop-1-ene-2-sulfonyl]-N,N-bis-(2-hydroxyethyl)-benzamide;
3-(3,5-Di-tert-butyl-4-hydroxy-phenyl)-2-[4-(3,5-dimethyl-morpholine-4-carbonyl)-benzenesulfonyl]-acrylonitrile;
3-(3,5-Di-tert-butyl-4-hydroxy-phenyl)-2-(2,5-dichloro-benzenesulfonyl)-acrylonitrile;
2-(4-tert-Butyl-benzenesulfonyl)-3-(3,5-di-tert-butyl-4-hydroxy-phenyl)-acrylonitrile;
3-(3,5-Di-tert-butyl-4-hydroxy-phenyl)-2-(naphthalene-1-sulfonyl)-acrylonitrile;
3-(3,5-Di-tert-butyl-4-hydroxy-phenyl)-2-(furan-2-ylmethanesulfonyl)-acrylonitrile;
3-(3,5-Di-tert-butyl-4-hydroxy-phenyl)-2-(2,4-dichloro-benzenesulfonyl)-acrylonitrile;
3-(3,5-Di-tert-butyl-4-hydroxy-phenyl)-2-(toluene-4-sulfonyl)-acrylonitrile;
3-(3,5-Di-tert-butyl-4-hydroxy-phenyl)-2-(4-isopropyl-benzenesulfonyl)-acrylonitrile;
2-(3,5-Di-tert-butyl-4-hydroxy-benzenesulfonyl)-3-(3,5-di-tert-butyl-4-hydroxy-phenyl)-acrylonitrile;
3-(3,5-Di-tert-butyl-4-hydroxy-phenyl)-2-(hexane-1-sulfonyl)-acrylonitrile;
2-(4-Bromo-benzenesulfonyl)-3-(3,5-di-tert-butyl-4-hydroxy-phenyl)-acrylonitrile;
3-(3,5-Di-tert-butyl-4-hydroxy-phenyl)-2-(4-methanesulfonyl-benzenesulfonyl)-acrylonitrile;
3-(3,5-Di-tert-butyl-4-hydroxy-phenyl)-2-(3-trifluoromethoxy-benzenesulfonyl)-acrylonitrile;
3-(3,5-Di-tert-butyl-4-hydroxy-phenyl)-2-(2-trifluoromethoxy-benzenesulfonyl)-acrylonitrile;
2-(5-Chloro-pyridine-2-sulfonyl)-3-(3,5-di-tert-butyl-4-hydroxy-phenyl)-acrylonitrile;
2-[3-Cyano-1-(3,5-di-tert-butyl-4-hydroxy-phenyl)-prop-1-ene-2-sulfonyl]-benzoic acid;
3-[3-Cyano-1-(3,5-di-tert-butyl-4-hydroxy-phenyl)-prop-1-ene-2-sulfonyo-benzoic acid;
3-(3,5-Di-tert-butyl-4-hydroxy-phenyl)-2-[2-(morpholine-4-carbonyl)-benzenesulfonyl]-acrylonitrile;
3-(3,5-Di-tert-butyl4-hydroxy-phenyl)-2-[3-(morpholine-4-carbonyl)-benzenesulfonyl]-acrylonitrile;
3-(3,5-Di-tert-butyl-4-hydroxy-phenyl)-2-(4-methoxy-benzenesulfonyl)-acrylonitrile;
3-(3,5-Di-tert-butyl-4-hydroxy-phenyl)-2-(3-methoxy-benzenesulfonyl)-acrylonitrile;
3-(3,5-Di-tert-butyl-4-hydroxy-phenyl)-2-(3,4-dimethoxy-benzenesulfonyl)-acrylonitrile;
3-(3,5-Di-tert-butyl-4-hydroxy-phenyl)-2-[4-(2,3-dihydro-furan-2-yl)-benzenesulfonyl]-acrylonitrile;
3-{4-[2-Cyano-1-(3,5-di-tert-butyl-4-hydroxy-phenyl)-ethenesulfonyl]-phenyl}-2-methyl-acrylic acid methyl ester;
3-(3,5-Di-tert-butyl-4-hydroxy-phenyl)-2-(4-styryl-benzenesulfonyl)-acrylonitrile;
3-(3,5-Di-tert-butyl-4-hydroxy-phenyl)-2-[4-(2-isobutoxy-vinyl)-benzenesulfonyl]-acrylonitrile;
2-[4-(2-Butoxy-vinyl)-benzenesulfonyl]-3-(3,5-di-tert-butyl-4-hydroxy-phenyl)-acrylonitrile;
3-(3,5-Di-tert-butyl-4-hydroxy-phenyl)-2-(4-phenylethynyl-benzenesulfonyl)-acrylonitrile;
3-(3,5-Di-tert-butyl-4-hydroxy-phenyl)-2-(2-ethoxy-ethanesulfonyl)-acrylonitrile;
3-(3,5-Di-tert-butyl-4-hydroxy-phenyl)-2-[2-(2-ethoxy-ethoxy)-ethanesulfonyl]-acrylonitrile;
2-(4-Chloro-benzenesulfonyl)-3-(3,5-di-sec-butyl-4-hydroxy-phenyl)-acrylonitrile;
(E)-2-(3-Bromo-benzenesulfonyl)-3-(3,5-di-tert-butyl-4-hydroxy-phenyl)-acrylonitrile;
3-(3,5-Di-tert-buty)-4-hydroxy-phenyl)-2-(hex-5-ene-1-sulfonyl)-acrylonitrile;
(E)-2-(4-Iodo-benzenesulfonyl)-3-(3,5-di-tert-butyl-4-hydroxy-phenyl)-acrylonitrile;
(E)-2-Cyclopentanesulfonyl-3-(3,5-di-tert-butyl-4-hydroxy-phenyl)-acrylonitrite;
(E)-3-(3,5-Di-tert-butyl-4-hydroxy-phenyl)-2-(nonane-1-sulfonyl)-acrylonitrile;
(E)-3-(3,5-Di-tert-butyl-4-hydroxy-phenyl)-2-(pentane-1-sulfonyl)-acrylonitrile;
(E)-3-(3,5-Di-tert-butyl-4-hydroxy-phenyl)-2-(heptane-1-sulfonyl)-acrylonitrile;
(E)-2-Cyclohexanesulfonyl-3-(3,5-di-tert-butyl-4-hydroxy-phenyl)-acrylonitrile;
(E-3-{4-[2-Cyano-1-(3,5-di-tert-butyl-4-hydroxy-phenyl)-ethenesulfonyl]-phenyl}-propionic acid;
(E)-3-(3,5-Di-tert-butyl-4-hydroxy-phenyl)-2-(4-methyl-2-oxo-2H-chromene-7-sulfonyl)-acrylonitrile;
(E)-3-(3,5-Di-tert-butyl-4-hydroxy-phenyl)-2-(4-trifluoromethoxy-phenylmethanesulfonyl)-acrylonitrile;
(E)-2-Cyclohexylmethanesulfonyl-3-(3,5-di-tert-butyl-4-hydroxy-phenyl)-acrylonitrile;
(E)-2-(Butane-1-sulfonyl)-3-(3,5-di-tert-butyl-4-hydroxy-phenyl)-acrylonitrile;
(E)-2-(Propane-1-sulfonyl)-3-(3,5-di-tert-butyl-4-hydroxy-phenyl)-acrylonitrile;
(E)-2-(Ethane-1-sulfonyl)-3-(3,5-di-tert-butyl-4-hydroxy-phenyl)-acrylonitrile;
(E)- 2-Cyclopropylmethanesulfonyl-3-(3,5-di-tert-butyl-4-hydroxy-phenyl)-acrylonitrile;
(E)- 2-Cycloheptanesulfonyl-3-(3,5-di-tert-butyl-4-hydroxy-phenyl)-acrylonitrile;
(E)- 2-Cyclobutylmethanesulfonyl-3-(3,5-di-tert-butyl-4-hydroxy-phenyl)-acrylonitrile;
(E)-3-(3,5-Di-tert-butyl-4-hydroxy-phenyl)-2-(tricyclo[2.2.1.0^{2,6}]heptane-3-sulfonyl)-acrylonitrile;
(E)-2-Cyclooctanesulfonyl-3-(3,5-di-tert-butyl-4-hydroxy-phenyl)-acrylonitrile;
(E)-3-(3-tert-Butyl-4-hydroxy-5-methyl-phenyl)-2-(4-trifluoromethoxy-benzenesulfonyl)-acrylonitrile;
(E)-3-(3-tert-Butyl-4-hydroxy-5-methyl-phenyl)-2-(3-trifluoromethoxy-benzenesulfonyl)-acrylonitrile;
(E)-3-(3,5-Di-tert-butyl-4-hydroxy-phenyl)-2-(5-methyl-hexane-1-sulfonyl)-acrylonltrile;
(E)-2-(2-Cyclohexyl-ethanesulfonyl)-3-(3,5-di-tert-butyl-4-hydroxy-phenyl)-acrylonitrile;
(E)-3-(3,5-Di-tert-butyl-4-hydroxy-phenyl)-2-(4-methyl-pentane-1-sulfonyl)-acrylonitrile;
(E)-3-(3-tert-Butyl-4-hydroxy-5-methyl-phenyl)-2-(hexane-1-sulfonyl)-acrylonitrile;
(E)-3-(4-Hydroxy-3,5-diisopropyl-phenyl)-2-(4-trifluoromethoxy-benzenesulfonyl)-acrylonitrile;
(E)-3-(4-Hydroxy-3,5-diisopropyl-phenyl)-2-(3-trifluoromethoxy-benzenesulfonyl)-acrylonitrile;
(E)-2-(4-Chloro-benzenesulfonyl)-3-(4-hydroxy-3,5-dimethyl-phenyl)-acrylonitrile;
(E)-2-(4-Chloro-benzenesulfonyl)-3-(4-hydroxy-3,5-dibromo-phenyl)-acrylonitrile;
(E)-2-(4-Chloro-benzenesulfonyl)-3-(4-hydroxy-3,5-dimethoxy-phenyl-acrylonitrile;
(E)-2-(4-Chloro-benzenesulfonyl)-3-(4-hydroxy-3-iodo-5-methoxy-phenyl)-acrylonitrile;
(E)-2-(Hexane-1-sulfonyl)-3-(4-hydroxy-3,5-diisopropyl-phenyl)-acrylonitrile;
(E)-3-(3,5-Di-tert-butyl-4-hydroxy-phenyl)-2-[2-(2-methoxy-ethoxy)-ethanesulfonyl]-acrylonitrile;
(E)-3-(3,5-Di-tert-butyl-4-hydroxy-phenyl)-2-(2-methoxy-ethanesulfonyl)-acrylonitrile;
(E)-3-(3,5-Di-tert-butyl-4-hydroxy-phenyl)-2-(pent-4-ene-1-sulfonyl)-acrylonitrile;
(E)-3-(3,5-Di-tert-butyl-4-hydroxy-phenyl)-2-(piperidine-1-sulfonyl)-acrylonitrile;
(E)-3-(3,5-Di-tert-butyl-4-hydroxy-phenyl)-2-(azepane-1-sulfonyl)-acrylonitrile;
(E)-3-(3,5-Di-tert-butyl-4-hydroxy-phenyl)-2-(pyrrolidine-1-sulfonyl)-acrylonitrile; and
(E)-3-(3,5-Di-tert-butyl-4-hydroxy-phenyl)-2-(morpholine-4-sulfonyl)-acrylonitrile.

Compounds according to formula I may comprise chiral carbon atoms or carbon-carbon double bonds which may give rise to stereo isomeric forms, e.g. enatiomers, diastereomers and geometric isomers. The present invention relates to all such isomers, either in pure form or as mixtures thereof. Pure isomeric forms may either be prepared from intermediates which are pure isomers themselves, by purification of a mixture of isomers after the synthesis, or by a combination of the two methods. Purification of isomeric forms are well-known in the art, e.g. as described by Jaques in Enantiomers, Racemates and Resolution, Wiley, 1981.

The compounds of the present invention are useful in the treatment of diseases or states that benefit from an increase in the mitochondrial respiration.

The compounds of the present invention are believed to be particular well-suited for the treatment of obesity as such or preventing weight gain and for the treatment of diseases or disorders where obesity is involved in the etiology. In one embodiment, the invention thus provides a method of treating the metabolic syndrome, insulin resistance, dyslipidemia, hypertension, obesity, type 2 diabetes, type 1 diabetes, diabetic late complications including cardiovascular diseases, cardiovascular disorders, disorders of lipid metabolism, neurodegenerative and psychiatric disorders, dysregulation of intraocular pressure including glaucoma, atherosclerosis, hypertension, coronary heart disease, gallbladder disease, osteoarthritis, and cancer.

More specifically such conditions include the metabolic syndrome, type 2 diabetes (especially in obese patients), diabetes as a consequence of obesity, insulin resistance, hyperglycemia, prandial hyperglycemia, hyperinsulinemia, impaired glucose tolerance (IGT), impaired fasting glucose (IFG), increased hepatic glucose production, type 1 diabetes, LADA, pediatric diabetes, dyslipidemia (especially in obese patients), diabetic dyslipidemia, hyperlipidemia, hypertriglyceridemia, hyperlipoproteinemia,, micro-/macroalbuminuria, nephropathy, retinopathy, neuropathy, diabetic ulcers, cardiovascular diseases, arteriosclerosis, coronary artery disease, cardiac hypertrophy, myocardial ischemia, heart insufficiency, congestional heart failure, stroke, myocardial infarction, arrythmia, decreased blood flow, erectile dysfunction (male or female), myopathy, loss of muscle tissue, muscle wasting, muscle catabolism, osteoporosis, decreased linear growth, neurodegenerative and psychiatric disorders, Alzheimers disease, neuronal death, impaired cognitive function, depression, anxiety, eating disorders, appetite regulation, migraine, epilepsia, addiction to chemical substances, disorders of intraocular pressure, bacterial infections, mycobacterial infections. In the present context cancer is intended to include forms such as hematological cancer, such as leukemia, acute myeloide leukemia, chronic myeloide leukemia, chronic lymphatic leukemia, myelodysplasia, multiple myeloma, Hodgkin's disease, or solid tumor forms, such as fibrosarcom, small or non-small cell long carcinoma, gastric, intestinal or colorectal cancer, prostate, endometrial, ovarian or breast cancer, brain, head or neck cancer, cancer in the urinary tract, such as kidney or bladder cancer, malignant melanoma, liver cancer, uterine and pancreatic cancer.

In another embodiment, the invention relates to the use of a chemical uncoupler according to the present invention for maintaining a weight loss.

Use of the compounds according to the present invention in the treatment of obesity may very likely reduce or eliminate the side effects such as irritation of the skin, glaucoma etc. known from treatment of obesity with DNP and other chemical uncouplers with narrow safety windows.

Uncouplers may also reduce insulin release from β-cells and may thus be useful in providing β-cell rest. Inducing β-cell rest may be useful in connection with β-cell transplantation, and it has also been described that inducing β-cell rest may be useful in preventing diabetes.

Obesity drugs which regulate the appetite and reduce food intake often suffer from lack of long-term efficiency in terms of body weight loss because the body in response to the treatment lowers the rate of the metabolism. In contrast hereto, the compounds of the present invention increases the metabolism, and they are therefore believed to be particular suited for maintaining a weight loss.

The compounds of the present invention are also believed to be particularly well-suited for the treatment of diseases or disorders where reactive oxygen species are involved in the etiology, and wherein a reduction in the amount of reactive oxygen species is beneficial. In one embodiment, the invention thus provides a method of treating, and in particular preventing ageing and damages to the heart, endothelial cells and neuronal tissue, diabetic microvascular diseases in the retina, the renal glomerus and the peripheral nerve cells, the method comprising administering to a patient in need thereof a therapeutically effective amount of one or more compound of the present invention to a patient need thereof.

The subject may be any mammal suffering from a condition benefiting from increased mitochondrial respiration. Such mammals may include, for instance, horses, cows, sleep, pigs, mice, rats, dogs, cats, primates such as chimpanzees, gorillas, rhesus monkeys, and, most preferably, humans.

It is well-known that many compounds used to combat insects and parasites, i.e. insecticides and parasiticides, are chemical uncouplers. It is thus believed that uncouplers according to the present invention could be used as insecticides or parasiticides.

In the methods of the present invention, the compounds of the present invention may be administered alone or in combination with other therapeutically active compounds, either concomitantly or sequentially, and at any suitable ratios. Such further active compounds may be selected from antidiabetic agents, antihyperlipidemic agents, antiobesity agents, antihypertensive agents and agents for the treatment of complications resulting from or associated with diabetes.

Suitable antidiabetic agents include insulin, GLP-1 (glucagon like peptide-1) derivatives such as those disclosed in WO 98/08871 (Novd Nordisk A/S), which is incorporated herein by reference, as well as orally active hypoglycemic agents.

Suitable orally active hypoglycemic agents preferably include imidazolines, sulfonylureas, biguanides, meglitinides, oxadiazolidinediones, thiazolidinediones, insulin sensitizers, α-glucosidase inhibitors, agents acting on the ATP-dependent potassium channel of the pancreatic β-cells eg potassium channel openers such as those disclosed in WO 97/26265, WO 99/03861 and WO 00/37474 (Novo Nordisk A/S) which are incorporated herein by reference, potassium channel openers, such as ormitiglinide, potassium channel blockers such as nateglinide or BTS-67582, glucagon antagonists such as those disclosed in WO 99/01423 and WO 00/39088 (Novo Nordisk A/S and Agouron Pharmaceuticals, Inc.), all of which are incorporated herein by reference, GLP-1 agonists such as those disclosed in WO 00/42026 (Novo Nordisk A/S and Agouron Pharmaceuticals, Inc.), which are incorporated herein by reference, DPP-IV (dipeptidyl peptidase-IV) inhibitors, PTPase (protein tyrosine phosphatase) inhibitors, glucokinase activators, such as those described in WO 02/08209 to Hoffmann La Roche, inhibitors of hepatic enzymes involved in stimulation of gluconeogenesis and/or glycogenolysis, glucose uptake modulators, GSK-3 (glycogen synthase kinase-3) inhibitors, compounds modifying the lipid metabolism such as antihyperlipidemic agents and antilipidemic agents, compounds lowering food intake, and PPAR (peroxisome proliferator-activated receptor) and RXR (retinoid X receptor) agonists such as ALRT-268, LG-1268 or LG-1069.

In one embodiment of the methods, the compound of the present invention may be administered in combination with insulin or insulin analogues.

In one embodiment, the compound of the present invention may be administered in combination with a sulphonylurea eg tolbutamide, chlorpropamide, tolazamide, glibenclamide, glipizide, glimepiride, glicazide or glyburide.

In one embodiment, the compound of the present invention may be administered in combination with a biguanide eg metformin.

In one embodiment of the methods of the present invention, the compound of the present invention may be administered in combination with a meglitinide eg repaglinide or senaglinide/nateglinide.

In one embodiment, the compound of the present invention may be administered in combination with a thiazolidinedione insulin sensitizer, e.g. troglitazone, ciglitazone, pioglitazone, rosiglitazone, isaglitazone, darglitazone, englitazone, CS-011/CI-1037 or T 174 or the compounds disclosed in WO 97/41097 (e.g. 5-[[4-[3-methyl-4-oxo-3,4-dihydro-2-quinazolinyl]methoxy]phenylmethyl]thiazolidine-2,4-dione), WO 97/41119, WO 97/41120, WO 00/41121 and WO 98/45292, which are incorporated herein by reference.

In one embodiment, the compound of the present may be administered in combination with an insulin sensitizer e.g. such as GI 262570, YM-440, MCC-555, JTT-501, AR-H039242, KRP-297, GW-409544, CRE-16336, AR-H049020, LY510929, MBX-102, CLX-0940, GW-501516 or the compounds disclosed in WO 99/19313 (NN622/DRF-2725). WO 00/50414, WO 00/63191, WO 00/63192, WO 00/63193 and WO 00/23425, WO 00/23415, WO 00/23451, WO 00/23445, WO 00/23417, WO 00/23416, WO 00/63153, WO 00/63196, WO 00/63209, WO 00/63190 and WO 00/63189, which are incorporated herein by reference.

In one embodiment, the compound of the present invention may be administered in combination with an α-glucosidase inhibitor eg voglibose, emiglitate, miglitol or arcarbose,

In one embodiment, the compound of the present invention may be administered in combination with a glycogen phosphorylase inhibitor eg the compounds described in WO 97/09040.

In one embodiment, the compound of the present may be administered in combination with a glucokinase activator.

In one embodiment, the compound of the present invention may be administered in combination with an agent acting on the ATP-dependent potassium channel of the pancreatic β-cells eg tolbutamide, glibenclamide, glipizide, glicazide, BTS-67582 or repaglinide.

In one embodiment, the compounds of the present invention may be administered in combination with nateglinide.

In one embodiment, the compound of the present invention may be administered in combination with an antihyperlipidemic agent or a antilipidemic agent eg cholestyramine, colestipol, clofibrate, gemfibrozil, lovastatin, pravastatin, simvastatin, probucol or dextrothyroxine.

In one embodiment, the compound of the present invention may be administered in combination with more than one of the above-mentioned compounds e.g. in combination with metformin and a sulphonylurea such as glyburide; a sulphonylurea and acarbose; nateglinide and metformin; acarbose and melformin; a sulfonylurea, metformin and troglitazone; insulin and a sulfonylurea; insulin and metformin; insulin, metformin and a sulfonylurea; insulin and troglitazone; insulin and lovastatin; etc.

In one embodiment, the compound of the present invention may be administered in combination with one or more antiobesity agents or appetite regulating agents.

Such agents may be selected from the group consisting of CART (cocaine amphetamine regulated transcript) agonists, NPY (neuropeptide Y) antagonists, MC3 (melanocortin 3) agonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 adrenergic agonists such as CL-316243, AJ-9677, GW-0604, LY362884, LY377267 or AZ-40140, MSH (melanocyte-stimulating hormone) agonists, MCH (melanocyte-concentrating hormone) antagonists, CCK (cholecystokinin) agonists, serotonin reuptake inhibitors (fluoxetine, seroxat or citalopram), norepinephrine reuptake inhibitors (e.g. sibutramine), 5HT (serotonin) agonists, bombesin agonists, galanin antagonists, growth hormone, growth factors such as prolactin or placental lactogen, growth hormone releasing compounds, TRH (thyreotropin releasing hormone) agonists, UCP 2 or 3 (uncoupling protein 2 or 3) modulators, leptin agonists, DA (dopamine) agonists (bromocriptin, doprexin), lipase/amylase inhibitors, PPAR modulators, RXR modulators, TR β agonists, adrenergic CNS stimulating agents, AGRP (agouti related protein) inhibitors, H3 histamine antagonists such as those disclosed in WO 00/42023, WO 00/63208 and WO 00/64884, which are incorporated herein by reference, exendin-4, GLP-1 agonists and ciliary neurotrophic factor. Further antiobesity agents are bupropion (antidepressant), topiramate (anticonvulsant), ecopipam (dopamine D1/D5 antagonist), naltrexone (opioid antagonist), and peptide YY₃₋₃₆ (Batterham et al, Nature 418, 650-654 (2002)).

In one embodiment, the antiobesity agent is leptin.

In one embodiment, the antiobesity agent is a lipase inhibitor eg orlistat.

In one embodiment, the antiobesity agent is an adrenergic CNS stimulating agent eg dexamphetamine, amphetamine, phentermine, mazindol phendimetrazine, diethylpropion, fenfluramine or dexfenfluramine.

In a further embodiment, the compounds of the present invention may be administered in combination with one or more antihypertensive agents. Examples of antihypertensive agents are β-blockers such as alprenolol, atenolol, timolol, pindolol, propranolol and metoprolol; ACE (angiotensin converting enzyme) inhibitors such as benazepril, captopril, enalapril, fosinopril, lisinopril, quinapril and ramipril; calcium channel blockers such as nifedipine, felodipine, nicardipine, isradipine, nimodipine, diltiazem and verapamil; and *α-*blockers such as doxazosin, urapidil, prazosin and terazosin.

It should be understood that any suitable combination of the compounds according to the invention with diet and/or exercise, one or more of the above-mentioned compounds and optionally one or more other active substances are considered to be within the scope of the present invention.

The present invention also provides pharmaceutical compositions comprising as an active ingredient, at least one compound of the present invention, preferably in a therapeutically effective amount, suitable for any of the methods according to the present invention together with one or more pharmaceutically acceptable carriers or excipients. Said pharmaceutical compositions may also comprise any of the further active compounds as indicated above

The pharmaceutical composition is preferably in unit dosage form, comprising from about 0.05 mg to about 1000 mg, preferably from about 0.1 mg to about 500 mg and especially preferred from about 0.5 mg to about 200 mg of a compound suitable for any of the methods described above.

### PHARMACEUTICAL COMPOSITIONS

The compounds for methods according to the present invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 20th Edition, Gennaro, Ed., Mack Publishing Co., Easton, PA, 2000.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, aqueous or oily suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 1000 mg, preferably from about 0.1 to about 500 mg, and more preferred from about 0.5 mg to about 200 mg.

For parenteral routes such as intravenous, intrathecal, intramuscular and similar administration, typically doses are in the order of about half the dose employed for oral administration.

The compounds for use according to the present invention are generally utilized as the free substance or as a pharmaceutically acceptable salt thereof. Examples are an acid addition salt of a compound having the utility of a free base and a base addition salt of a compound having the utility of a free acid. The term "pharmaceutically acceptable salts" refers to non-toxic salts of the compounds for use according to the present invention which salts are generally prepared by reacting the free base with a suitable organic or inorganic acid or by reacting the acid with a suitable organic or inorganic base. When a compound for use according to the present invention contains a free base such salts are prepared in a conventional manner by treating a solution or suspension of the compound with a chemical equivalent of a pharmaceutically acceptable acid. When a compound for use according to the present invention, contains a free acid such salts are prepared in a conventional manner by treating a solution or suspension of the compound with a chemical equivalent of a pharmaceutically acceptable base. Physiologically acceptable salts of a compound with a hydroxy group include the anion of said compound in combination with a suitable cation such as sodium or ammonium ion. Other salts which are not pharmaceutically acceptable may be useful in the preparation of compounds of the invention and these form a further aspect of the invention.

For parenteral administration, solutions of the compounds for use according to the present invention in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the compounds for use according to the present invention and the pharmaceutically acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contains the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or a soft gelatine capsules wherein the active ingredient is mixed with water or an oil medium, for example peanut oil, liquid paraffin, or olive oil.

Aqueous suspensions may contain the compound for use according to the present invention in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions comprising compounds for use according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the invention are contemplated. For the purpose of this application, topical applications shall include mouth washes and gargles.

The compounds of the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the compounds of the present invention may form solvates with water or common organic solvents. Such solvates are also encompassed within the scope of the invention.

Thus, in a further embodiment, there is provided a pharmaceutical composition comprising a compound for use according to the present invention, or a pharmaceutical acceptable salt, solvate, or prodrug thereof, and one or more pharmaceutically acceptable carriers, excipients, or diluents.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

A typical tablet that may be prepared by conventional tabletting techniques may contain:

| Core: | | |
|---|---|---|
| Example 2 (as free compound or salt thereof) | | 5.0 mg |
| Lactosum Ph. Eur. | | 67.8 mg |
| Cellulose, microcryst. (Avicel) | | 31.4 mg |
| Amberlite®IRP88* | | 1.0 mg |
| Magnesii stearas Ph. Eur. | | q.s. |
| | | |

| Coating: | | |
|---|---|---|
| Hydroxypropyl methylcellulose | approx. | 9 mg |
| Mywacett 9-40 T** | approx. | 0.9 mg |

| | | |
|---|---|---|
| * Polacrillin potassium NF, tablet disintegrant, Rohm and Haas. ** Acylated monoglyceride used as plasticizer for film coating. | | |

If desired, the pharmaceutical composition comprising a compound for use according to the present invention may comprise a compound for use according to the present invention in combination with further active substances such as those described in the foregoing.

The present invention also provides methods for the preparation of compounds for use according to the present invention. The compounds can be prepared readily according to the following general procedures (in which all variables are as defined before, unless so specified) using readily available starting materials, reagents and conventional synthesis procedures. In these reactions, it is also possible to make use of variants which are themselves known to those of ordinary skill in this art, but are not mentioned in greater detail.

### Examples:

### HPLC-MS (Method A)

The following instrumentation is used:
- Hewlett Packard series 1100 G1312A Bin Pump
- Hewlett Packard series 1100 Column compartment
- Hewlett Packard series 1100 G1315A DAD diode array detector
- Hewlett Packard series 1100 MSD
- Sedere 75 Evaporative Light Scattering detector

The instrument is controlled by HP Chemstation software.

The HPLC pump is connected to two eluent reservoirs containing:
A: 0.01% TFA in water
B: 0.01% TFA in acetonitrile

The analysis is performed at 40°C by injecting an appropriate volume of the sample (preferably 1 µl) onto the column which is eluted with a gradient of acetonitrile.

The HPLC conditions, detector settings and mass spectrometer settings used are giving in the following table.
Column: Waters Xterra MS C-18 X 3 mm id 5 µm
Gradient: 5% - 100% acetonitrile linear during 7.5 min at 1.5ml/min
Detection: 210 nm (analogue output from DAD (diode array detector)) ELS (analogue output from ELS)
MS ionisation mode API-ES
Scan 100-1000 amu step 0.1 amu

After the DAD the flow is divided yielding approx 1 ml/min to the ELS and 0.5 ml/min to the MS.

### Starting materials:

General procedure for the preparation of substituted sulfonylacetonitriles

### General procedure A

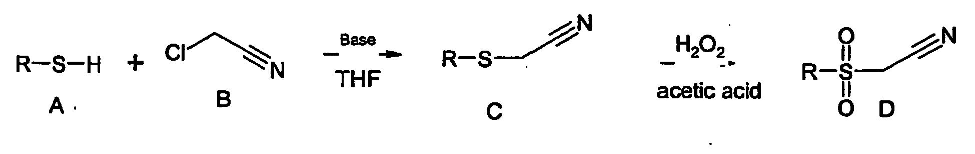

To a solution of the thiol compound A (1 equivalent) in tetrahydrofuran (THF), chloroacetonitril B (1 equivalent) was added. With cooling on an ice bath 1N NaOH (1 eqivalent) was added. The reaction mixture was stirred until the starting materials disappeared. The alkylated thiols C were isolated after aqueous work-up and extraction with and organic solvent. Compound C was dissolved in acetic acid and treated with a 30% hydrogenperoxide ( 6 equivalents). The reaction mixture was heated at reflux for 4-8 hours. Compounds D were isolated by standard procedures.

### General procedure B

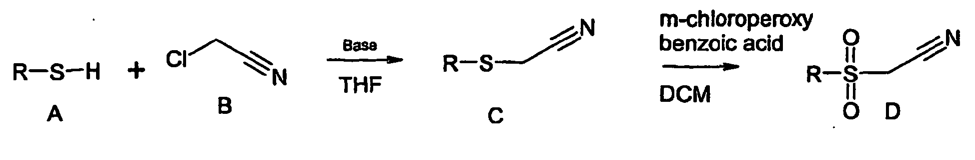

To a solution of the thiol compound A (1 equivalent) in tetrahydrofuran (THF), chloroacetonitril B (1 equivalent) was added. With cooling on an ice bath 1 N NaOH (1 equivalent) was added. The reaction mixture was stirred until the starting materials disappeared. The alkylated thiols C were isolated after aqueous work-up and extraction with and organic solvent. Compound C was dissolved in methylenechloride and treated with m-chloroperoxybenzoic acid (2 equivalents). The reaction mixture was stirred at room temperature for 24-48 hours. Compounds D were isolated by standard procedures.

### General procedure A:

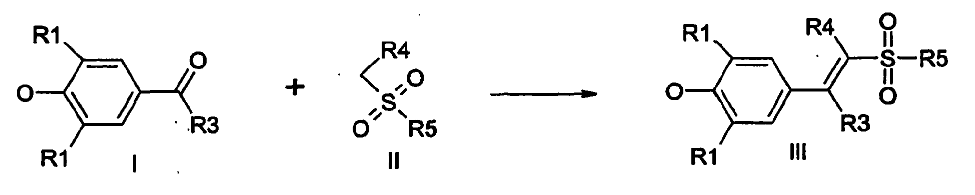

To a solution of the appropriate carbonylcompound I (1 mmole) in ethanol 4 ml, the appropriate sulfonylcompound II (1 mmole) and a catalytic amount of piperidine (0.1 mmole) was added. The reaction mixture was heated at reflux for 12 hours. The products III were isolated either by,
Step A: cooling filtration and crystallisation
   or
Step B: cooling, separation of the compound with water, and crystallisation with an organic solvent
   or
Step C: Aqueous work up followed by column chromatography

### General procedure B:

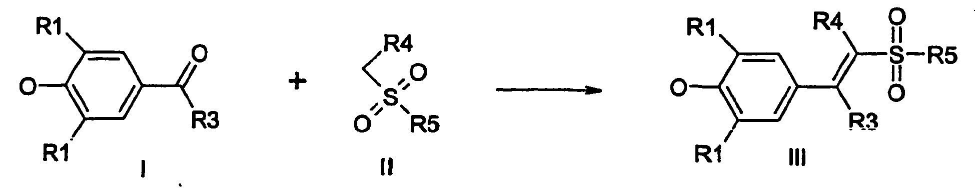

To a solution of the appropriate carbonylcompound I (1 mmole) in toluene 10 ml, the appropriate sulfonylcompound II (1 mmole) and ammonium acetate (1 mmole) was added. The reaction mixture was heated at reflux for 12 hours with continuous separation of water. The products III were isolated either by
Step A: Aqueous work up followed by column chromatography
   or
Step C: Aqueous work up followed by crystallisation.

### General procedure C

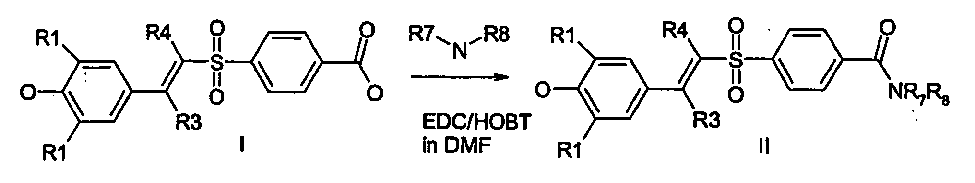

To a solution of compound of the general formula I in DMF, 1.5 molar equivalent of 1-ethyl-3-(3-dimethylaminopropyl)carbodiimide and 1 molar equivalent of hydroxybenzotriazole were added. The reaction mixture was stirred at room temperature for 0.5 hour, whereupon a compound of the general formula and triethylamine 1 equivalent was added. The reaction mixture is stirred at room temperature overnight The compounds synthesised by this general procedure were purified by aqueous work-up followed by crystallisation from an organic solvent.

### General procedure D

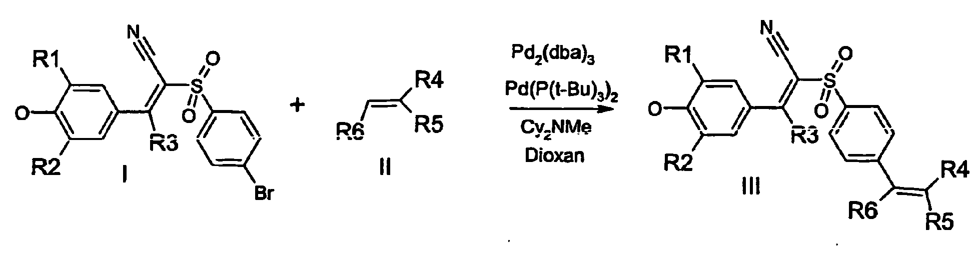

To a solution of the bromo compound I (1 equivalent) in an organic solvent like dioxane or tetrahydrofuran, Pd₂(dba)₃ (0.01 equivalent) and Pd(P(t-bu)₃)₂ (0.02 equivalent) were added. To this solution the appropriate vinyl compound II (1 equivalent) followed by dicyclohexylmethylamine (1.1 equivalent) were added. The reaction mixture was stirred at room temperature for 1-3 days. The compounds III were isolated by aqueous work-up followed by column chromatography.

### Example 1 (General procedure (A))

### (E)-3-(3,5-Di-tert-butyl-4-hydroxy-phenyl)-2-methanesulfonyl-acrylonitrile

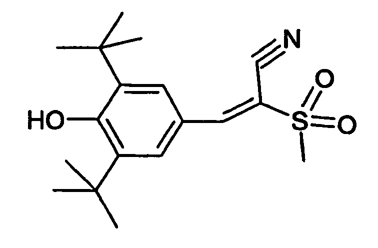

Step A: The title compound was prepared from 3,5-di-tert-butyl-4-hydroxybenzaldehyde and methanesulfonylacetonitrile in 35 % yield.

¹H NMR (CDCl₃ ): δ 1.47 (s, 18 H) 3.18 (s, 3 H) 6.03 (s, 1 H) 7.86 (s, 2 H) 8.04 (s, 1 H); HPLC-MS (Method A): *m*/*z* = 358 (M+Na); Rₜ = 4.79 min.

### Example 2 (General procedure (A))

### (E)-2-(4-Chloro-benzenesulfonyl)-3-(3,5-di-tert-butyl-4-hydroxy-phenyl)-acrylonitrile

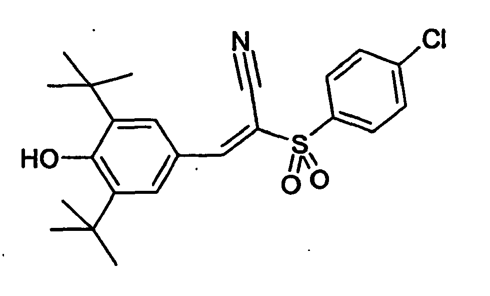

Step A: The title compound was prepared from 3,5-di-tert-butyl-4-hydroxybenzaldehyde and 4-chlorophenylsulfonylacetonitrile in 72 % yield

¹H NMR (CDCl₃): δ 1.45 (s, 18 H) 6.01 (s, 1 H) 7.56 (d, *J*=8.59 Hz, 2 H) 7.83 (s, 2 H) 7.94 (d, *J*=8.59 Hz, 2 H) 8.12 (s, 1 H); HPLC-MS (Method A): *m*/*z* = 432 (M+1); Rₜ = 4.88 min.

### Example 3 (General procedure (A))

### (E)-3-(3,5-Di-tert!-butyl-4-hydroxy-phenyl)-2-(pyridine-2-sulfonyl)-acrylonitrile

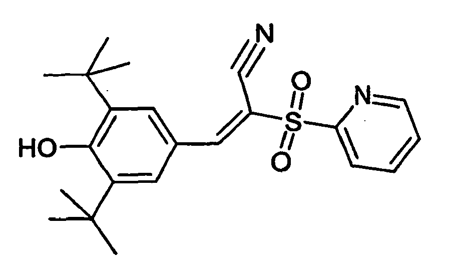

Step A: The title compound was prepared from 3,5-di-tert-butyl-4-hydroxybenzaldehyde and pyridine-2-sulfonylacetonitrile in 88 % yield

¹H NMR (DMSO-*d*₆): δ ppm 1.38 (s, 18 H) 7.81 (m, 1H) 8.00 (s, 2 H) 8.23 (m, 2 H) 8.41 (s, 1 H) 8.51 (m, 1 H) 8.81 (m, 1H); HPLC-MS (Method A): *m*/*z* = 400 (M+1); Rₜ = 4.9 min.

### Example 4 (General procedure (A))

### (E)-2-(4-Chloro-benzenesulfonyl)-3-(4-hydroxy-3-nitro-phenyl)-acrylonitrile

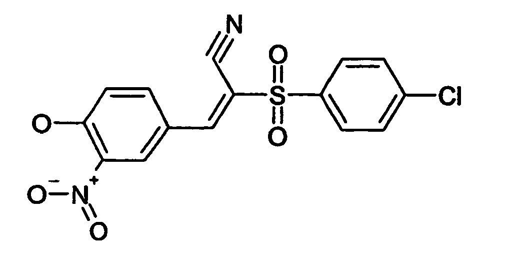

Step A: The title compound was prepared from 3-tert-butyl-5-nitro-4-hydroxybenzaldehyde and 4-chlorophenylsulfonylacetonitrile in 67 % yield

¹H NMR (DMSO-*d*₆): δ ppm 7.30 (d, *J*=9.10 Hz, 1 H) 7.83 (d, *J*=8.59 Hz, 2 H) 8.02 (d, *J*=8.59 Hz, 2 H) 8.21 (dd, 1 H) 8.55 (s, 1 H) 8.66 (d, *J*=2.02 Hz, 1 H); HPLC-MS (Method A): *m*/*z* = 365 (M+1); Rₜ = 3.88 min.

### Example 5 (General procedure (A))

### (E)-3-(4-Hydroxy-3-nitro-phenyl)-2-methanesulfonyl-acrylonitrile

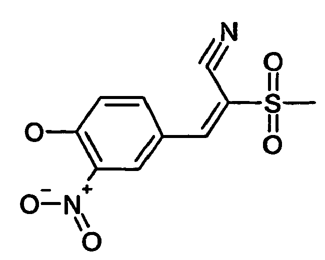

Step A: The title compound was prepared from 3-tert-butyl-5-nitro-4-hydroxybenzaldehyde and methanesulfonylacetonitrile in 57 % yield.

¹H NMR (DMSO-*d*₆): δ ppm 3.34 (m, 3 H) 7.28 (d, *J*=9.10 Hz, 1 H) 8.20 (dd, *J*=9.10, 2.53 Hz, 1 H) 8.27 (s, 1 H) 8.64 (d, *J*=2.02 Hz, 1 H); HPLC-MS (Method A): *m*/*z* = 269 (M+1); Rₜ = 2.67 min.

### Example 6 (General procedure (A))

### (E)-3-(3,5-Di-tert-butyl-4-hydroxy-phenyl)-2-(1-methyl-1H-imidazole-2-sulfonyl)-acrylonitrile

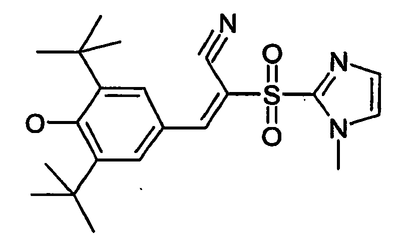

Step C: The title compound was prepared from 3,5-di-tert-butyl-4-hydroxybenzaldehyde and 1-methylimidazol-2-ylsulfonylacetonitrile in 8 % yield.

¹H NMR (DMSO-*d*₆): δ ppm 1.29 (s, 18 H) 3.95 (m, 3 H) 7.05 (s, 1 H) 7.44 (s, 1 H) 7.52 (s, 1 H) 7.63 (s, 2 H) 8.22 (s, 1H); HPLC-MS (Method A): *m*/*z* = 421 (M+1); Rₜ =4.6 min.

### Example 7 (General procedure (B))

### (E/Z)-2-(4-Chloro-benzenesulfonyl)-3-(3,5-di-tert-butyl-4-hydroxy-phenyl)-but-2-enenitrile

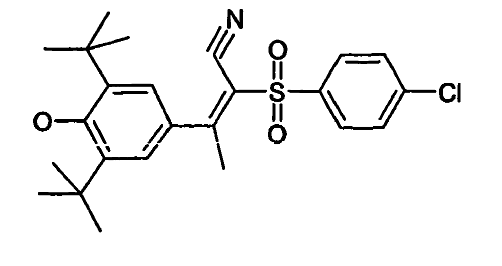

Step A: The title compound was prepared from 3,5-di-tert-butyl-hydroxyacetophenone and 4-chloro-benzenesulfonyl acetonitrile in 2 % yield.

¹H NMR (CDCl₃): (E) δ ppm 1.43 (s, 18 H) 2.69 (s, 3 H) 5.66 (m, 1 H) 7.33 (s, 2 H) 7.59 (d, *J*=9.04 Hz, 2 H) 8.00 (d, *J*=8.67 Hz, 2 H); ¹H NMR (CDCl₃): (Z) δ ppm 1.41 (s, 18 H) 2.51 (m, 3 H) 5.55 (s, 1 H) 6.92 (s, 2 H) 7.26 (d, 2 H) 7.39 (d, 2 H); HPLC-MS (Method A): *m*/*z* = 468 (M+23); Rₜ = 5.6 min.

### Example 8 (General procedure (A))

### (E)-4-[3-Cyano-1-(3,5-di-tert-butyl-4-hydroxy-phenyl)-prop-1-ene-2-sulfonyl]-benzoic acid

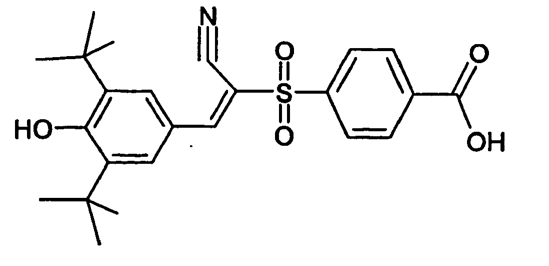

Step A: The title compound was prepared from 3,5-di-tert-butyl-4-hydroxybenzaldehyde and 4-carboxyphenylsulfonylacetonitrile in 90 % yield

¹H NMR (DMSO-*d*₆): δ ppm 1.39 (s, 18 H) 7.97 (s, 2 H) 8.11 (d, *J*=8.08 Hz, 2 H) 8.22 (d, *J*=8.08 Hz, 2 H) 8.48 (s, 1 H) 8.54 (m, 1 H) 13.65 (m, 1 H).

### Example 9 (General procedure (A))

### (E)-3-(3,5-Di-tert-butyl-4-hydroxy-phenyl)-2-(4-nitro-benzenesulfonyl)-acrylonitrile

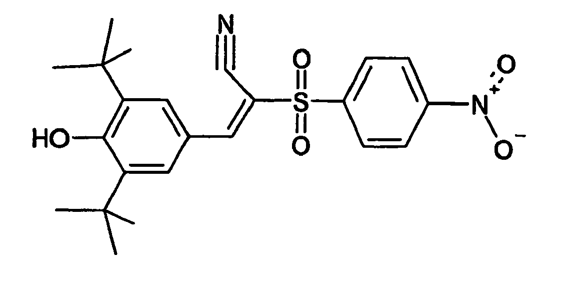

Step C: The title compound was prepared from 3,5-di-tert-butyl-4-hydroxybenzaldehyde and 4-nitrophenylsulfonylacetonitrile in 27 % yield

¹H NMR (DMSO-*d*₆): δ ppm 1.39 (s, 18 H) 7.69 (s, 1 H) 7.98 (s, 2 H) 8.27 (d, *J*=8.59 Hz, 2 H) 8.49 (d, 2 H) 8.53 (s, 1 H).

### Example 10 (General procedure (A))

### (E)-3-(3,5-Di-tert-butyl-4-hydroxy-phenyl)-2-(4-fluoro-benzenesulfonyl)-acrylonitrile

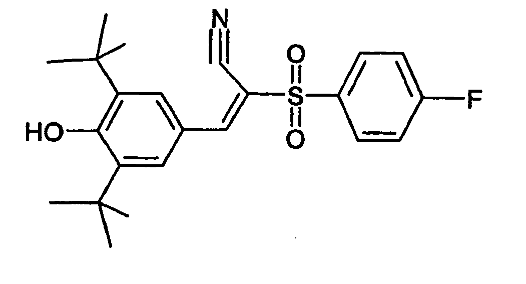

Step A: The title compound was prepared from 3,5-di-tert-butyl-4-hydroxybenzaldehyde and 4-fluoronitrophenylsulfonylacetonitrile in 37 % yield

¹H NMR (DMSO-*d*₆): δ ppm 1.39 (m, 18 H) 7.58 (dd, *J*=8.84 Hz, 2 H) 7.95 (s, 2 H) 8.08 (dd, *J*=9.10, 5.05 Hz, 2 H) 8.44 (s, 1 H) 8.46 (s, 1 H); HPLC-MS (Method A): *m*/*z* = 416 (M+1).

### Example 11 (General procedure (A))

### (E)-3-(3,5-Di-tert-butyl-4-hydroxy-phenyl)-2-(4-trifluoromethoxy-benzenesulfonyl)-acrylonitrile

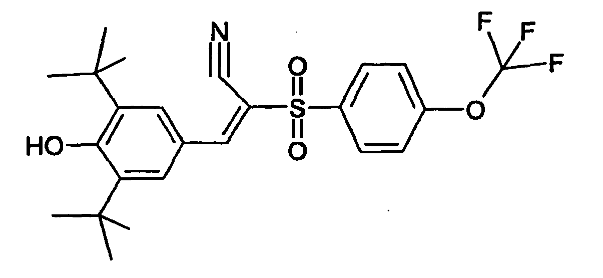

Step A: The title compound was prepared from 3,5-di-tert-butyl-4-hydroxybenzaldehyde and 4-trifluoromethoxyphenylsulfonylacetonitrile in 69 % yield.

¹H NMR (DMSO-*d*₆): δ ppm 1.41 (m, 18 H) 7.72 (d, *J*=8.08 Hz, 2 H) 7.96 (s, 2 H) 8.14 (d, *J*=9.10 Hz, 2 H) 8.46 (s, 1 H) 8.50 (s, 1 H); HPLC-MS (Method A): *m*/*z* = 482 (M+1).

### Example 12

### (E)-3-(3-tert-Butyl-4-hydroxy-5-nitro-phenyl)-2-(4-chloro-benzenesulfonyl)-acrylonitrile

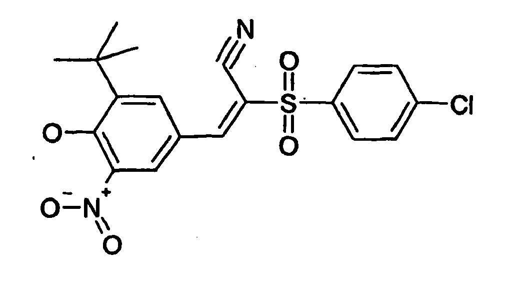

To a suspension of (E)-2-(4-Chloro-benzenesulfonyl)-3-(3,5-di-tert-butyl-4-hydroxyphenyl)-acrylonitrile (250 mg, 0.58 mmole) in acetic acid, nitric acid (72 mg, 1.16 mmole) was added. The reaction mixture was stirred at room temperature for 1 hour. Water was added to the reaction mixture and the separated crystals were filtered. The title compound was purified by column chromatography with dichloromethane as eluent. Yield 35 mg, 14%.

¹H NMR (CDCl₃): δ ppm 1.46 (s, 9 H) 7.60 (d, *J*=8.67 Hz, 2 H) 7.96 (d, *J*=8.67 Hz, 2 H) 8.15 (s, 1 H) 8.33 (d, *J*=1.88 Hz, 1 H) 8.52 (d, *J*=2.26 Hz, 1 H) 12.03 (s, 1 H); HPLC-MS (Method A): *m*/*z* = 444 (M+Na); Rₜ = 5.4 min.

### Example 13 (General procedure (C))

### (E)-4-[3-Cyano-1-(3,5-di-tert-butyl-4-hydroxy-phenyl)-prop-1-ene-2-sulfonyl]-N,N-bis-(2-methoxy-ethyl)-benzamide

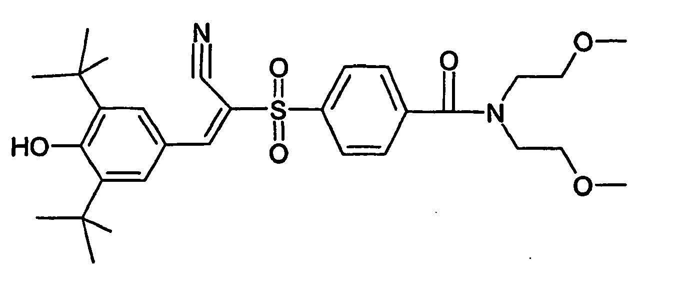

Step A: the title compound was prepared from (E)-4-[3-Cyano-1-(3,5-di-tert-butyl-4-hydroxy-phenyl)-prop-1-ene-2-sulfonyl]-benzoic acid and bis(2-methoxyethyl)amine in 62 % yield.

¹H NMR (CDCl₃): δ ppm 1.46 (s, 18 H) 3.27 (s, 3 H) 3.38 (s, 3 H) 3.46 (m, 4 H) 3.67 (m, *J*=4.55 Hz, 2 H) 3.75 (m, *J*=4.55 Hz, 2 H) 6.02 (s, 1 H) 7.64 (d, *J*=8.59 Hz, 2 H) 7.84 (s, 2 H) 8.03 (d, *J*=8.59 Hz, 2 H) 8.13 (s, 1 H); HPLC-MS (Method A): *m*/*z =* 557 (M+1): R₄ = 4.92 min.

### Example 14 (General procedure (C))

### (E)-4-[3-Cyano-1-(3,5-di-tert-butyl-4-hydroxy-phenyl)-prop-1-ene-2-sulfonyl]-N,N-dimethyl-benzamide

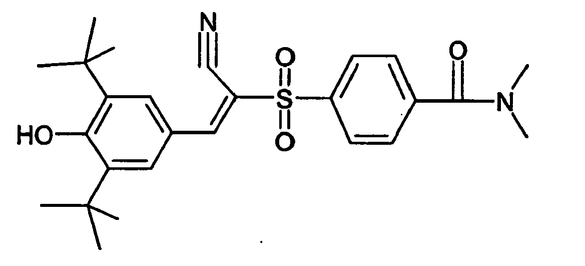

The title compound was prepared from (E)-4-[3-Cyano-1-(3,5-di-tert-buty)-4-hydroxyphenyl)-prop-1-ene-2-sulfonyl]-benzoic acid dimethylamine in 90 % yield.

¹H NMR (DMSO-*d*₆): δ ppm 1.46 (s,18H) 2.86 (s, 6 H) 7.72 (d, *J*=8.59 Hz, 2 H) 7.95 (s, 2 H) 8.03 (d, *J*=8.08 Hz, 2 H) 8.44 (s, 1 H) 8.47 (s, 1 H); HPLC-MS (Method A): *m*/*z* = 469 (M+1); R₁ = 4.99 min.

### Example 15 (General procedure (C))

### (E)-4-[3-Cyano-1-(3,5-di-tert!-butyl-4-hydroxy-phenyl)-prop-1-ene-2-sulfonyl]-N,N-bis-(2-hydroxy-propyl)-benzamide.

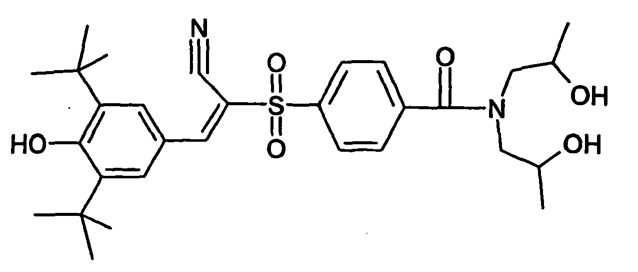

The title compound was prepared from (E)-4-(3-Cyano-1-(3,5-di-tert-butyl-4-hydroxy-phenyl)-prop-1-ene-2-sulfonyl]-benzoic acid diisopropanolamine in 32 % yield.

¹H (NMR (DMSO-*d*₆): δ ppm 1.10 (d, 6 H) 1.38 (s, 18 H) 3.14 (m, 2 H) 3.76 (m,1 H) 4.00 (m, 1 H) 4.91 (m, 2 H) 7.71 (d, *J*=8.59 Hz, 2 H) 7.96 (s, 2 H) 8.03 (d, *J*=7.07 Hz, 2 H) 8.46 (s, 2 H); HPLC-MS (Method A): *m*/*z* = 557(M+1); Rₜ = 4.71 min.

### Example 16 (General procedure (C))

### (E)-3-(3,5-Di-tert-butyl-4-hydroxy-phenyl)-2-[4-(4-methyl-piperazine-1-carbonyl)-benzenesulfonyl]-acrylonitrile.

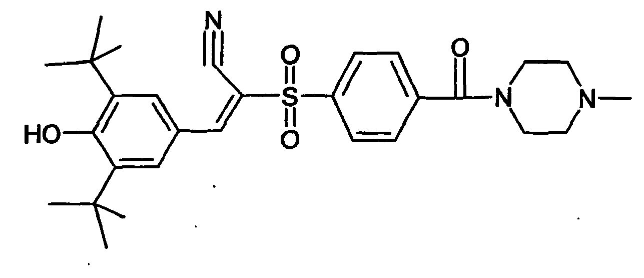

Step A: The title compound was prepared from (E)-4-[3-Cyano-1-(3,5-di-tert-butyl-4-hydroxyphenyl)-prop-1-ene-2-sulfonyl]-benzoic acid N-methylpiperazine in 38 % yield.

¹H NMR (DMSO-*d*₆): δ ppm 1.39 (s, 18 H) 2.81 (s, 3 H) 3.53 (m, 8 H) 7.78 (d, *J*=8.59 Hz, 2 H) 7.97 (s, 2 H) 8.10 (d, *J*=8.59 Hz, 2 H) 8.47 (s, 1 H); HPLC-MS (Method A): *m*/*z* = 534 (M+1); Rₜ = 3.85 min.

### Example 17 (General procedure (C))

### (E)-4-[3-Cyano-1-(3,5-di-tert-butyl-4-hydroxy-phenyl)-prop-1-ene-2-sulfonyl]-N-(2-hydroxy-ethyl)-benzamide.

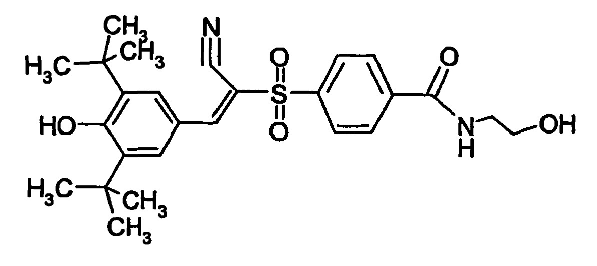

Step A: The title compound was prepared from (E)-4-[3-Cyano-1-(3,5-di-tert-butyl-4-hydroxy phenyl)-prop-1-ene-2-sulfonyl]-benzoic acid and ethanolamine in 28 % yield.

¹H NMR (DMSO-*d*₆): δ ppm 1.38 (s, 18 H) 3.29 (m, 2 H) 3.51 (t, *J*=6.06 Hz, 2 H) 4.77 (m, 1 H) 7.93 (s, 2 H) 8.07 (d, *J*=8.59 Hz, 2 H) 8.11 (d, 2 H) 8.42 (s, 1 H) 8.49 (m, 1 H) 8.75 (t, *J*=5.81 Hz, 1 H); HPLC-MS (Method A): *m*/*z* = 486 (M+1); Rₜ = 4.23 min:

### Example 18 (General procedure (C))

### (E)-3-(3,5-Di-tert-butyl-4-hydroxy-phenyl)-2-[4-(2,6-dimethyl-morpholine-4-carbonyl)-benzenesulfonyl]-acrylonitrile.

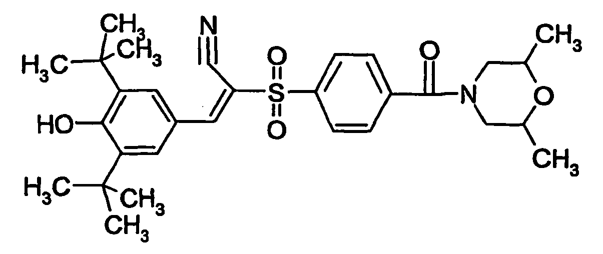

Step A: The title compound was prepared from (E)-4-[3-Cyano-1-(3,5-di-tert-butyl-4-hydroxyphenyl)-prop-1-ene-2-sulfonyl]-benzoic acid and 2,6-dimethylmorpholine in 65 % yield.

¹H NMR (DMSO-*d*₆): δ ppm 0.97 (d, *J*=5.05 Hz, 3 H) 1.15 (d, *J*=5.05 Hz, 3 H) 1.38 (s, 18 H) 2.82 (t, 1 H) 3.32 (m, 2 H) 3.55 (m, 2 H) 4.37 (d, *J*=12.13 Hz, 1 H) 7.72 (d, *J*=8.08 Hz, 2 H) 7.96 (s, 2 H) 8.05 (d, *J*=8.08 Hz, 2 H) 8.46 (s, 1 H) 8.48 (s, 1 H); HPLC-MS (Method A): *m*/*z* = 538 (M+1); Rₜ = 5.05 min.

### Example 19 (General procedure (C))

### (E)-3-(3,5-Di-tert-butyl-4-hydroxy-phenyl)-2-[4-(morpholine-4-carbonyl)-benzenesulfonyl]-acrylonitrile.

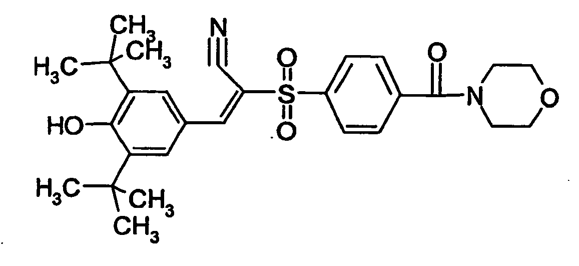

Step A: The title compound was prepared from (E)-4-[3-Cyano-1-(3,5-di-tert-butyl-4-hydroxyphenyl)-prop-1-ene-2-sulfonyl]-benzoic acid and morpholine in 83 % yield.

¹H NMR (DMSO-*d*₆): δ ppm 1.39 (s, 18 H) 3.27 (s, 2 H) 3.53 (s, 2 H) 3.65 (m, 4 H) 7.74 (d, J=8.59 Hz, 2 H) 7.97 (s, 2 H) 8.06 (d, *J*=8.08 Hz, 2 H) 8.47 (s, 1 H) 8.49 (m, 1 H); HPLC-MS (Method A): *m*/*z* = 511 (M+1); Rₜ = 4.70 min.

### Example 20 (General procedure (A))

### (E)-2-(4-Amino-benzenesulfonyl)-3-(3,5-di-tert-butyl-4-hydroxy-phenyl)-acrylonitrile

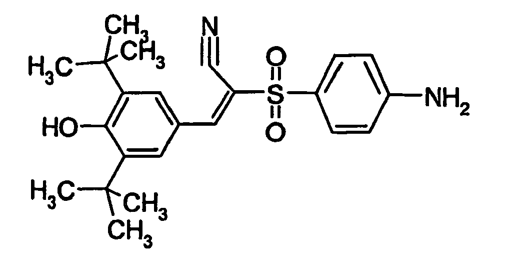

Step C: The title compound was prepared from 3,5-di-tert-butyl-4-hydroxybenzaidehyde and 4-aminophenylsulfonylacetonifile in 55 % yield.

¹H NMR (CDCl₃): δ ppm 1.45 (s, 18 H) 4.28 (s, 2 H) 5.92 (s, 1 H) 6.71 (d, *J*=8.59 Hz, 2 H) 7.74 (d, *J*=9.10 Hz, 2 H) 7.81 (s, 2 H) 8.07 (s, 1 H); HPLC-MS (Method A): *m*/*z* = 413 (M+1);Rₜ= 4.80 min.

### Example 21 (General procedure (A))

### (E)-2-Benzenesulfonyl-3-(3,5-di-tert-butyl-4-hydroxy-phenyl)-acrylonitrile

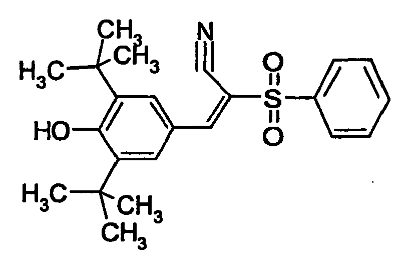

Step A: The title compound was prepared from 3,5-di-tert-butyl-4-hydroxybenzaldehyde and phenylsulfonylacetonitrile in 61 % yield

¹H NMR (DMSO-*d*₆): δ ppm 1.39 (s, 18 H) 7.76 (m, 3 H) 7.95 (s, 2 H) 8.00 (d, *J*=7.16 Hz, 2 H) 8.44 (s, 2 H); HPLC-MS (Method A): *m*/*z* = 399 (M+1 ); Rₜ = 5.5 min.

### Example 22 (General procedure (A))

### (E)-2-[2-Cyano-1-(3,5-di-tert-butyl-4-hydroxy-phenyl)-ethenesulfonyl]-3-(3,5-di-tert-butyl-4-hydroxy-phenyl)-acrylonitrile

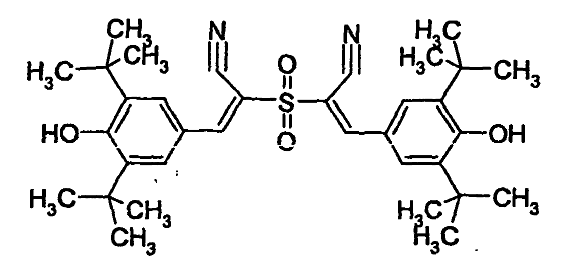

Step A: The title compound was prepared from 3,5-di-tert-butyl-4-hydroxybenzaldehyde and sulfonyldiacetonitrile in 14 % yield

¹H NMR (DMSO-*d*₆): δ ppm 1.41 (s, 36 H) 8.03 (s, 4 H) 8.38 (s, 2 H) 8.58 (s, 2 H); HPLC-MS (Method A): *m*/*z*= 600 (M+23); Rₜ = 6.6 min.

### Example 23 (General procedure (A))

### (E)-3-(3,5-Di-tert-butyl-4-hydroxy-phenyl)-2-(thiophene-2-sulfonyl)-acrylonitrile

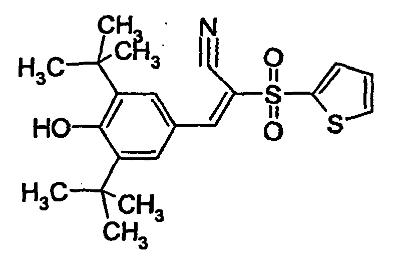

Step A: The title compound was prepared from 3,5-di-tert-butyl-4-hydroxybenzaldehyde and thiophene-2-sulfonylacetonitrile in 66 % yield

¹H NMR (DMSO-*d*₆): δ 1.39 (s, 18 H) 7.34 (dd, *J*=4.52 Hz, 1 H) 7.91 (d, *J*=3.77 Hz, 1 H) 7.96 (s, 2 H) 8.23 (d, *J*=4.90 Hz, 1 H) 8.42 (s, 1 H) 8.46 (m, 1 H); HPLC-MS (Method A): *m*/*z* = 404 (M+23); Rₜ = 5.3 min.

### Example 24 (General procedure (A))

### E)-2-(4-Chloro-phenylmethanesulfonyl)-3-(3,5-di-tert-butyl-4-hydroxy-phenyl)-acrylonitrile

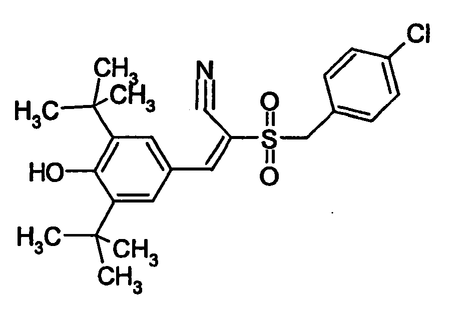

Step A: The title compound was prepared from 3,5-di-tert-butyl-4-hydroxybenzaldehyde and 4-chlorobenzylsulfonylacetonitrile in 17 % yield

¹H NMR (DMSO-*d*₆): δ ppm 1.39 (s, 18 H) 4.82 (s, 2 H) 7.42 (d, *J*=8.67 Hz, 2 H) 7.48 (d, 2 H) 7.83 (s, 2 H) 7.92 (s, 1 H) 8.43 (s, 1 H); HPLC-MS (Method A): *m*/*z* = 469 (M+1); Rₜ = 5.4 min.

### Example 25 (General procedure (A))

### (E)-3-(3,5-Di-tert-butyl-4-hydroxy-phenyl)-2-(propane-2-sulfonyl)-acrylonitrile

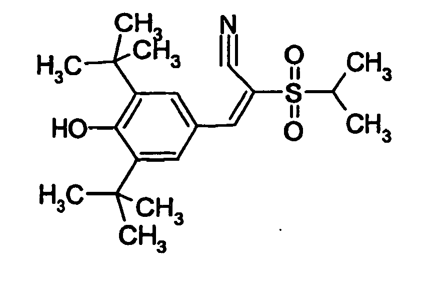

Step A: The title compound was prepared from 3,5-di-tert-butyl-4-hydroxybenzaldehyde and propane-2-sulphonylacetonitrile in 34 % yield

¹H NMR (DMSO-*d*₆): δ ppm 1.32 (d, *J*=6.78 Hz, 6 H) 1.41 (s, 18 H) 3.56 (m, 1 H) 7.98 (s, 2 H) 8.16 (s, 1 H) 8.41 (s, 1 H); HPLC-MS (Method A): *m*/*z* = 464 (M+1); Rₜ = 5.0 min.

### Example 26 (General procedure (C))

### 4-[3-Cyano-1-(3,5-di-tert-butyl-4-hydroxy-phenyl)-prop-1-ene-2-sulfonyl]-N,N-bis-(2-hydroxy-ethyl)-benzamide

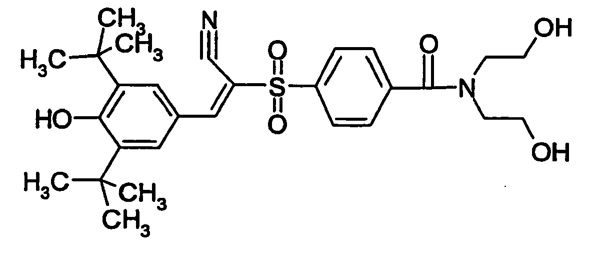

Step A: The title compound was prepared from (E)-4-[3-Cyano-1-(3,5-di-tert-butyl-4-hydroxyphenyl)-prop-1-ene-2-sulfonyl]-benzoic acid and diethanolamine in 14 % yield.

¹H (400 MHz, CHLOROFORM-D) δ ppm 1.47 (s, 18 H) 3.42 (m, 2 H) 3.74 (m, 4 H) 4.04 (m, 2 H) 6.02 (s, 1 H) 7.74 (d, *J*=8.59 Hz, 2 H) 7.84 (s, 2 H) 8.04 (d, *J*=8.59 Hz, 2 H) 8.14 (s, 1H); HPLC-MS (Method A): *m*/*z* = 529 (M+1); Rₜ = 4.10 min.

### Example 27 (General procedure (C))

### 3-(3,5-Di-tert-butyl-4-hydroxy-phenyl)-2-[4-(3,5-dimethyl-morpholine-4-carbonyl)-benzenesulfonyl]-acrylonitrile.

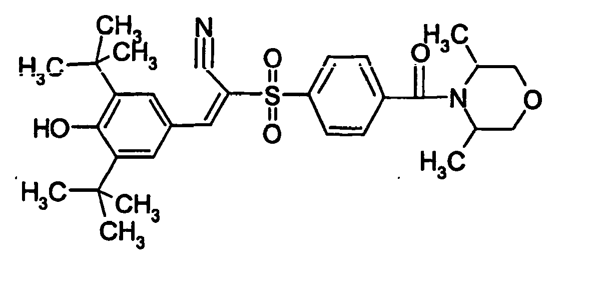

Step A: The title compound was prepared from (E)-4-[3-Cyano-1-(3,5-di-tert-butyl-4-hydroxyphenyl)-prop-1-ene-2-sulfonyl]-benzoic acid and 3,5-dimethyl-morpholine in 44 % yield.

¹H (400 MHz, CHLOROFORM-D) δ ppm 1.15 (m, 6 H) 1.45 (s, 18 H) 2.58 (m, 1 H) 2.84 (m, 1 H) 3.53 (m, 4 H) 6.03 (s, 1 H) 7.59 (d, *J*=8.59 Hz, 2 H) 7.84 (s, 2 H) 8.07 (d, *J*=8.59 Hz, 2 H) 8.14 (s, 1 H); HPLC-MS (Method A): *m*/*z* = 539 (M+1); Rₜ = 5.21 min.

### Example 28 (General procedure (A))

### 3-(3,5-Di-tert-butyl-hydroxy-phenyl)-2-(2,5-dichloro-benzenesulfonyl)-acrylonitrile

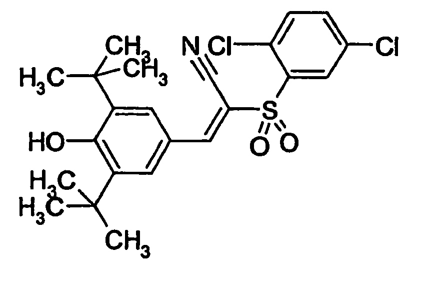

Step A: The title compound was prepared from 3,5-di-tert-butyl-4-hydroxybenzaldehyde and 2,5-dichlorophenylsulfonylacetonitrile in 68 % yield.

¹H NMR (DMSO-*d*₆): δ ppm 1.39 (s, 18 H) 7.82 (d, *J*=8.29 Hz, 1 H) 7.93 (dd, 1 H) 8.00 (s, 2 H) 8.16 (d, z=2.26 Hz, 1 H) 8.50 (s, 1 H) 8.58 (m, 1 H); HPLC-MS (Method A): *m*/*z* = 465, 467 (M+1); Rₜ = 6.0 min.

### Example 29 (General procedure (A))

### 2-(4-tert-Butyl-benzenesulfonyl)-3-(3,5-di-tert-butyl-4-hydroxy-phenyl)-acrylonitrile

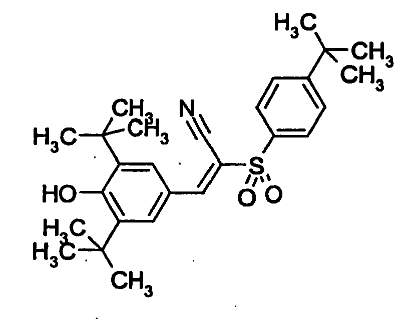

Step A: The title compound was prepared from 3,5-di-tert-butyl-4-hydroxybenzaldehyde and 4-tert-butylphenylsulfonylacetonitrile in 80 % yield

¹H NMR (DMSO-*d*₆): δ ppm 1.31 (s, 9 H) 1.38 (s, 18 H) 7.73 (d, *J*=8.67 Hz, 2 H) 7.91 (d, *J*=8.67 Hz, 2 H) 7.94 (s, 2 H) 8.41 (s, 2 H); HPLC-MS (Method A): *m*/*z*= 455 (M+1); Rₜ = 6.3 min.

### Example 30 (General procedure (A))

### 3-(3,5-Di-tert-butyl-4-hydroxy-phenyl)-2-(naphthalene-1-sulfonyl)-acrylonitrile

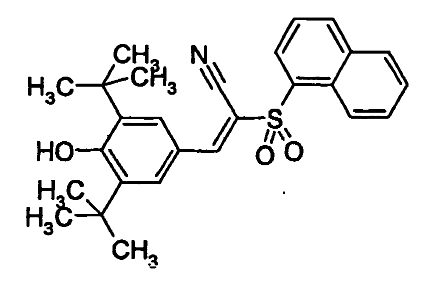

Step A: The title compound was prepared from 3,5-di-tert-butyl-4-hydroxybenzaldehyde and naphthalene-1-sulfonylacetonitrile in 56 % yield

¹H NMR (DMSO-*d*₆): δ ppm 1.38 (s, 18 H) 7.77 (m, 2 H) 7.94 (m, 3 H) 8.11 (d, *J*=7.91 Hz, 1 H) 8.26 (m, 2 H) 8.43 (s, 1 H) 8.49 (s, 1 H) 8.72 (d, *J*=1.51 Hz, 1 H); HPLC-MS (Method A): *m*/*z* = 449 (M+1);Rₜ= 6.3 min.

### Example 31 (General procedure (A))

### 3-(3,5-Di-tert-butyl-4-hydroxy-phenyl)-2-(furan-2-ylmethanesulfonyl)-acrylonitrile

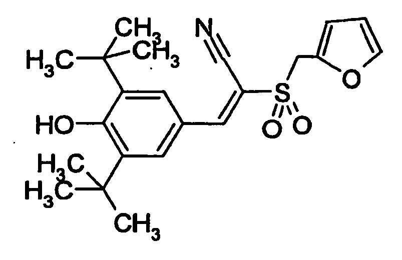

Step A: The title compound was prepared from 3,5-di-tert-butyl-4-hydroxybenzaldehyde and 2-(2-furylmethyl)sulfonyl)acetonitrile in 93 % yield

¹H NMR (DMSO-*d*₆): δ ppm 1.40 (s, 18 H) 4.95 (s, 2 H) 6.50 (m, 1 H) 6.55 (m, 1 H) 7.76 (m, 2 H) 7.74 (s, 1 H) 7.89 (s, 2 H) 8.04 (s, 1 H) 8.44 (s, 1 H); HPLC-MS (Method A): *m*/*z* = 403 (M+1); Rₜ = 5.3 min.

### Example 32 (General procedure (A))

### 3-(3,5-Di-tert-butyl-4-hydroxy-phenyl)-2-(2,4-dichloro-benzenesulfonyl)-acrylonitrile

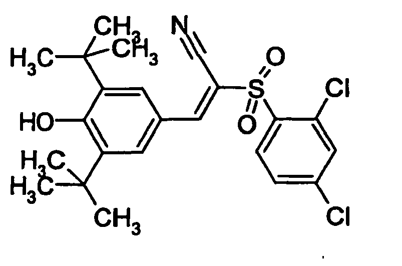

Step A: The title compound was prepared from 3,5-di-tert-butyl-4-hydroxybenzaldehyde and 3,5-dichlorophenylsulfonylacetonitrile in 68 % yield.

¹H NMR (DMSO-*d*₆): δ ppm 1.39 (s, 18 H) 7.82 (dd, *J*=8.48, 2.07 Hz, 1 H) 8.01 (m, 3 H) 8.21 (d, *J*=8.67 Hz, 1 H) 8.48 (s, 1 H) 8.55 (s, 1 H); HPLC-MS (Method A): *m*/*z* = 467 (M+1); Rₜ = 6.1 min.

### Example 33 (General procedure (A))

### 3-(3,5-Di-tert-butyl-4-hydroxy-phenyl)-2-(toluene-4-sulfonyl)-acrylonitrile.

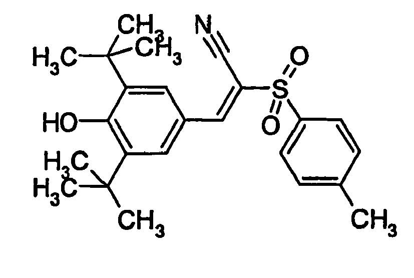

Step A: The title compound was prepared from 3,5-di-tert-butyl-4-hydroxybenzaldehyde and 4-methylphenylsulfonylacetonitrile in 70 % yield.

¹H NMR (DMSO-*d*₆): δ ppm 1.38 (s, 18 H) 2.43 (s, 3 H) 7.52 (d, *J*=8.29 Hz, 2 H) 7.87 (d, *J*=8.29 Hz, 2 H) 7.93 (s, 2 H) 8.41 (s, 2 H); HPLC-MS (Method A): *m*/*z* = 413 (M+1); Rₜ = 5.8 min.

### Example 34 (General procedure (A))

### 3-(3,5-Di-tert-butyl-4-hydroxy-phenyl)-2-(4-isopropyl-benzenesulfonyl)-acrylonitrile

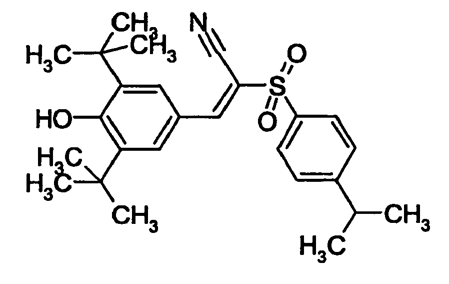

Step A: The title compound was prepared from 3,5-di-tert-butyl-4-hydroxybenzaldehyde and 4-isopropylphenylsulfonylacetonitrile in 75 % yield.

¹H NMR (DMSO-*d₆*): δ ppm 1.22 (d, *J*=6.78 Hz, 6 H) 1.38 (s, 18 H) 3.01 (m, *J*=13.94, 6.78 Hz, 1 H) 7.59 (d, *J*=8.29 Hz, 2 H) 7.90 (d, *J*=8.29 Hz, 2 H) 7.94 (s, 2 H) 8.41 (m, 2 H); HPLC-MS (Method A): *m*/*z* = 441 (M+1); Rₜ = 6.1 min.

### Example 35 (General procedure (A))

### 2-(3,5-Di-tert-butyl-4-hydroxy-benzenesulfonyl)-3-(3,5-di-tert-butyl-4-hydroxyphenyl)-acrylonitrile.

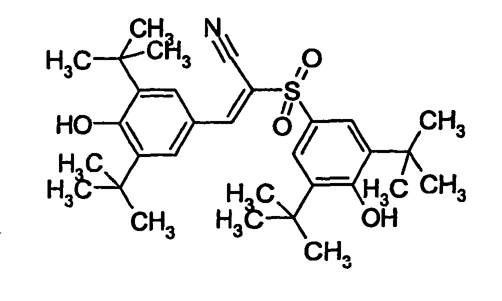

Step A: The title compound was prepared from 3,5-di-tert-butyl-4-hydroxybenzaldehyde and (3,5-Di-tert-butyl-4-hydroxy-benzenesulfonyl)-acetonitrile in 80 % yield

¹H NMR (DMSO-*d*₆): δ ppm 1.38 (s, 18 H) 1.41 (s, 18 H) 7.70 (s, 2 H) 7.92 (s, 2 H) 8.32 (s, 1 H) 8.33 (s, 2 H); HPLC-MS (Method A): *m*/*z*= 527 (M+1); Rₜ = 6.8 min.

### Example 36 (General procedure (A))

### 3-(3,5-Di-tert-butyl-4-hydroxy-phenyl)-2-(hexane-1-sulfonyl)-acrylonitrile.

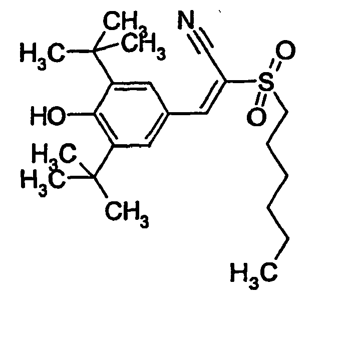

Step A: The title compound was prepared from 3,5-di-tert-butyl-4-hydroxybenzaldehyde and hexane-1-sulfonylacetonitrile in 68 % yield

¹H NMR (DMSO-*d*₆): δ ppm 0.85 (t 3 H) 1.26 (m, 4 H) 1.40 (m, 2 H) 1.40 (s, 18 H) 1.69 (m, 2 H) 3.40 (m, 2 H) 7.95 (s, 2 H) 8.17 (s, 1 H) 8.40 (s, 1 H); HPLC-MS (Method A): *m*/*z* = 407 (M+1); Rₜ = 6.4 min.

### Example 37 (General procedure (A))

### 2-(4-Bromo-benzenesulfonyl)3-(3,5-di-tert-butyl-4-hydroxy-phenyl)-acrylonitrile

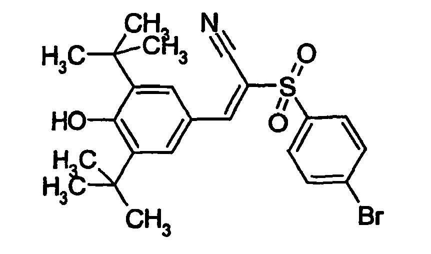

Step A: The title compound was prepared from 3,5-di-tert-butyl-4-hydroxybenzaldehyde and 4-bromophenylsulfonylacetonitrile in 53 % yield.

### ¹H NMR (DMSO-d₆): δ ppm 1.38 (s, 18 H) 7.93 (d, J=4.52 Hz, 6 H) 8.41 (s, 1 H) 8.46 (m, 1 H); HPLC-MS (Method A): m/z = 475, 477 (M+1); Rₜ = 5.7 min.

### Example 38 (General procedure (A))

### 3-(3,5-Di-tert-butyl-4-hydroxy-phenyl)-2-(4-methanesulonyl-benzenesulfonyl)-acrylonitrile

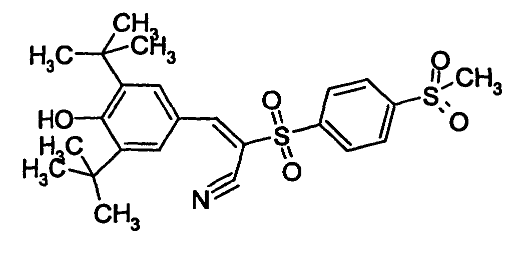

Step A: The title compound was prepared from 3,5-di-tert-butyl-4-hydroxybenzaldehyde and 4-methanesulfonylphenylsulfonylacetonitrile in 90 % yield.

¹H NMR (DMSO-*d*₆): δ ppm 1.39 (s, 18 H) 3.38 (s, 3 H) 7.98 (s, 2 H) 8.26 (s, 4 H) 8.51 (s, 1 H) 8.62 (m, 1 H).

### Example 39 (General procedure (A))

### 3-(3,5-Di-tert-butyl-4-hydroxy-phenyl)-2-(3-trifluoromethoxy-benzenesulfonyl)-acrylonitrile

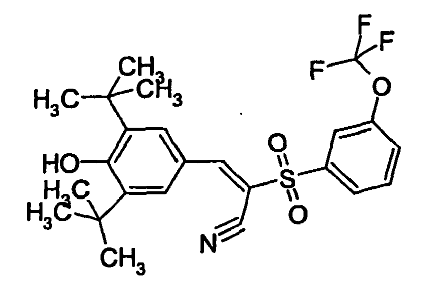

Step A: The title compound was prepared from 3,5-di-tert-butyl-4-hydroxybenzaldehyde and 3-trifluoromethoxyphenylsulfonylacetonitrile in 83 % yield.

¹H NMR (DMSO-*d*₆): δ ppm 1.39 (s, 18 H) 7.88 (m, *J*=6.03 Hz, 2 H) 7.96 (m, 3 H) 8.05 (m, 1 H) 8.48 (s, 1 H) 8.56 (m, 1 H).

### Example 40 (General procedure (A))

### 3-(3,5-Di-tert-butyl-4-hydroxy-phenyl)-2-(2-trifluoromethoxy-benzenesulfonyl)-acrylonitrile.

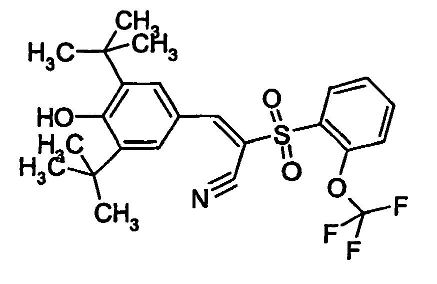

Step A: The title compound was prepared from 3,5-di-tert-butyl-4-hydroxybenzaldehyde and 2-trifluoromethoxyphenylsulfonylacetonitrile in 75 % yield.

¹H NMR (DMSO-*d*₆): δ ppm 1.39 (s, 18 H) 7.72 (m, 2 H) 7.96 (m, 3 H) 8.19 (dd, *J*=7.91, 1.88 Hz, 1 H) 8.38 (s, 1 H) 8.57 (m, 1 H).

### Example 41 (General procedure (A))

### 2-(5-Chloro-pyridine-2-sulfonyl)-3-(3,5-di-tert-butyl-4-hydroxy-phenyl)-acrylonitrile

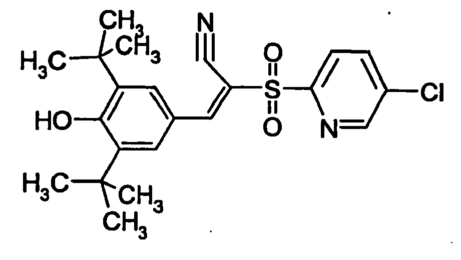

Step A: The title compound was prepared from 3,5-di-tert-butyl-4-hydroxybenzaldehyde and (5-Chloro-pyridine-2-sulfonyl)-acetonitrile in 13 % yield.

¹H NMR (DMSO-*d*₆): δ ppm 1.47 (s, 18 H) 6.05 (s, 1 H) 7.89 (s, 2 H) 7.97 (dd, *J*=8.34, 2.27 Hz, 1 H) 8.19 (d, *J*=8.08 Hz, 1 H) 8.25 (s, 1 H) 8.68 (d, *J*=2.02 Hz, 1 H); HPLC-MS (Method A): *m*/*z* = 432, 434 (M+1); Rₜ = 5.19 min.

### Example 42 (General procedure (A))

### 2-[3-Cyano-1-(3,5-di-tert-butyl-4-hydroxy-phenyl)-prop-1-ene-2-sulfonyl]-benzoic acid

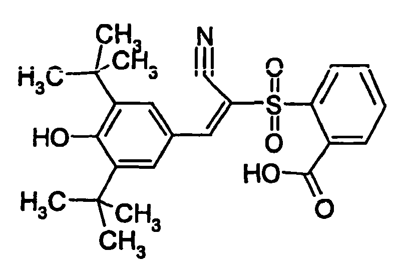

Step A: The title compound was prepared from 3,5-di-tert-butyl-4-hydroxybenzaldehyde and 2-carboxyphenylsulfonylacetonitrile in 95 % yield.

¹H NMR (DMSO-*d*₆): δ ppm 1.40 (s, 18 H) 7.77 (d, *J*=7.58 Hz, 1 H) 7.87 (m, 4 H) 8.15 (d, *J*=7.58 Hz, 1 H) 8.20 (s, 1 H) 8.46 (s, 1 H) 13.87 (m, 1 H); HPLC-MS (Method A): *m*/*z* = 464 (M+23); Rₜ = 4.2 min.

### Example 43 (General procedure (A))

### 3-[3-Cyano-1-(3,5-di-tert-butyl-4-hydroxy-phenyl)-prop-1-ene-2-sulfonyl]-benzoic acid

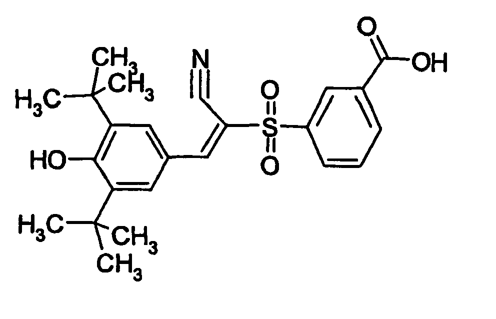

Step A: The title compound was prepared from 3,5-di-tert-butyl-4-hydroxybenzaldehyde and 3-carboxyphenylsulfonylacetonitrile in 83 % yield.

¹H NMR (DMSO-*d*₆): δ ppm 1.39 (s, 18 H) 7.88 (t, *J*=7.83 Hz, 1 H) 7.97 (s, 2 H) 8.24 (d, *J*=7.58 Hz, 1 H) 8.33 (d, *J*=7.58 Hz, 1 H) 8.45 (s, 1 H) 8.49 (s, 2 H) 13.66 (m, 1 H); HPLC-MS (Method A): *m*/*z* = 442 (M+1); Rₜ = 4.4 min.

### Example 44 (General procedure (C))

### 3-(3,5-Di-tert-butyl-4-hydroxy-phenyl)-2-[2-(morpholine-4-carbonyl)-benzenesulfonyl]-acrylonitrile.

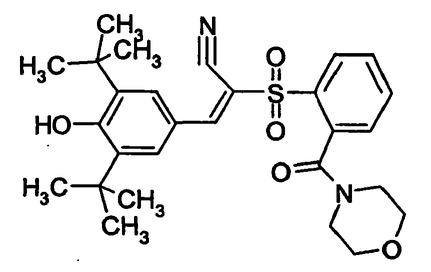

Step A: The title compound was prepared from (E)-2-[3-Cyano-1-(3,5-di-tert-butyl-4-hydroxyphenyl)-prop-1-ene-2-suffonyl]-benzoic acid and morpholine in 91 % yield.

¹H NMR (DMSO-*d*₆): δ ppm 1.39 (s, 18 H) 3.12 (m, 2 H) 3.60 (m, 6 H) 7.55 (d, *J*=7.07 Hz, 1 H) 7.78 (t, *J*=7.33 Hz, 1 H) 7.86 (m, *J*=7.58 Hz, 3 H) 8.11 (d, *J*=8.08 Hz, 1 H) 8.19 (s, 1 H) 8.46 (s, 1 H).

### Example 45 (General procedure (C))

### 3-(3,5-Di-tert-butyl-4-hydroxy-phenyl)-2-[3-(morpholine-4-carbonyl)-benzenesulfonyl]-acrylonitrile

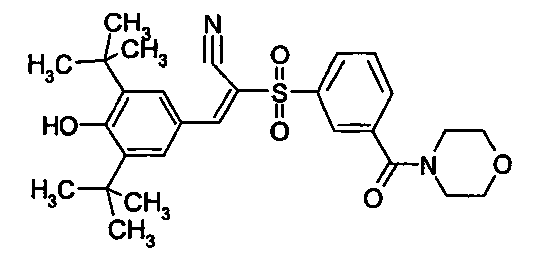

Step A: The title compound was prepared from (E)-3-[3-Cyano-1-(3.5-di-tert-butyl-4-hydroxyphenyl)-prop-1-ene-2-sulfonyl]-benzoic acid and morpholine in 91 % yield.

¹H NMR (DMSO-*d₆*): δ ppm 1.39 (s, 18 H) 3.26 (m, 2 H) 3.52 (m, 2 H) 3.65 (m, 4 H) 7.82 (m, 2 H) 7.98 (m, 3 H) 8.08 (d, *J*=7.58 Hz, 1 H) 8.48 (s, 2 H).

### Example 46 (General procedure (A))

### 3-(3,5-Di-tert-butyl-4-hydroxy-phenyl)-2-(4-methoxy-benzenesulfonyl)-acrylonitrile

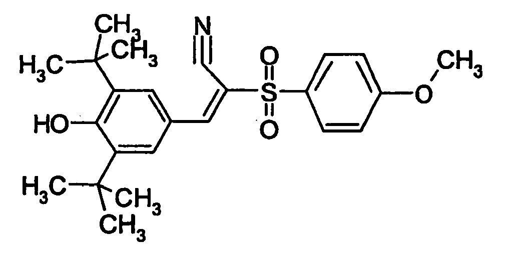

Step A: The title compound was prepared from 3,5-di-tert-butyl-4-hydroxybenzaldehyde and 4-methoxyphenylsulfonylacetonitrile in 42 % yield.

¹H NMR (DMSO-*d₆*): δ ppm 1.38 (s, 18 H) 3.88 (s, 3 H) 7.91 (d, *J*=9.10 Hz, 2 H) 7.92 (s, 2 H) 8.37 (s, 2 H); HPLC-MS (Method A): *m*/*z* = 428 (M+1); Rₜ = 5.8 min.

### Example 47 (General procedure (A))

### 3-(3,5-Di-tert-butyl-4-hydroxy-phenyl)-2-(3-methoxy-benzenesulfonyl)-acrylonitrile

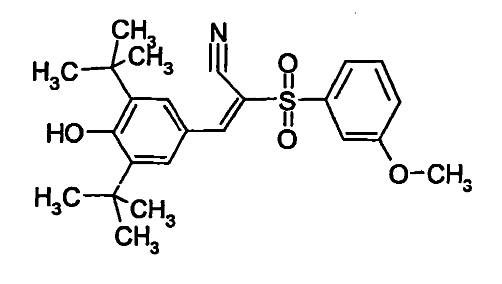

Step A: The title compound was prepared from 3,5-di-tert-butyl-4-hydroxybenzaldehyde and 3-methoxyphenylsulfonylacetonitrile in 39 % yield

¹H NMR (DMSO-*d*₆): δ ppm 1.38 (m, 18 H) 3.86 (m, 3 H) 7.38 (dd, *J*=7.83, 2.27 Hz, 1 H) 7.44 (s, 1 H) 7.56 (d, *J*=8.59 Hz, 1 H) 7.65 (t, *J*=7.83 Hz, 1 H) 7.95 (s, 2 H) 8.44 (s, 1 H) 8.44 (s, 1 H); HPLC-MS (Method A): *m*/*z* = 428 (M+1); Rₜ = 5.8 min.

### Example 48 (General procedure (A))

### 3-(3,5-Di-tert-butyl-4-hydroxy-phenyl)-2-(3,4-dimethoxy-benzenesulfonyl)-acrylonitrile

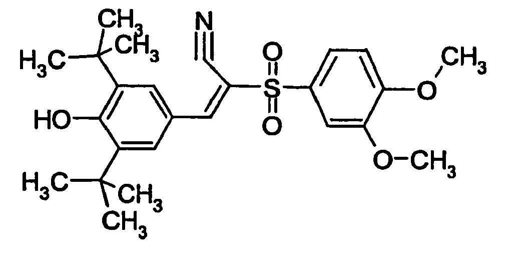

Step A: The title compound was prepared from 3,5-di-tert-butyl-4-hydroxybenzaldehyde and 3,4-dimethoxyphenylsulfonylacetonitrile in 44 % yield.

¹H NMR (DMSO-*d*₆): δ ppm 1.41 (s, 18 H) 7.72 (d, *J*=8.08 Hz, 2 H) 7.96 (s, 2 H) 8.14 (d, *J*=9.10 Hz, 2 H) 8.46 (s, 1 H) 8.50 (s, 1 H); HPLC-MS (Method A): *m*/*z* = 458 (M+1); Rₜ = 5.5 min.

### Example 49 (General procedure (D))

### 3-(3,5-Di-tert-butyl-4-hydroxy-phenyl)-2-[4-(2,3-dihydro-furan-2-yl)-benzenesulfonyl]-acrylonitrile

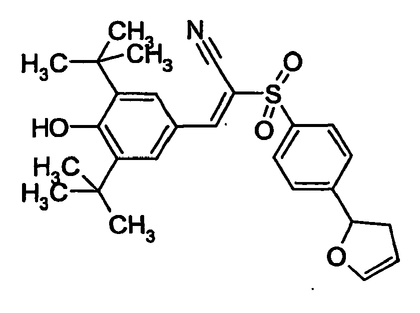

Step A: The title compound was prepared from 2-(4-Bromo-benzenesulfonyl)-3-(3,5-di-tert-butyl-4-hydroxy-phenyl)-acrylonitrile and 2,3-dihydro-furan in 18 % yield

¹H NMR (DMSO-*d*₆): δ ppm 1.38 (s, 18 H) 2.44 (m, 1 H) 3.13 (m, 1 H) 5.01 (d, *J*=2.02 Hz, 1 H) 5.67 (dd, *J*=10.86, 7.83 Hz, 1 H) 6.63 (s, 1 H) 7.65 (d, *J*=8.08 Hz, 2 H) 7.94 (s, 2 H) 7.99 (d, *J*=8.08 Hz, 2 H) 8.41 (s, 1 H) 8.44 (s, 1 H); HPLC-MS (Method A): *m*/*z*= 467 (M+1); Rₜ = 5.6 min.

### Example 50 (General procedure (D))

### 3-{4-[2-Cyano-1-(3,5-di-tert-butyl-4-hydroxy-phenyl)-ethenesulfonyl]-phenyl}-2-methyl-acrylic acid methyl ester

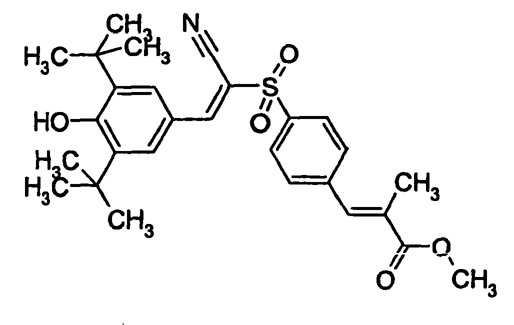

Step A: The title compound was prepared from 2-(4-Bromo-benzenesulfonyl)-3-(3,5-di-tert-butyl-4-hydroxy-phenyl)-acrylonitrile and methyl metacrylate in 10 % yield

¹H NMR (400 MHz, CHLOROFORM-D): Mixture of Z and E isomers: δ ppm 1.45 (s, 36H)1.61 (s,3H)2.11 (s, 3 H) 3.73 (s, 3 H) 3.84 (s, 3 H) 5.57 (s, 1 H) 5.98 (s, 1 H) 6.01 (s, 1 H) 6.30 (s, 1 H) 7.42 (d, *J*=8.08 Hz, 2 H) 7.56 (d, *J*=8.08 Hz, 2 H) 7.83 (s, 2 H) 7.85 (s, 2 H) 7.92 (d, *J*=8.59 Hz, 2 H) 8.02 (d, *J*=8.59 Hz, 2 H) 8.12 (s, 1 H) 8.14 (s, 1 H); HPLC-MS (Method A): *m*/*z* = 497 (M+1); Rₜ = 5.5 and 5.7 min.

### Example 51 (General procedure (D))

### 3-(3,5-Di-tert-butyl-4-hydroxy-phenyl)-2-(4-styryl-benzenesulfonyl)-acrylonitrile

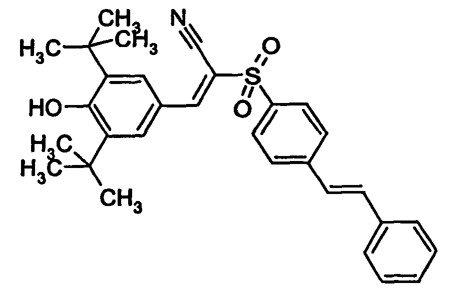

Step A: The title compound was prepared from 2-(4-Bromo-benzenesulfonyl)-3-(3,5-di-tert-butyl-4-hydroxy-phenyl)-acrylonitrile and styrene in 23 % yield

¹H NMR (DMSO-*d*₆): δ ppm 1.40 (s, 18 H) 7.39 (m, 5 H) 7.67 (d, 2 H) 7.94 (m, 6 H) 8.43 (m, 1 H) 8.45 (m, 1 H); HPLC-MS (Method A): *m*/*z* = 500 (M+1); Rₜ = 6.1 min.

### Example 52 (General procedure (D))

### 3-(3,5-Di-tert-butyl-4-hydroxy-phenyl)-2-[4-(2-isobutoxy-vinyl)-benzenesulfonyl]-acrylonitrile.

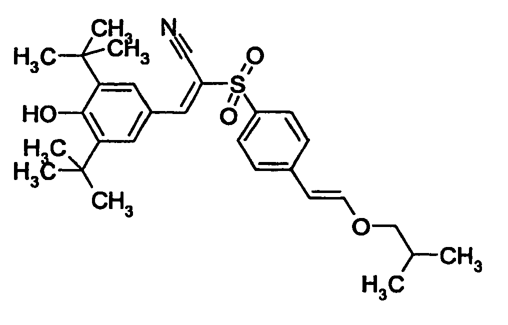

Step A: The title compound was prepared from 2-(4-Bromo-benzenesulfonyl)-3-(3,5-di-tert-butyl-4-hydroxy-phenyl)-acrylonitrile and isobutyl vinyl ether in 5 % yield.

¹H NMR (DMSO-*d₆*): δ ppm 0.93 (d, *J*=6.41 Hz, 6 H) 1.38 (s, 18 H) 1.95 (m, 1 H) 3.70 (d, *J*=6.41 Hz, 2 H) 5.96 (d, *J*=12.81 Hz, 1 H) 7.49 (d, 1 H) 7.58 (d, *J*=7.91 Hz, 2 H) 7.80 (d, *J*=7.91 Hz, 2 H) 7.92 (s, 1 H) 8.37 (s, 1 H) 8.46 (m, 1 H); HPLC-MS (Method A): *m*/*z* = 497 (M+1); Rₜ = 6.1 min.

### Example 53 (General procedure (D))

### 2-[4-(2-Butoxy-vinyl)-benzenesulfonyl]-3-(3,5-di-tert-butyl-4-hydroxy-phenyl)-acrylonitrile.

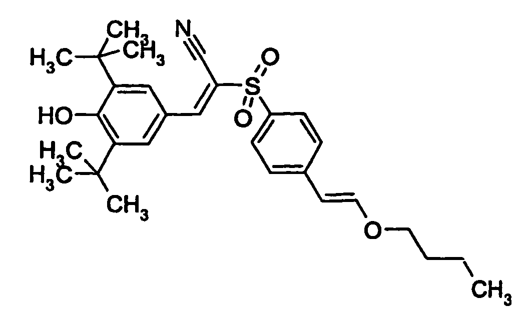

Step A: The title compound was prepared from 2-(4-Bromo-benzenesulfonyl)-3-(3,5-di-tert-butyl-4-hydroxy-phenyl)-acrylonitrile and butyl vinyl ether in 34 % yield.

¹H NMR (300 MHz, CHLOROFORM-D) δ ppm 0.96 (t, 3 H) 1.45 (s, 18 H) 1.69 (m, 4 H) 3.88 (t, *J*=6.41 Hz, 2 H) 5.83 (d, *J*=13.19 Hz, 1 H) 5.96 (s, 1 H) 7.16 (d, *J*=12.81 Hz, 1 H) 7.36 (d, *J*=8.29 Hz, 2 H) 7.84 (m, 4 H) 8.10 (s, 1 H); HPLC-MS (Method A): *m*/*z* = 497 (M+1); Rₜ = 6.1 min.

### Example 54 (General procedure (D))

### 3-(3,5-Di-tert-butyl-4-hydroxy-phenyl)-2-(4-phenylethynyl-benzenesulfonyl)-acrylonitrile

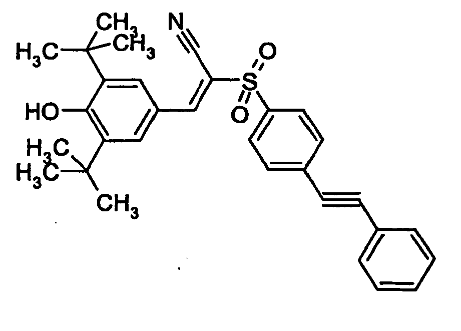

Step A: The title compound was prepared from 2-(4-Bromo-benzenesulfonyl)-3-(3,5-di-tert-butyl-4-hydroxy-phenyl)-acrylonitrile and phenylacetylene in 9 % yield.

### Example 55 (General procedure (A))

### 3-(3,5-Di-tert-butyl-4-hydroxy-phenyl)-2-(2-ethoxy-ethanesulfonyl)-acrylonitrile

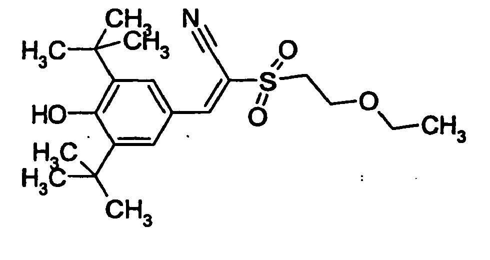

Step A: The title compound was prepared from 3,5-di-tert-butyl-4-hydroxybenzaldehyde and ethoxyethylsulfonylacetonitrile in 29 % yield.

¹H NMR (DMSO-*d*₆): δ ppm 1.00 (t, *J*=6.97 Hz, 3 H) 1.40 (s, 18 H) 3.40 (q, 2 H) 3.63 - 3.71 (m, 2 H) 3.72 - 3.78 (m, 2 H) 7.93 (s, 2 H) 8.14 (s, 1 H) 8.35 (s, 1 H)); HPLC-MS (Method A): *m*/*z* = 395 (M+1); Rₜ = 5.0 min.

### Example 56 (General procedure (D))

### 3-(3,5-Di-tert-butyl-4-hydroxy-phenyl)-2-[2-(2-ethoxy-ethoxy)-ethanesulfonyl]-acrylonitrile.

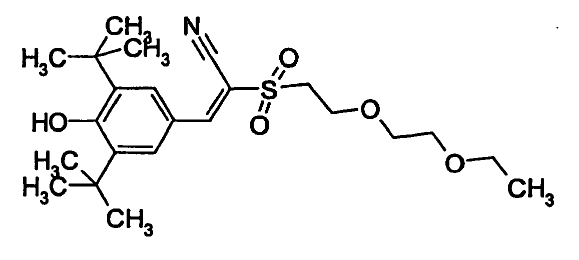

Step A: The title compound was prepared from 3,5-di-tert-butyl-4-hydroxybenzaldehyde and 2-(2-ethoxy-ethoxy)-ethanesulfonylacetonitrile in 77 % yield

¹H NMR (DMSO-*d*₆): *δ* ppm 0.93 (t, *J*=6.97 Hz, 3 H) 1.40 (s, 18 H) 3.22 (q, *J*=6.78 Hz. 2 H) 3.32 (t, 2 H) 3.44 - 3.50 (t, 2 H) 3.69 (t. *J*=4.90 Hz, 1 H) 3.80 (t, *J*=4.90 Hz, 2 H) 7.93 (s, 2 H) 8.14 (s, 1 H) 8.36 (s, 1 H)); HPLC-MS (Method A): *m*/*z* = 439 (M+1); Rₜ = 4.9 min.

### Example 57 (General procedure (D))

### 2-(4-Chloro-benzenesulfonyl)-3-(3,5-di-sec-butyl-4-hydroxy-phenyl)-acrylonitrile

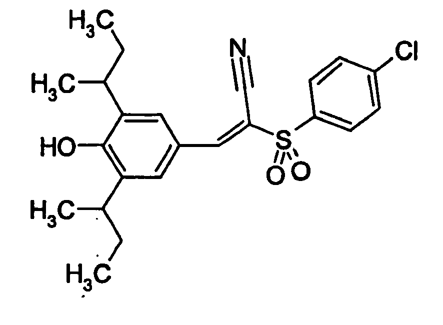

Step A: The title compound was prepared from 3,5-di-sec-butyl-4-hydroxybenzaldehyde and 4-Chloro-benzenesulfonylacetonitrile in 86 % yield

¹H NMR (DMSO-*d₆*): *δ* ppm 0.78 (t, 6 H) 1.13 (d, *J*=6.78 Hz, 6 H) 1.43 -1.67 (m, 4 H) 3.02 - 3.20 (m, 2 H) 7.81 (d, *J*=4.14 Hz, 2 H) 7.79 (s, 2 H) 8.00 (d, *J*=8.67 Hz, 2 H) 8.39 (s, 1 H) 9.69 (s, 1 H); HPLC-MS (Method A): *m*/*z* = 432, 434 (M+1); Rₜ = 5.5 min.

### Example 58 (General procedure (A))

### (E)-2-(3-Bromo-benzenesulfonyl)-3-(3,5-di-tert-butyl-4-hydroxy-phenyl)-acrylonitrile

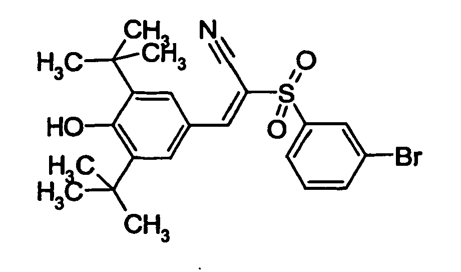

Step A: The title compound was prepared from 3,5-di-tert-butyl-4-hydroxybenzaldehyde and 3-bromo-phenylsulfonylacetonitrile in 72 % yield

¹H NMR (DMSO-*d*₆): δ 1.42 (s, 18 H) 7.69 (t, *J*=7.91 Hz, 1 H) 7.96 (s, 2 H) 7.98 - 8.06 (m, *J*=6.97, 6.97 Hz, 2 H) 8.15 (d, *J*=1.88 Hz, 1 H) 8.47 (s, 1 H) 8.49 - 8.56 (s, 1 H); HPLC-MS (Method A): *m*/*z* = 477, 479 (M+1); Rₜ = 5.7 min.

### Example 59 (General procedure (D))

### 3-(3,5-Di-tert-butyl-4-hydroxy-phenyl)-2-(hex-5-ene-1-sulfonyl)-acrylonitrile

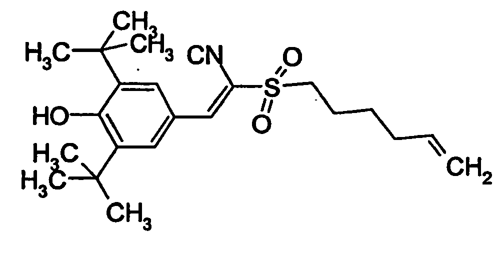

Step A: The title compound was prepared from 3,5-di-tert-butyl-4-hydroxybenzaldehyde and hex-5-enesulfonylacetonitrile in 48 % yield

¹H NMR (DMSO-*d*₆): δ ppm 1.40 (s, 18 H) 1.45 -1.56 (m, 2 H) 1.63 - 1.78 (m, 2 H) 2.05 (q, *J*=6.91 Hz, 2 H) 3.38 - 3.47 (m, 2 H) 4.91 - 5.07 (m, 2 H) 5.68 - 5.86 (m, 1 H) 7.95 (s, 2 H) 8.17 (s, 1 H) 8.40 (s, 1 H); HPLC-MS (Method A): *m*/*z* = 405 (M+1); Rₜ = 5.5 min.

### Example 60 (General procedure (A))

### (E)-2-(4-Iodo-benzenesulfonyl)-3-(3,5-di-tert-butyl-4-hydroxy-phenyl)-acrylonitrile

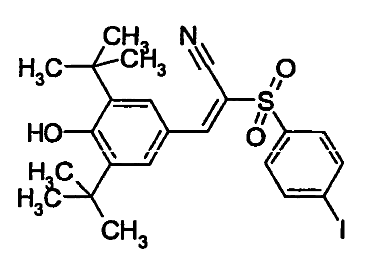

Step A: The title compound was prepared from 3,5-di-tert-butyl-4-hydroxybenzaldehyde and 4-iodo-phenylsulfonylacetonitrile in 79 % yield

¹H NMR (DMSO-*d*₆): δ 1.42 (s, 18 H) 7.73 (d, *J*=8.67 Hz, 2 H) 7.95 (s, 2 H) 8.12 (d, *J*=8.67 Hz, 2 H) 8.44 (s, 1 H) 8.48 (s, 1 H); HPLC-MS (Method A): *m*/*z* = 524 (M+1); Rₜ = 5.7 min.

### Example 61 (General procedure (A))

### (E)-2-Cyclopentanesulfonyl-3-(3,5-di-tert-butyl-4-hydroxy-phenyl)-acrylonitrile

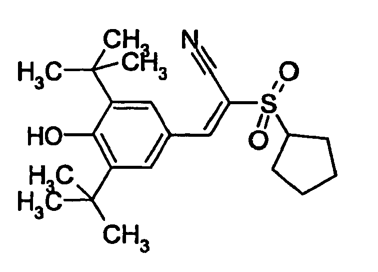

Step A: The title compound was prepared from 3,5-di-tert-butyl-4-hydroxybenzaldehyde and cyclopentanesulfonylacetonitrile in 66 % yield

¹H NMR (DMSO-*d*₆): δ 1.46 (s, 18 H) 1.56 - 1.77 (m, 4 H) 1.88 - 2.07 (m, 4 H) 3.81 - 3.95 (m, 1 H) 7.97 (s, 2 H) 8.19 (s, 1 H) 8.40 (s, 1 H); HPLC-MS (Method A): *m*/*z*= 391 (M+1); Rₜ = 5.3 min.

### Example 62 (General procedure (A))

### (E)-3-(3,5-Di-tert-butyl-4-hydroxy-phenyl)-2-(nonane-1-sulfonyl)-acrylonitrile

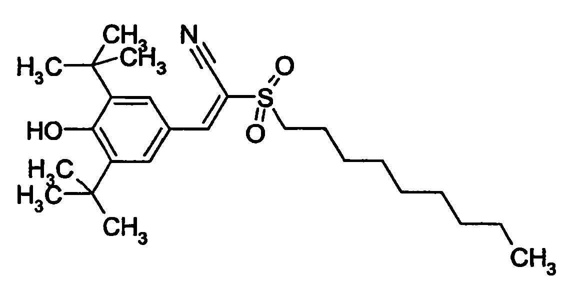

Step A: The title compound was prepared from 3,5-di-tert-butyl-4-hydroxybenzaldehyde and nonane-1-sulfonylacetonitrile in 87 % yield

¹H NMR (DMSO-*d*₆): δ 0.83 (t, 3 H) 1.17 - 1.31 (m, 12 H) 1.42 (s, 18 H) 1.62 -1.76 (m, 2 H) 3.39 (t, 2 H) 7.94 (s, 2 H) 8.13 (s, 1 H) 8.34 - 8.49 (m, 1 H); HPLC-MS (Method A): *m*/*z* = 448 (M+1); Rₜ = 5.30 min.

### Example 63 (General procedure (A))

### (E)-3-(3,5-Di-tert-butyl-4-hydroxy-phenyl)-2-(pentane-1-sulfonyl)-acrylonitrile

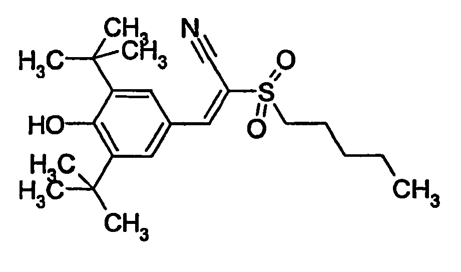

Step A: The title compound was prepared from 3,5-di-tert-butyl-4-hydroxybenzaldehyde and pentane-1-sulfonylacetonitrile in 59 % yield.

¹H NMR (DMSO-*d*₆): δ 0.86 (t, 3 H) 1.25 -1.37 (m, 4 H) 1.44 (s, 18 H) 1.63 -1.77 (m, 2 H) 3.41 (t, 2 H) 7.95 (s, 2 H) 8.17 (s, 1 H) 8.40 (s, 1 H); HPLC-MS (Method A): *miz* = 393 (M+1); Rₜ = 5.5 min.

### Example 64 (General procedure (A))

### (E)-3-(3,5-Di-tert-butyl-4-hydroxy-phenyl)-2-(heptane-1-sulfonyl)-acrylonitrile

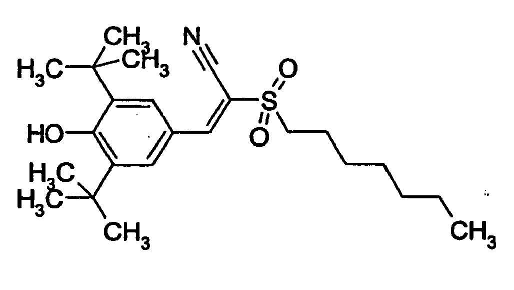

Step A: The title compound was prepared from 3,5-di-tert-butyl-4-hydroxybenzaldehyde and heptane-1-sulfonylacetonitrile in 73 % yield.

¹H NMR (DMSO-*d*₆): δ 0.84 (t, 3 H) 1.17 - 1.35 (m, 8 H) 1.43 (s, 18 H) 1.62 -1.76 (m, 2 H) 3.40 (t, 2 H) 7.96 (s, 2 H) 8.17 (s, 1 H) 8.39 (s, 1 H); HPLC-MS (Method A): *m*/*z* = 421 (M+1); Rₜ = 5.9 min.

### Example 65 (General procedure (A))

### (E)-2-Cyclohexanesulfonyl-3-(3,5-di-tert-butyl-4-hydroxy-phenyl)-acrylonitrile

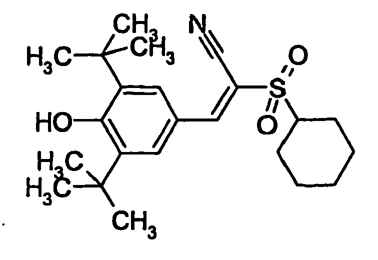

Step A: The title compound was prepared from 3,5-di-tert-butyl-4-hydroxybenzaldehyde and cyclohexanesulfonylacetonitrile in 80 % yield.

¹H NMR (DMSO-*d*₆): δ 1.10 -1.37 (m, 5 H) 1.41 (s, 18 H) 1.58 - 1.67 (m, 1 H) 1.76 - 1.86 (m, 2 H) 2.02 - 2.12 (m, 2 H) 3.28 - 3.32 (m, 1 H) 7.98 (s, 2 H) 8.12 (s, 1 H) 8.41 (s, 1 H); HPLC-MS (Method A): *m*/*z* = 405 (M+1); Rₜ = 5.6 min.

### Example 66 (General procedure (A))

### (E-3-{4-[2-Cyano-1-(3,5-di-tert-butyl-4-hydroxy-phenyl)-ethenesulfonyl]-phenyl}-propionic acid

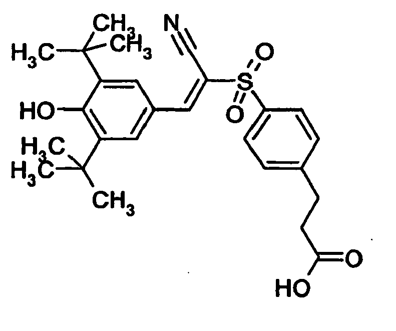

Step A: The title compound was prepared from 3,5-di-tert-butyl-4-hydroxybenzaldehyde and 3-(4-cyanomethylsulfonyl-phenyl)-propionic acid in 92 % yield.

¹H NMR (DMSO-*d*₆): δ 1.38 (s, 18 H) 2.61 (t, *J*=7.54 Hz, 2 H) 2.94 (t, *J*=7.54 Hz, 2 H) 7.58 (d, *J*=8.29 Hz, 2 H) 7.89 (d, *J*=8.29 Hz, 2 H) 7.94 (s, 2 H) 8.40 (s, 1 H) 8.44 (s, 1 H) 12.22 (s, 1 H); HPLC-MS (Method A): *m*/*z* = 471 (M+1); Rₜ = 4.9 min.

### Example 67 (General procedure (A))

### (E)-3-(3,5-Di-tert-butyl-4-hydroxy-phenyl)-2-(4-methyl-2-oxo-2H-chromene-7-sulfonyl)-acrylonitrile

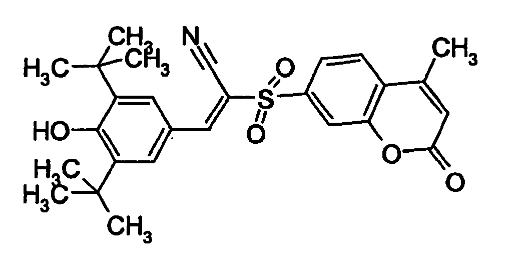

Step A: The title compound was prepared from 3,5-di-tert-butyl-4-hydroxybenzaldehyde and - (4-methyl-2-oxo-2H-chromene-7-sulfonyl)-acetonitrile in 92 % yield.

¹H N MR (DMSO-*d*₆): δ 1.39 (s, 18 H) 2.48 (s, 3 H) 6.64 (s, 1 H) 7.89 - 7.99 (m, 4 H) 8.10 (d, *J*=8.29 Hz, 1 H) 8.51 (s, 1 H) 8.52 (s, 1 H); HPLC-MS (Method A): *m*/*z* = 481 (M+1); Rₜ = 5.2 min.

### Example 68 (General procedure (A))

### (E)-3-(3,5-Di-tert-butyl-4-hydroxy-phenyl)-2-(4-trifluoromethoxyphenylmethanesulfonyl)-acrylonitrile.

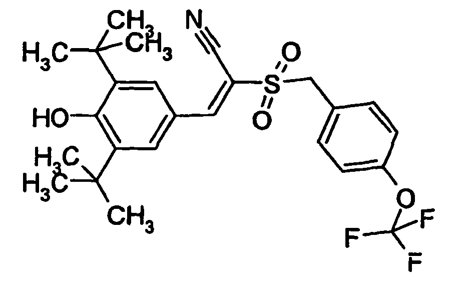

Step A: The title compound was prepared from 3,5-di-tert-butyl-4-hydroxybenzaldehyde and-(4-trifluoromethoxy-phenylmethanesulfonyl)-acetonitrile in 45 % yield.

¹H NMR (DMSO-*d*₆): δ 1.39 (s, 18 H) 4.88 (s, 2 H) 7.41 (d, 2 H) 7.54 (d, 2 H) 7.84 (s, 2 H) 7.96 (s, 1 H) 8.43 (s, 1 H); HPLC-MS (Method A): *m*/*z* = 497 (M+1); Rₜ = 5.6 min.

### Example 69 (General procedure (A))

### (E)-2-Cyclohexylmethanesulfonyl-3-(3,5-di-tert-butyl-4-hydroxy-phenyl)-acrylonitrile

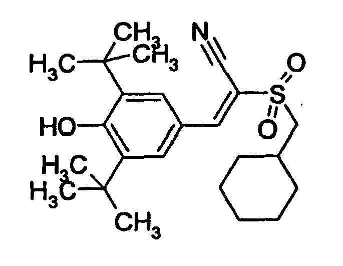

Step A: The title compound was prepared from 3,5-di-tert-butyl-4-hydroxybenzaldehyde and 2-cyclohexanemethanesulfonylacetonitrile in 78 % yield.

¹H NMR (DMSO-*d*₆): δ 1.05 - 1.32 (m, 6 H) 1.41 (s, 18 H) 1.52 -1.73 (m, 3 H) 1.80 1.99 (m, 2 H) 3.31 (d, 2 H) 7.95 (s, 2 H) 8.18 (s, 1 H) 8.38 (s, 1 H); HPLC-MS (Method A): *m*/*z* = 419 (M+1); Rₜ = 5.9 min

### Example 70 (General procedure (A))

### (E)-2-(Butane-1-sulfonyl)-3-(3,5-di-tert-butyl-4-hydroxy-phenyl)-acrylonitrile

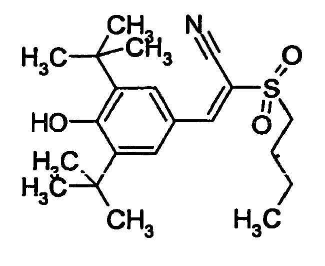

Step A: The title compound was prepared from 3,5-di-tert-butyl-4-hydroxybenzaldehyde and butanesulfonylacetonitrile in 89 % yield.

¹H NMR (DMSO-*d*₆): δ 0.89 (t, *J*=7.35 Hz, 3 H) 1.41 (s, 18 H) 1.42 -1.49 (m, 2 H) 1.61 -1.74 (m, 2 H) 3.37 - 3.45 (m, 2 H) 7.96 (s, 2 H) 8.17 (s, 1 H) 8.40 (s, 1 H); HPLC-MS (Method A): *m*/*z* = 379 (M+1); Rₜ = 5.37 min

### Example 71 (General procedure (A))

### (E)-2-(Propane-1-sulfonyl)-3-(3,5-di-tert-butyl-4-hydroxy-phenyl)-acrylonitrile

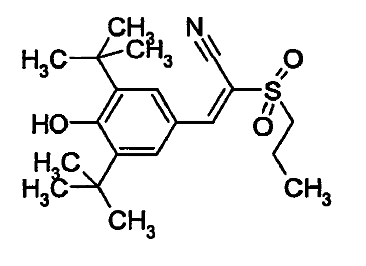

Step A: The title compound was prepared from 3,5-di-tert-butyl-4-hydroxybenzaldehyde and propanesulfonylacetonitrile in 85 % yield.

¹H NMR (DMSO-*d₆*): δ 1.00 (t, *J*=7.35 Hz, 3 H) 1.41 (s, 18 H) 1.67 -1.81 (m, 2 H) 3.36 - 3.44 (m, 2 H) 7.96 (s, 2 H) 8.17 (s, 1 H) 8.40 (s, 1 H); HPLC-MS (Method A): *m*/*z* = 365 (M+1); Rₜ = 5.1 min.

### Example 72 (General procedure (A))

### (E)-2-(Ethane-1-sulfonyl)-3-(3,5-di-tert-butyl-4-hydroxy-phenyl)-acrylonitrile

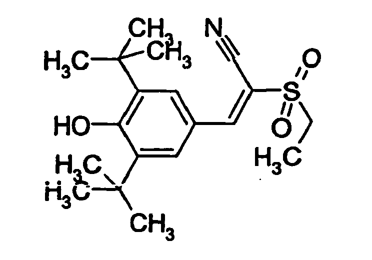

Step A: The title compound was prepared from 3,5-di-tert-butyl-4-hydroxybenzaldehyde and ethanesulfonylacetonitrile in 81 % yield.

¹H NMR (DMSO-*d*₆): δ 1.26 (t, *J*=7.35 Hz, 3 H) 1.40 (s, 18 H) 3.41 (q, *J*=7.54 Hz, 2 H) 7.96 (s, 2 H) 8.15 (s, 1 H) 8.40 (s, 1 H); HPLC-MS (Method A): *m*/*z*= 350 (M+1); Rₜ = 5.19 min.

### Example 73 (General procedure (A))

### (E)-2-Cyclopropylmethanesulfonyl-3-(3,5-di-tert-butyl-4-hydroxy-phenyl)-acrylonitrile

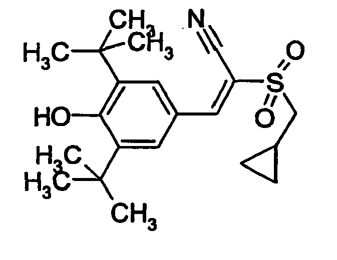

Step A: The title compound was prepared from 3,5-di-tert-butyl-4-hydroxybenzaldehyde and cylopropylmethanesulfonylacetonitrile in 41 % yield.

¹H NMR (DMSO-*d*₆): δ 0.32 - 0.42 (m, 2 H) 0.56 - 0.66 (m, 2 H) 0.98 -1.12 (m, 1 H) 1.40 (s, 18 H) 3.38 (d, *J*=7.16 Hz, 2 H) 7.94 (s, 2 H) 8.15 (s, 1 H) 8.38 (s, 1 H); HPLC-MS (Method A): *m*/*z* = 377 (M+1); Rₜ = 5.0 min

### Example 74 (General procedure (A))

### (E)-2-Cycloheptanesulfonyl-3-(3,5-di-tert-butyl-4-hydroxy-phenyl)-acrylonitrile

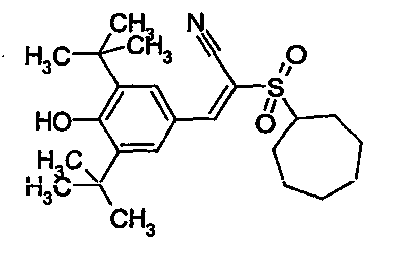

Step A: The title compound was prepared from 3,5-di-tert-butyl-4-hydroxybenzaldehyde and cycloheptanesulfonylacetonitrile in 76 % yield.

¹H NMR (DMSO-*d*₆): δ 1.40 (s, 18 H) 1.45 -1.60 (m, 6 H) 1.62 - 1.83 (m, 4 H) 2.08 - 2.23 (m, 2 H) 3.38 - 3.52 (m, 1 H) 7.97 (s, 2 H) 8.13 (s, 1 H) 8.40 (s, 1 H); HPLC-MS (Method A): *m*/*z* = 419 (M+1); Rₜ = 5.7 min.

### Example 75 (General procedure (A))

### (E)-2-Cyclobutylmethanesulfonyl-3-(3,5-di-tert-butyl-4-hydroxy-phenyl)-acrylonitrile

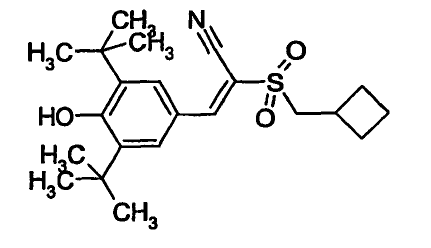

Step A: The title compound was prepared from 3,5-di-tert-butyl-4-hydroxybenzaldehyde and cyclobutylmethanesulfonylacetonitrile in 71 % yield.

¹H NMR (DMSO-*d*₆): δ 1.40 (s, 18 H) 1.74 -1.94 (m, 4 H) 2.01 - 2.16 (m, 2 H) 2.64-2.82 (m, 1 H) 3.52 (d, *J*=7.16 Hz, 2 H) 7.91 (s, 2 H) 8.07 (s, 1 H) 8.40 (s, 1 H); HPLC-MS (Method A): *m*/*z* = 391 (M+1); Rₜ = 5.3 min.

### Example 76 (General procedure (A))

### (E)-3-(3,5-Di-tert-butyl-4-hydroxy-phenyl)-2-(tricyclo[2.2.1.0^{2,6}]heptane-3-sulfonyl)-acrylonitrile

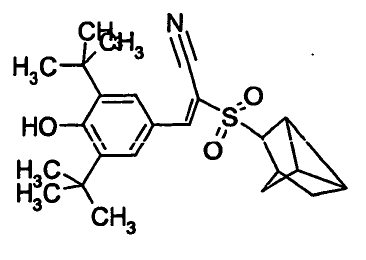

Step A: The title compound was prepared from 3,5-di-tert-butyl-4-hydroxybenzaldehyde and (tricyclo[2.2.1.0^{2,6}]heptane-3-sulfonyl)acetonitrile in 43 % yield.

¹H NMR (DMSO-*d*₆): δ 1.27 (d, *J*=10.55 Hz, 1 H) 1.33 -1.51 (m, 4 H)1.43 (s, 18 H) 1.56 (t, *J*=5.09 Hz, 1 H) 2.09 (d, *J*=10.55 Hz, 1 H) 2.40 (s, 1 H) 3.45 (s, 1 H) 7.96 (s, 2 H) 8.15 (s, 1 H) 8.39 (s, 1 H); HPLC-MS (Method A): *m*/*z* = 415 (M+1); Rₜ = 5.4 min

### Example 77 (General procedure (A))

### (E)-2-Cyclooctanesulfonyl-3-(3,5-di-tert-butyl-4-hydroxy-phenyl)-acrylonitrile

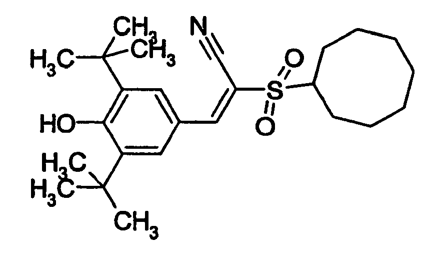

Step A: The title compound was prepared from 3,5-di-tert-butyl-4-hydroxybenzaldehyde and cyclooctanesulfonylacetonitrile in 25 % yield.

¹H NMR (DMSO-*d*₆): δ 1.40 (s, 18 H) 1.45 - 1.62 (m, 8 H) 1.66 - 1.85 (m, 4 H) 2.03 - 2.19 (m, 2 H) 3.38 - 3.50 (m, 1 H) 7.97 (s, 2 H) 8.16 (s, 1 H) 8.41 (s, 1 H); HPLC-MS (Method A): *m*/*z* = 433 (M+1); Rₜ = 5.9 min.

### Example 78 (General procedure (A))

### (E)-3-(3-tert-Butyl-4-hydroxy-5-methyl-phenyl)-2-(4-trifluoromethoxybenzenesulfonyl)-acrylonitrile

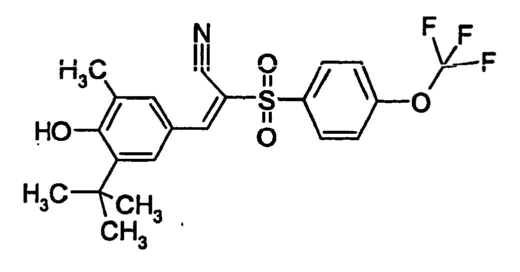

Step A: The title compound was prepared from 3-tert-butyl-4-hydroxy-5-methylbenzaldehyde and 4-trifluoromethoxy-benzenesulfonylacetonitrile in 92 % yield.

¹H NMR (DMSO-*d*₆): δ 1.36 (s, 9 H) 2.23 (s, 3 H) 7.72 (d, 2 H) 7.73 (s, 1 H) 7.94 (s, 1 H) 8.13 (d, 2 H) 8.36 (s, 1 H) 9.88 (s, 1 H); HPLC-MS (Method A): *m*/*z* = 440 (M+1); Rₜ = 5.23 min.

### Example 79 (General procedure (A))

### (E)-3-(3-tert-Butyl-4-hydroxy-5-methyl-phenyl)-2-(3-trifluoromethoxybenzenesulfonyl)-acrylonitrile.

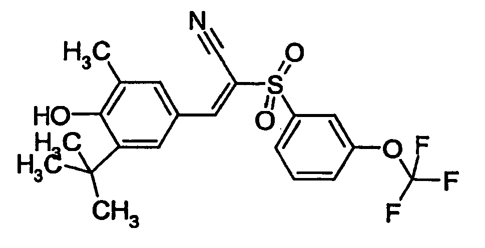

Step A: The title compound was prepared from 3-tert-butyl-4-hydroxy-5-methylbenzaldehyde and 3-trifluoromethoxy-benzenesulfonylacetonitrile in 56 % yield.

¹H NMR (DMSO-*d*₆): δ 1.36 (s, 9 H) 2.23 (s, 3 H) 7.73 (d, *J*=1.88 Hz, 1 H) 7.84 - 7.90 (m, 2 H) 7.92 - 7.97 (m, 2 H) 8.01 - 8.08 (m, 1 H) 8.37 (s, 1 H) 9.92 (s, 1 H); HPLC-MS (Method A): *m*/*z* = 440 (M+1); Rₜ = 5.1 min.

### Example 80 (General procedure (A))

### (E)-3-(3,5-Di-tert-butyl-4-hydroxy-phenyl)-2-(5-methyl-hexane-1-sulfonyl)-acrylonitrile

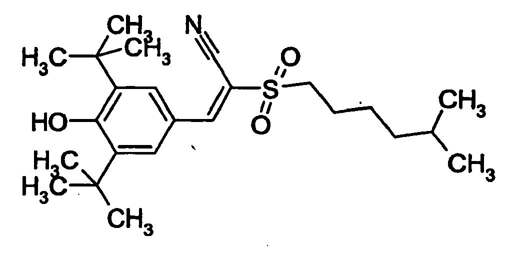

Step A: The title compound was prepared from 3,5-di-tert-butyl-4-hydroxybenzaldehyde and 5-methyl-hexane-1-sulfonyl)-acetonitrile in 18 % yield.

¹H NMR (DMSO-*d₆*): δ 0.83 (d, *J*=6.41 Hz, 6 H) 1.11 -1.21 (m, 2 H) 1.24 - 1.31 (m, 2 H) 1.40 (s, 18 H) 1.46 - 1.54 (m, 1 H) 1.59 - 1.74 (m, 2 H) 3.36 - 3.45 (m, 2 H) 7.95 (s, 2 H) 8.16 (s, 1 H) 8.40 (s, 1 H); HPLC-MS (Method A): *m*/*z* = 421 (M+1); Rₜ = 5.9 min.

### Example 81 (General procedure (A))

### (E)-2-(2-Cyclohexyl-ethanesulfonyl)-3-(3,5-di-tert-butyl-4-hydroxy-phenyl)-acrylonitrile

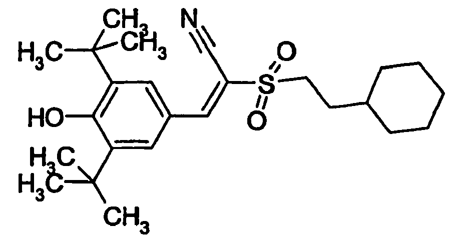

Step A: The title compound was prepared from 3,5-di-tert-butyl-4-hydroxybenzaldehyde and - (2-Cyclohexyl-ethanesulfonyl)-acetonitrile in 94 % yield.

¹H NMR (DMSO-*d₆*): δ 0.83 - 0.97 (m, 2 H) 1.07 - 1.26 (m, 4-H) 1.31- 1.38 (m, 1H) 1.40 (s, 18 H) 1.53 - 1.77 (m, 6 H) 3.37 - 3.45 (m. 2 H) 7.95 (s, 2 H) 8.17 (s. 1 H) 8.40 (s. 1 H); HPLC-MS (Method A): *m*/*z* = 433 (M+1); Rₜ = 5.9 min.

### Example 82 (General procedure (A))

### (E)-3-(3,5-Di-tert-butyl)-4-hydroxy-phenyl)-2-(4-methyl-pentane-1-sulfonyl)-acrylonitrile

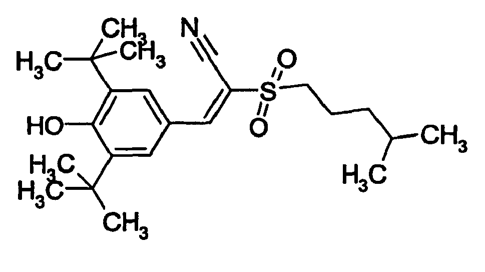

Step A: The title compound was prepared from 3,5-di-tert-butyl-4-hydroxybenzaldehyde and (4-methyl-pentane-1-sulfonyl)-acetonitrile in 37 % yield.

¹H NMR (DMSO-*d₆*): δ 0.85 (d, *J*=6.78 Hz, 6 H) 1.23 - 1.34 (m, 2 H) 1.41 (s, 18 H) 1.49 1.61 (m, 1 H) 1.64 - 1.78 (m, 2 H) 3.35 - 3.45 (m, 2 H) 7.95 (s, 2 H) 8.18 (s, 1 H) 8.40 (s, 1 H); HPLGMS (Method A): *m*/*z* = 407 (M+1); Rₜ = 5.68 min.

### Example 83 (General procedure (A))

### (E)- 3-(3-tert-Butyl-4-hydroxy-5-methyl-phenyl)-2-(hexane-1-sulfonyl)-acrylonitrile

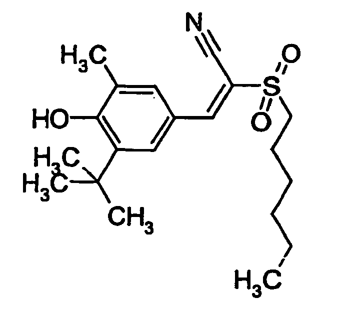

Step A: The title compound was prepared from 3-tert-butyl-4-hydroxy 5-methylbenzaldehyde and (hexane-1-sulfonyl)acetonitrile in 56 % yield.

¹H NMR (DMSO-*d*₆): *δ* 0.85 (t, 3 H) 1.18 - 1.32 (m, 4 H) 1.36 - 1.46 (m, 2 H) 1.36 - 1.42 (m, 9 H) 1.61 - 1.76 (m, 2 H) 2.23 (s, 3 H) 3.35 - 3.43 (m, 2 H) 7.71 (d, Z=1.88 Hz, 1 H) 7.95 (d, *J*=2.26 Hz, 1 H) 8.04 (s, 1 H) 9.77 (s, 1 H); HPLC-MS (Method A): *m*/*z* = 365 (M+1); Rₜ=5.1 min

### Example 84 (General procedure (A))

### (E)- 3-(4-Hydroxy-3,5-diisopropyl-phenyl)-2-(4-trifluoromethoxy-benzenesulfonyl)-acrylonitrile.

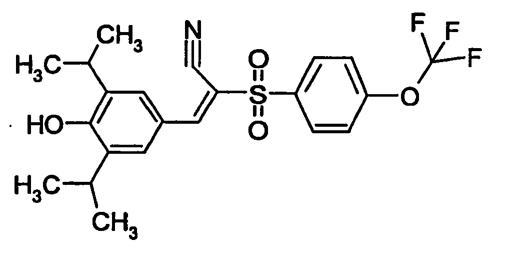

Step A: The title compound was prepared from 3,5 diisopropyl-4-hydroxybenzaldehyde and 4-trifluoromethoxy-benzenesulfonylacetonitrile in 56 % yield.

¹H NMR (DMSO-*d₆*): δ 1.16 (d, *J*=6.78 Hz, 12 H) 3.23 - 3.31 (m, 2 H) 7.72 (d, *J*=7.91 Hz. 2 H) 7.86 (s, 2 H) 8.14 (d, *J*=9.04 Hz, 2 H) 8.41 (s, 1 H) 9.73 - 9.92 (m, 1 H); HPLC-MS (Method A): *m*/*z* = 454 (M+1); Rₜ = 5.3 min.

### Example 85 (General procedure (A))

### (E)-3-(4-Hydroxy-3,5=diisopropyl-phenyl)-2-(3-trifluoromethoxy-benzenesulfonyl)-acrylonitrile

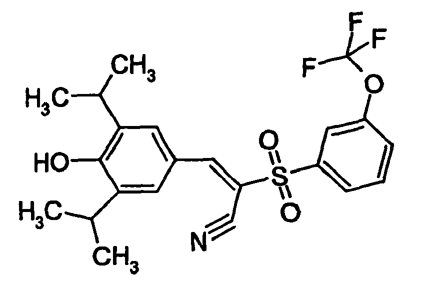

Step A: The title compound was prepared from 3,5 diisopropyl-4-hydroxybenzaldehyde-and 3-trifluoromethoxy-benzenesulfonylacetonitrile in 53 % yield.

¹H NMR (DMSO-*d₆*): δ 1.17 (d, *J*=6.78 Hz, 12 H) 3.24 - 3.36 (m, 2 H) 7.83 - 7.92 (m, 4 H) 7.96 (s, 1 H) 8.06 (d, *J*=6.41 Hz, 1 H) 8.45 (s, 1 H) 9.85 (s, 1 H); HPLC-MS (Method A): *m*/*z* = 454 (M+1); Rₜ = 5.2 min.

### Example 86 (General procedure (A))

### (E)- 2-(4-Chloro-benzenesulfonyl)-3-(4-hydroxy-3,5-dimethyl-phenyl)-acrylonitrile

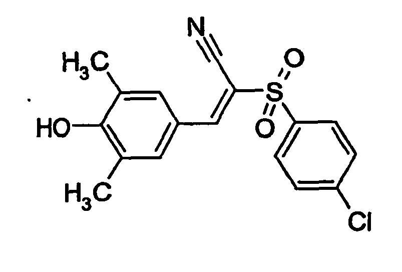

Step A: The title compound was prepared from 3,5 dimethyl-4.-hydroxybenzaldehyde and 4-chloro-benzenesulfonylacetonitrile in 62 % yield.

¹H NMR (DMSO-*d*₆)**:** δ 2.19 (s, 6 H) 7.73 (s, 2 H) 7.80 (d, *J*=8.67 Hz, 2 H) 7.99 (d, *J*=8.67 Hz, 2 H) 8.27 (s, 1 H) 9.96 (s, 1 H); HPLC-MS (Method A): *m*/*z* = 349 (M+1); Rₜ = 5.9 min

### Example 87 (General procedure (A))

### (E)- 2-(4-Chloro-benzenesulfanyl)-3-(4-hydroxy-3,5-dibromo-phenyl)-acrylonitrile

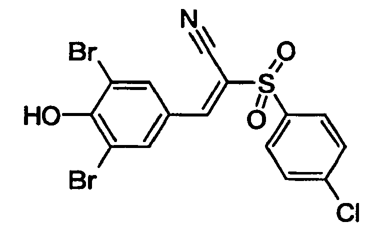

Step A: The title compound was prepared from 3,5-dibromo-4-hydroxybenzaldehyde and 4-chloro-benzenesulfonylacetonitrile in 50 % yield.

¹H NMR (DMSO-*d₆*): δ 7.82 (d, *J*=8.67 Hz, 2 H) 7.99 (d, *J*=8.67 Hz, 2 H) 8.27 (s, 2 H) 8.36 (s, 1 H); HPLGMS (Method A): *m*/*z* = 476, 478, 480 (M+1); Rₜ = 5.6 min.

### Example 88 (General procedure (A))

### (E)- 2-(4-Chloro-benzenesulfonyl)-3-(4-hydroxy-3,5-dimethoxy-phenyl)-acrylonitrile

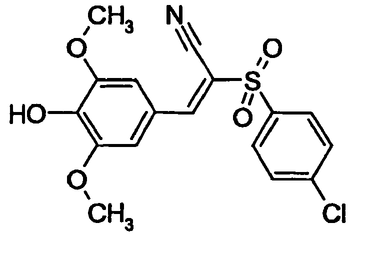

Step A: The title compound was prepared from 3,5-dimethoxy-4-hydroxybenzaldehyde and 4-chloro-benzenesulfonylacetonitrile in 50 % yield.

¹H NMR (DMSO-*d₆*): δ 3.81 (s, 6 H) 7.48 (s, 2 H) 7.82 (d, 2 H) 7.99 (d, 2 H) 8.38 (s, 1 H) 10.22 (s, 1 H); HPLC-MS (Method A): *m*l*z* = 380, 382 (M+1); Rₜ = 4.9 min

### Example 89 (General procedure (A))

### (E)- 2-(4-Chloro-benzenesulfonyt)-3-(4-hydroxy-3-iodo-5-methoxy-phenyl)-acrylonitrile

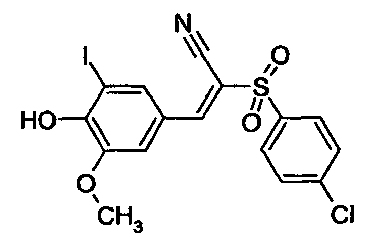

Step A: The title compound was prepared from 3-iodo-5-methoxy-4-hydroxybenzaldehyde and 4-chloro-benzenesulfonylacetonitrile in 77 % yield.

¹H NMR (DMSO-*d₆*): δ 3.85 (s, 3 H) 7.73 (d, *J*=1.88 Hz, 1 H) 7.82 (d, *J*=8.67 Hz, 2 H) 7.99 (d, 2 H) 8.10 (d, *J*=2.26 Hz, 1 H) 8.38 (s, 1 H) 11.26 (s, 1 H); HPLGMS (Method A): *m*/*z* = 476, 478 (M+1); Rₜ = 5.2 min.

### Example 90 (General procedure (A))

### (E)-2-(Hexane-1-sulfonyl)-3-(4-hydroxy-3,5-diisopropyl-pheny)-acrytonitrile

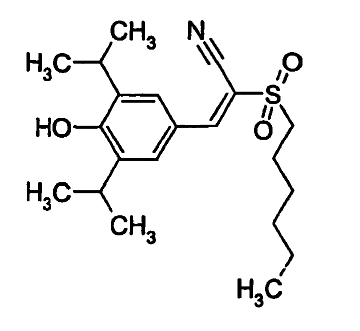

Step A: The title compound was prepared from 3,5-diisopropyl-4-hydroxybenzaldehyde and hexylsulfonylacetonitrile in 56 % yield.

HPLC-MS (Method A): *m*/*z* = 379 (M+1); Rₜ = 5.2 min.

### Example 91 (General procedure (A))

### (E)-3-(3,5-Di-tert-butyl-4-hydroxy-phenyl)-2-[2-(2-methoxy-ethoxy)-ethanesulfonyl]-acrylonitrile.

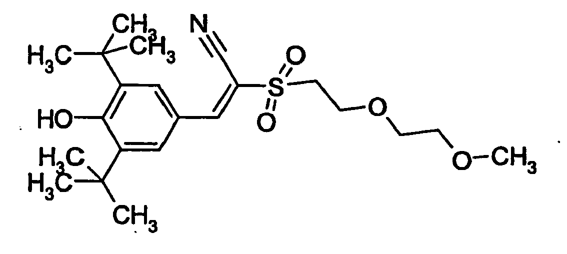

Step A: The title compound was prepared from 3,5-di-tert-butyl-4-hydroxybenzaldehyde and 2-(2-methoxy-ethoxy)-ethanesulfonylacetonitrile in 25 % yield.

HPLC-MS (Method A): *m*/*z* = 424 (M+1); Rₜ = 4.7 min.

### Example 92 (General procedure (A))

### (E)- 3-(3,5-Di-tert-butyl-4-hydroxy-phenyl)-2-(2-methoxy-ethanesulfonyl)-acrylonitrile

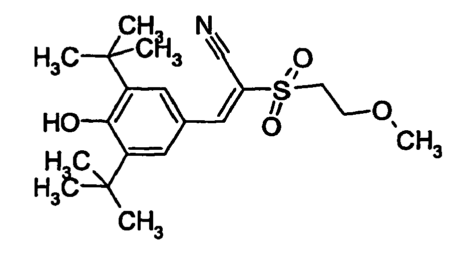

Step A: The title compound was prepared from 3,5-di-tert-butyl-4-hydroxybenzaldehyde and 2-methoxy-ethanesulfonylacetonitrile in 20 % yield.

¹H NMR (DMSO-*d₆*): δ ppm 1.41 (s, 18 H) 3.22 (s, 3 H) 3.63 - 3.78 (m, 4 H) 7.93 (s, 2 H) 8.16 (s, 1 H) 8.37 (s, 1 H); HPLC-MS (Method A): *m*/*z* = 380 (M+1); Rₜ = 4.7 min.

### Example 93 (General procedure (A))

### (Eb3-(3,5-Di-tert-butyl-4-hydroxy-phenyl)-2-(pent-4-ene-1-sulfonyl) acrylonitrile

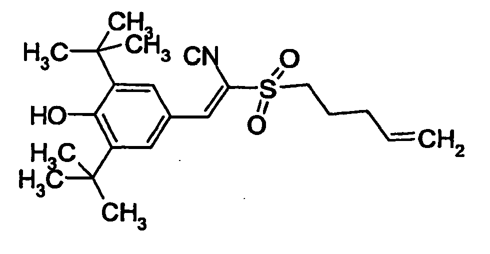

Step A: The title compound was prepared from 3,5-di-tert-butyl-4-hydroxybenzaldehyde and pent-4-ene-1-sulfonylacetonitrile in 98 % yield.
¹H NMR (DMSO-*d₆*): δ ppm 1.71 -1.87 (m, 2 H) 2.17 (q, *J*=6.78 Hz, 2 H) 3.36 - 3.44 (m, 2 H) 4.96 - 5.11 (m, 2 H) 5.71 - 5.89 (m, 1 H) 7.96 (s, 2 H) 8.18 (s, 1 H) 8.41 (s, 1 H); HPLC-MS (Method A): *m*/*z* = 390 (M+1); Rₜ = 5.4 min.

### Example 94 (General procedure (A))

### (E)-3-(3,5-Di-tert-butyl-4-hydroxy-phenyl)-2-(piperidine-1-sulfonyl)-acrylonitrile

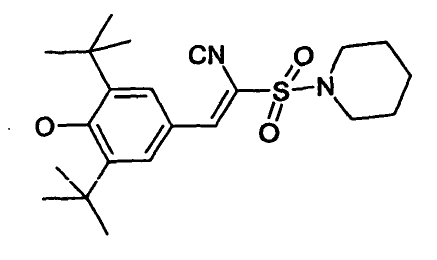

Step A: The title compound was prepared from 3,5-di-tert-butyl-4-hydroxybenzaldehyde and (piperidine-1-sulfonyl)-acetonitrile.

### Example 95 (General procedure (A))

### (E)-3-(3,5-Di-tert-butyl-4-hydroxy-phenyl)-2-(azepane-1-sulfonyl)-acrylonitrile

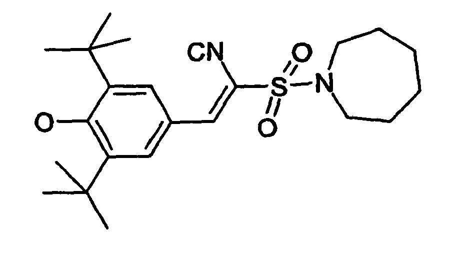

Step A: The title compound was prepared from 3,5-di-tert-butyl-4-hydroxybenzaldehyde and (azepane-1-sulfony)-acetonitrile.

HPLC-MS (Method A): *m*/*z* = 419 (M+1); Rₜ = 5.7 min.

### Example 96 (General procedure (A))

### (E)-3-(3,5-Di-ten:-butyl-4-hydroxy-phenyl)-2-(pyrrolidine-1-sulfonyl)-acrylonitrile

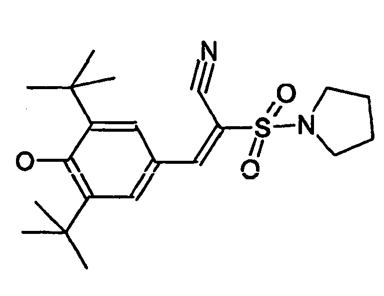

Step A: The title compound was prepared from 3,5-di-tert-butyl-4-hydroxybenzaldehyde and (pyrrolidine-1-sulfonyl)-acetonitrile.

### Example 97 (General procedure (A))

### (E)-3-(3,5-Di-tert-butyl-4-hydroxy-phenyl)-2-(morpholine-4-sulfonyl)-acrylonitile

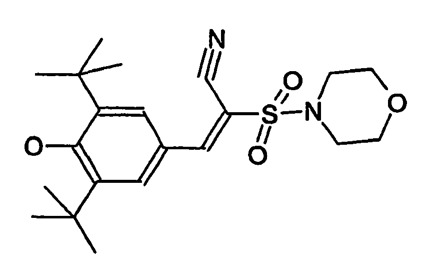

Step A: The title compound was prepared from 3,5-di-tert-butyl-4-hydroxybenzaidehyde and (morpholine-4-sulfonyl)-acetonitrile.

### PHARMACOLOGICAL METHODS

### Assay (I): Glucose utilisation in a human epithelia cell line (FSK-4 cells)

### Assay description:

The assay measures indirectly the activity of the respiratory chain in Ask-4 cells by using D-(6-³H(N))-glucose. The ³H-proton will first be released in the TCA cyclus and transported to the respiratory chain where it will be incorporated into water. The water is thereafter separated from the D-(6-³H(N))-glucose by evaporation. Finally, the radioactivity in the water is determined using a Topcounter.

### Method:

FSK-4 cells obtained from ATCC (Maryland, USA), are cultured in growth medium (McCoy's medium with the following addition 100 units/ml penicillin and streptomycin and 10 % FCS (fetal calf serum)) at 37°C and 5% CO₂. All media are obtained by Gibco (Life Technologies, Maryland, USA) where not otherwise mentioned.

At day zero the cells are harvested using trypsin-EDTA and washed in assay medium (MEM medium with the following addition 1x non-essential amino acids (M7145, 2 mM glutamin, 100 units/ml pencillin and streptomycin, 0.0075% sodium bicarbonate, 1 mM sodium pyruvate and 2 % horse serum) using centrifugation. The cells are plated into single StripPlates wells (Coming B.V.Life Sciences, The Netherlands) that are placed into 24-well plates (Coming B.V.Life Sciences, The Netherlands) with a concentration of 1,5x10⁴ cells/100 µl assay medium/well. The cells are then incubated at 37°C and 5% CO₂ overnight.

The next day the compounds to be tested are diluted to different concentrations in DMSO (Sigma, Missouri, USA) to 100 times final concentration. They are then diluted to a final concentration in assay medium containing 10 µCi/ml D-(6-³H(N))-glucose (PerkinElmer Life Sciences Inc.,Boston, USA). The medium is removed from the cells and 200 µl of the compound dilutions are added in duplicates. The cells are then incubated for another 24 hours at 37°.C and 5% CO₂. Finally the cells are lysed by adding 50 µl 10% TCA (trichloroacetate). 300 µl of sterile water is then added to the 24-wells that surrounds the Strip-Plate wells. The plate is sealed with Top-seal-tape (Packard, PerkinElmer Life Sciences Inc.,Boston, USA) and the plate is incubated in a heating cupboard at 50°C to equilibrium the radioactive water formed in the respiratory chain into the water in the 24-well plate by evaporate. The plates incubate for 8 hours where the heating cupboard is turned off. The top seal is removed when the samples have reached room temperature. One ml scintillation liquid (Packard Microscient, PerkinElmer Life Sciences Inc.,Boston, USA) is added to all the samples and the radioactivity is determined using a Topcounter (Packard, PerkinElmer Life Sciences Inc.,Boston, USA). Non-specific activity is determined by evaporating 200 µl of the dilution medium containing the D-(6-³H(N))-glucose into 300 µl sterile water, and total radioactivity is determined by counting 5 µl assay medium with 10 µCi/ml D-(6-³H(N))-glucose.

### Calculations

The half maximal concentration (EC₅₀) and maximal efficacy (Eₘₐₓ) are calculated using the Hill equation in GraphPad Prism 3.0 (GraphPad software, Inc.). In studies where the linear slope is determined the following concentration of the compound is used; 5x, 3x, 2x, 1,5x, 1,25x, 1x, 0.85x, 0.7x, 0.5x, 0.3x, 0.2x and 0x EC₅₀. From the percentage increase in glucose utilisation the linear slope is calculated using the Michaelis-Menten equation.

### Assay (II):The effect of chemical uncouplers on mitochondrial respiration using Isolated mitochondrial.

This assay is used to investigate if the increase in glucose utilisation caused by the test compounds observed in the glucose utilisation assay is due to an increase in the respiration of the mitochondria. This is done by measuring oxygen consumption in isolated rat liver mitochondria.

A Clark oxygen electrode is used to determine the oxygen consumption. The isolated mitochondria are added to assay medium (D-Mannitol 220mM, MagnesiumCloride 5mM, HEPES 2 mM and PotassiumPhosphate 5mM, pH = 7,4) containing rotenone (an in- : hibitor of clomplex 1) and oligomyocin (an inhibitor of the ATP-synthase) and the rate of oxygen consumptions is measured, when stabilized nutrient (e.g. succinate) is added and an increase in the rate of oxygen consumption is measured. When the rate of oxygen consumption again has stabilized the test compound is added and the oxygen consumption is measured. If the test compound stimulates the rate of oxygen consumption, it is regarded as a chemical uncoupler.

### Assay (111): Identification of chemical uncouplers that increase energy expenditure in vivo

The effect of the chemical uncouplers on energy expenditure (oxygen consumption) *in vivo* is determined by indirect calorimetry. Briefly, animals are placed in airtight chambers. Air is continuously led to and from the chambers. The gas concentrations of oxygen (O₂) and carbondioxide (CO₂) in the air led to and from the chambers (inlet and outlet air) are recorded and the consumption of O₂ and the production of CO₂ are calculated. Based on the amount of O₂ consumed and CO₂ produced, energy expenditure is calculated. Compounds which at a given dose increase whole body energy expenditure without obvious deleterious effects are deemed to be chemical uncouplers that increase energy expenditure.

Table 1 shows assay results for compounds of the presents invention.

**TABLE 1**

| **Example No.** | **GlucosFSK4 EC50** **(µM)** | **GlucosFSK4 Slope** **(dimensionless)** | **GlucosFSK4 Slope** **(% of FCCP)** |
|---|---|---|---|
| 1 | 1.4 | 1 | 100 |
| 2 | 0.8 | 0.75 | 49 |
| 3 | 9 | 0.75 | 65 |
| 4 | >10 | | |
| 5 | | | |
| 6 | 8.4 | 0.8 | 53 |
| 7 | 10 | 1.25 | 94 |
| 8 | | | |
| 9 | 1.4 | 0.45 | |
| 10 | 1 | 0.65 | 31 |
| 11 | 2.2 | 0.8 | 43 |
| 12 | 6 | 1.3 | 64 |
| 13 | 4 | 0.87 | 41 |
| 14 | 2.3 | 0.9 | 68 |
| 15 | 10 | 1.1 | 77 |
| 16 | 3.2 | 0.95 | 52 |
| 17 | 7.4 | 0.95 | 55 |
| 18 | 1.7 | 0.55 | 29 |
| 19 | 5.1 | 0.75 | 41 |
| 20 | 2.8 | 1.55 | 84 |
| 21 | 0.7 | 0.75 | 41 |
| 22 | 0.4 | 1.65 | 90 |
| 23 | 1.2 | 0.6 | 42 |
| 24 | 0.7 | 1 | 57 |
| 25 | 0.5 | 1.8 | 103 |
| 26 | 3.7 | 1.5 | 138 |
| 27 | 3.5 | 0.73 | 46 |
| 28 | 0.5 | | 84 |
| 29 | 2.9 | 1.1 | 83 |
| 30 | 1 | 0.8 | 69 |
| 31 | 2 | 0.65 | 48 |
| 32 | 0.6 | 1.1 | 83 |
| 33 | 1.1 | 0.75 | 58 |
| 34 | 2.3 | 0.95 | 72 |
| 35 | 3.3 | 1.2 | 92 |
| 36 | 3.8 | 0.6 | 47 |
| 37 | 1 | 0.65 | 48 |
| 38 | 0.7 | 0.6 | 50 |
| 39 | 0.5 | 1.1 | 55 |
| 40 | 0.6 | 0.7 | 58 |
| 41 | 0.9 | 0.6 | 33 |
| 42 | 9 | | |
| 43 | 1.7 | | |
| 44 | 3 | 0.8 | 41 |
| 45 | 3 | | |
| 46 | 1 | 1.1 | 61 |
| 47 | 0.8 | 0.7 | 36 |
| 48 | 1.5 | 1.1 | 55 |
| 49 | 2.2 | | |
| 50 | 1.2 | 1 | 51 |
| 51 | 0.9 | 0.8 | 65 |
| 52 | 1 | 0.7 | 63 |

## Claims

1. A compound of formula I 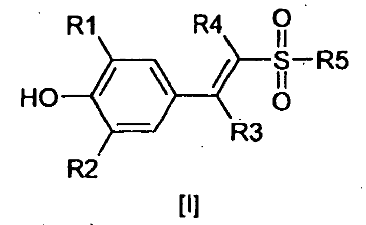
wherein R1 and R2 independently represent hydrogen, nitro, cyano, halogen, alkyl, alkenyl, alkynyl, aryl, heteroaryl, haloalkyl, alkoxy, alkylamino, -C(O)OR6, -S(O)₂OR6, -S(O)ₙR6, -OC(O)R6,-NHC(O)R6 or -N(C(O)R6)₂;
R3 represents hydrogen, nitro, cyano, halogen, alkyl, alkenyl, alkynyl, alkoxy ,alkylamino, -C(O)OR6, -S(O)₂OR6, -S(O)ₙR6, -OC(O)R6, -NHC(O)R6 or -N(C(O)R6)₂;
R4 represents nitro, cyano, halogen, haloalkyl, -C(O)R6, -C(O)OR6, -C(O)N(R6)₂, -S(O)₂OR6, S(O)ₙR6, S(O)₂N(R6)₂, -P(O)(OR6)₂ or -B(OR6)₂;
R6 represents hydrogen or alkyl, aryl or heteroaryl, all of which may optionally be substituted with one or more substituents selected from amongst hydroxy, halogen, nitro and cyano; n represents 0, 1 or 2;
R5 represents alkyl, alkenyl, alkynyl, cycloalkyl, aryl, heteroaryl, heterocyclyl, all of which may optionally be substituted with one or more substituents selected among alkyl, alkenyl, alkynyl, phenyl, heteroaryl, heterocyclyl, halogen, hydroxy, cyano, nitro, carboxyl, haloalkyl,-O-R7, -S(O)ₙR7, -O-C(O)R7, -C(O)-O-R7, -C(O)-R7, -C(O)-N(R7)(R8), -N(R7)(R8), -(CH₂)_{P}-N(R8)-C(O)-R7, -B(OR7)(OR8), -(CH₂)ₚ-O-R7, -NR7-C(O)R7, NR7-S(O)ₙR7 and -(CH₂)ₚ-N(R7)(R8), said alkyl, alkenyl, alkynyl, phenyl, heteroaryl or heterocyclyl optionally being substituted with one or more substituents selected among alkyl, halogen, haloalkyl, hydroxyalkyl, cyano, nitro, O-R13, -S(O)ₙR11, -O-C(O)R11, -C(O)-O-R11, -C(O)-R11 -C(O)-N(R11)(R12), -N(R11)(R12), -(CH₂)ₚ-N(R11) -C(O)-R12, -B(OR11) (OR12), -(CH₂)ₚ-O-R11, and -(CH₂)ₚ-N(R11)(R12) ;
R7 and R8 independently represent hydrogen, haloalkyl, hydroxyalkyl, alkyl, alkenyl, alkynyl, cycloalkyl, heterocyclyl or phenyl, said phenyl or heterocyclyl optionally being substituted with one or more substituents selected among alkyl, halogen, haloalkyl, hydroxy, hydroxyalkyl, cyano, nitro, -N(R9)(R10) and -(CH₂)ₚ-N(R9)(R10); or R7 and R8 when bound to a nitrogen together with said nitrogen form a 5-8 membered heterocycle which may be further substituted with alkyl;
R9 and R10 independently represent hydrogen, alkyl, alkenyl, alkynyl, haloalkyl, hydroxyalkyl or cycloalkyl;
or R4 and R5 together with the atoms to which they are attached constitute a 5, 6, 7 or 8 membered ring, which may be saturated or may be partly or fully unsaturated, and wherein said ring is optionally substituted with one or more substituents selected among alkyl, halogen, hydroxy, cyano and nitro;
each R11 and R12 independently represents hydrogen: haloalkyl, hydroxyalkyl, alkyl, a!kenyl, alkynyl, cycloalkyl or phenyl;
R13 represents haloalkyl, hydroxyalkyl, alkyl, alkenyl, alkynyl or cycloalkyl;
p represents 0, 1 or 2;
or a pharmaceutically acceptable salt, solvate or hydrate thereof;
optionally in combination with another therapeutically active compound; for use in the treatment of obesity, type 2 diabetes, dyslipidemia; hypertension or gallbladder diseases; prevention of weight gain; or maintenance of weight loss.

2. The compound according to claim 1, wherein R1 and R2 in said compound of formula I are independently selected among hydrogen, alkyl, alkoxy, aryl, heteroaryl, halogen, nitro, -C(O)OR6 and -S(O)₂OR6.

3. The compound according to claim 2, wherein R1 and R2 in said compound of formula I independently represent alkyl, halogen or nitro.

4. The compound according to claim 3, wherein R1 and R2 in said compound of formula I independently represent C₁₋₆alkyl.

5. The compound according to claim 2, wherein R1 and R2 in said compound of formula I both represent methyl, isopropyl, sec.butyl, tert.butyl, methoxy or bromo.

6. The compound according to any one of claims 1-5, wherein R3 in said compound of formula I represents hydrogen, alkyl, alkenyl, alkynyl, alkoxy or alkylamino.

7. The compound according to any one of claims 1-6, wherein R3 in said compound of formula I is C₁₋₄alkyl.

8. The compound according to any one of claims 1-6, wherein R3 in said compound of formule I is methyl.

9. The compound according to any one of claims 1-8, wherein R4 in said compound of formula I represents nitro, cyano, -C(O)R6, -C(O)OR6, -C(O)N(R6)2, -S(O)₂OR6, -S(O)₂N(R6)₂ or -S(O)ₙR6, wherein n represents 1 or 2.

10. The compound according to claim 9, wherein R4 in said compound of formula I represents nitro.

11. The compound according to claim 9, wherein R4 in said compound of formula I represents cyano.

12. The compound according to claim 9, wherein R4 in said compound of formula I represents -C(O)R6.

13. The compound according to claim 9, wherein R4 in said compound of formula I represents -C(O)OR6.

14. The compound according to claim 9, wherein R4 in said compound of formula I represents -C(O)N(R6)₂.

15. The compound according to claim 9, wherein R4 in said compound of formula I represents -S(O)₂OR6,

16. The compound according to claim 9, wherein R4 in said compound of formula I represents-S(O)ₙR6.

17. The compound according to claim 9. wherein R4 in said compound of formula I represents -S(O)₂N(R6)₂.

18. The compound according to any one of claims 1-8, wherein R4 and R5 in said compound of formula I together with the atoms to which they are attached constitute a 5 or 6 membered ring which may be saturated, or partly or fully unsaturated, and wherein said ring is optionally substituted with one or more substituents selected among alkyl, halogen, hydroxy, cyano and nitro.

19. The compound according to claim 18, wherein R4 and R5 in said compound of formula I together with the atoms to which they are attached constitute a ring selected among [1,3]dithiolane 1,1,3,3-tetraoxide, 1,1-dioxo-tetrahydro-1-thiophen-3-one, 1,1-dioxo-thiazolidine-4-one, 1,1-dioxo-thiomorpholine-3-one tetrahydrothiopyran-1,1-dioxide and tetrahydrothiophen-1,1-dioxide, all of which may optionally be substituted with one or more substituents selected among alkyl, halogen, hydroxy, cyano and nitro.

20. The compound according to any one of claims 1-17, wherein R5 in said compound of formula I represents alkyl, cycloalkyl, alkenyl, aryl, heteroaryl or heterocyclyl, all of which may optionally be substituted with one or more substituents selected among alkyl, halogen, hydroxy, cyano, nitro, haloalkyl, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7 and -NR7-S(O)ₙ-R7.

21. The compound according to claim 20, wherein R5 in said compound of formula I represents C₁₋₁₂alkyl which may optionally be substituted with -OR7, -C(O)R7 or cycloalkyl.

22. The compound according to claim 21, wherein R5 in said compound of formula I represents methyl, propyl, 2-propyl, butyl, pentyl, hexyl, octyl or nonyl.

23. The compound according to claim 21 or 22, wherein said cycloalkyl is cyclopropyl, cyclobutyl or cyclohexyl.

24. The compound according to any one of claims 21-23, wherein R7 in said compound of formula I represents hydrogen or ethyl.

25. The compound according to claim 20, wherein R5 represents C₂₋₈alkenyl.

26. The compound according to claim 25, wherein R5 represents pent-4-enyl or hex-5-enyl.

27. The compound according to claim 20, wherein R5 represents cycloalkyl, which may optionally be substituted with C₁₋₆alkyl.

28. The compound according to claim 27, wherein R5 represents cyclopentyl, cyclohexyl, cycloheptyl, cyclooctyl or tricyclo[2.2.1.0²⁻⁶]heptyl.

29. The compound according to claim 20, wherein R5 represents aryl, phenyl or naphthyl, all of which may optionally be substituted with one or more substituents selected among alkyl, alkenyl, alkynyl, heteroaryl, heterocyclyl, halogen, hydroxy, cyano, nitro, haloalkyl, -O-R7, -S(O)ₙR7, -O-C(O)R7, -C(O)-O-R7, -C(O)-R7, -C(O)-N(R7)(R8), -N(R7)(R8), -(CH₂)p-N(R8)-C(O)-R7. -B(OR7)(OR8), -(CH₂)ₚ-O-R7, -(CH₂)ₚ-N(R7)(R8), -NR7-C(O)R7 and -NR7-S(O)ₙ-R7.

30. The compound according to claim 29, wherein R5 represents phenyl substituted with chloro, bromo or iodo.

31. The compound according to claim 29, wherein R5 represents phenyl substituted with C₁-₆alkyl, C₂₋₆alkenyl or C₂₋₆alkynyl, all of which may optionally be further substituted with -C(O)R7, -OR7 or phenyl.

32. The compound according to claim 31, wherein R7 represents hydrogen or C₁₋₆alkyl.

33. The compound according to claim 29, wherein R5 represents phenyl substituted with heterocyclyl.

34. The compound according to claim 29, wherein R5 represents phenyl substituted with -OR7, -C(O)OR7 or -S(O)₂R7.

35. The compound according to claim 34, wherein R7 represents hydrogen, C₁₋₄alkyl or C₁₋₄haloalkyl.

36. The compound according to claim 29, wherein R5 represents phenyl substituted with -C(O)-N(R7)(R8).

37. The compound according to claim 36, wherein R7 and R8 independently represent C₁₋₄alkyl or C₁₋₄hydroxyalkyl, or R7 and R8 together with the nitrogen to which they are bound form a 6-membered heterocycle comprising N and optionally O. wherein said heterocycle may optionally be further substituted with C₁₋₄alkyl.

38. The compound according to claim 20, wherein R5 represents a heteroaryl selected among pyridyl, furanyl and imidazolyl, optionally substituted with a substituent selected among alkyl, halogen, hydroxy, cyano, nitro, haloalkyl, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7 and -NR7-5(O)ₙ-R7.

39. The compound according to claim 38, wherein said heteroaryl is substituted with a substituent selected among fluoro, chloro, methyl, hydroxy, cyano, nitro, -C(O)-O-R7 and -C(O)-N(R7)(R8).

40. The compound according to claim 20, wherein R5 represents a heterocyclyl selected among piperidinyl, morpholinyl, pyrrolidinyl and azepanyl, optionally substituted with a substituent selected among alkyl, halogen, hydroxy, cyano, nitro, haloalkyl, -O-R7, -S(O)ₙR7, -O-C(O)R7, -C(O)-O-R7, -C(O)R7, -C(O)-N(R7)(R8), -N(R7)(R8), -(CH₂)ₚ-N(R8)-C(O)-R7, -B(OR7)(OR8), -(CH₂)p-O-R7, -(CH₂)ₚ-N(R7)(R8), -NR7-C(O)R7 and -NR7-S(O)ₙR7.

41. The compound according to claim 40, wherein said heterocyclyl is substituted with a substituent selected among fluoro, chloro, hydroxy, cyano, nitro, -C(O)-O-R7 and -C(O)-N(R7)(R8).

42. The compound according to any one of claims 1-41, wherein R9 and R10 in said compound of formula I independently represent hydrogen, alkyl, haloalkyl or hydroxyalkyl.

43. The compound according to any one of claims 1-42, wherein R11 and R12 independently represent hydrogen, C₁-₆alkyl, C₁-₆haloalkyl or C₁-₆hydroxyalkyl.

44. The compound according to claim 43, wherein R11 and R12 independently represent hydrogen, methyl, ethyl, trifluoromethyl, hydroxymethyl or 2-hydroxyethyl.

45. The compound according to any one of claims 1-44, wherein R13 represents C₁₋₆alkyl, C₁₋₆haloalkyl or C₁₋₆hydroxyalkyl.

46. The compound according to claim 45, wherein R13 represents methyl, ethyl, trifluoromethyl, hydroxymethyl or 2-hydroxyethyl.

47. The compound according to any one of claims 1-46, wherein n in said compound of formula I is 2.

48. The compound according to any one of claims 1-47, wherein p in said compound of formula I is 1.

49. The compound according to claim 1, wherein said compound of formula is selected among:
(E)-3-(3,5-Di-tert-butyl-4-hydroxy-phenyl)-2-methanesulfonyl-acrylonitrile;
(E)-2-(4-Chloro-benzenesulfonyl)-3-(3,5-di-tert-butyl-4-hydroxy-phenyl)-acrylonitrile;
(E)-2-(4-Chloro-benzenesulfonyl)-3-(4-hydroxy-3-nitro-phenyl)-acrylonitrile;
(E)-3-(4-Hydroxy-3-nitro-phenyl)-2-methanesulfonyl-acrylonitrile;
(E)-3-(3,5-Di-tert-butyl-4-hydroxy-phenyl)-2-(1-methyl-1H-imidazole-2-sulfonyl)-acrylonitrile;
(E/Z)-2-(4-Chloro-benzenesulfonyl)-3-(3,5-di-tert-butyl-4-hydroxy-phenyl-but-2-enenitrile;
(E)-4-[3-Cyano-1-(3,5-di-tert-butyl-4-hydroxy-phenyl)-prop-1-ene-2-sulfonyl]-benzoic acid;
(E)-3-(3,5-Di-tert-butyl-4-hydroxy-phenyl)-2-(4-nitro-benzenesulfonyl)-acrylonitrile;
(E)-3-(3,5-Di-tert-butyl-4-hydroxy-phenyl)-2-(4-trifluoromethoxy-benzenesulfonyl)-acrylonitrile;
(E)-3-(3-tert-Butyl-4-hydroxy-5-nitro-phenyl)-2-(4-chloro-benzenesulfonyl)-acrylonitrile;
(E)-4-[3-Cyano-1-(3,5-di-tert-butyl-4-hydroxy-phenyl)-prop-1-ene-2-sulfonyl]-N.N-dimethyl-benzamide;
(E)-4-[3-Cyano-1-(3,5-di-tert-butyl-4-hydroxy-phenyl)-prop-1-ene-2-sulfonyl]-N,N-bis-(2-hydroxy-propyl)-benzamide;
(E)-3-(3,5-Di-tert-butyl-4-hydroxy-phenyl)-2-[4-(4-methyl-piperazine-1-carbonyl)-benzenesulfonyl]-acrylonitrile;
(E)- 4-[3-Cyano-1-(3,5-di-tert-butyl-4-hydroxy-phenyl)-prop-1-ene-2-sulfonyl]-N-(2-hydroxyethyl)-benzamide;
(E)- 3-(3,5-Di-tert-butyl-4-hydroxy-phenyl-2-[4-(2,6-dimethyl-morpholine-4-carbonyl)-benzenesulfonyl]-acrylonitrile;
(E)- 3-(3,5-Di-tert-butyl-4-hydroxy-phenyl)-2-[4-(morpholine-4-carbonyl)-benzenesulfonyl]-acrylonitrile;
(E)-2-(4-Amino-benzenesulfonyl)-3-(3.5-di-tert-butyl-4-hydroxy-phenyl)-acrylonitrile;
(E)-2-[2-Cyano-1-(3,5-di-tert-butyl-4-hydroxy-phenyl)-ethenesulfonyl]-3-(3,5-di-tert-butyl-4-hydroxy-phenyl)-acrylonitrile;
(E)-2-(4-Chloro-phenylmethanesulfonyl}-3-(3,5-di-tert-butyl-4-hydroxy-phenyl}-acrylonitrile;
(E)-3-(3,5-Di-tert-butyl-4-hydroxy-phenyl)-2-(propane-2-sulfonyl)-acrylonitrile:
4-[3-Cyano-1-(3,5-di-tert-butyl-4-hydroxy-phenyl)-prop-1-ene-2-sulfonyl]-N,N-bis-(2-hydroxyethyl)-benzamide;
3-(3,5-Di-tert-butyl-4-hydroxy-phenyl)-2-[4-(3,5-dimethyl-morpholine-4-carbonyl)-benzenesulfonyl]-acrylonitrile;
3-(3,5-Di-tert-butyl-4-hydroxy-phenyl)-2-(2,5-dichloro-benzenesulfonyl)-acrylonitrile;
2-(4-tert-Butyl-benzenesulfonyl)-3-(3,5-di-tert-butyl-4-hydroxy-phenyl)-acrylonitrile;
3-(3,5-Di-tert-butyl-4-hydroxy-phenyl)-2-(naphthalene-1-sulfonyl)-acrylonitrile;
3-(3,5-Di-tert-butyl-4-hydroxy-phenyl)-2-(furan-2-ylmethanesulfonyl)-acrylonitrile;
3-(3,5-Di-tert-butyl-4-hydroxy-phenyl)-2-(2,4-dichloro-benzenesulfonyl)-acrylonitrile;
3-(3,5-Di-tert-butyl-4-hydroxy-phenyl)-2-(toluene-4-sulfonyl)-acrylonitrile;
3-(3,5-Di-tert-butyl-4-hydroxy-phenyl)-2-(4-isopropyl-benzenesulfonyl)-acrylonitrile;
2-(3,5-Di-tert-butyl-4-hydroxy-berizenesulfonyl)-3-(3,5-di-tert-butyl-4-hydroxy-phenyl)-acrylonitrile;
3-(3,5-Di-tert-butyl-4-hydroxy-phenyl)2-(hexane-1-sulfonyl)-acrylonitrile;
2-(4-Bromo-benzenesulfonyl)-3-(3,5-di-tert-butyl-4-hydroxy-phenyl)-acrylonitrile;
3-(3,5-Di-tert-butyl-4-hydroxy-phenyl)-2-(4-methanesulfonyl-benzenesulfonyl)-acrylonitrile;
3-(3,5-Di-tert-butyl-4-hydroxy-phenyl)-2-(3-trifluoromethoxy-benzenesulfony)-acrylonitrile;
3-(3,5-Di-tert-butyl-4-hydroxy-phenyl)-2-(2-trifluoromethoxy-benzenesulfonyl)-acrylonitrile;
2-(5-Chloro-pyridine-2-sulfonyl)-3-(3,5-di-tert-butyl-4-hydroxy-phenyl)-acrylonitrile;
2-[3-Cyano-1-(3,5-di-tert-butyl-4-hydroxy-phenyl)-prop-1-ene-2-sulfonyl]-benzoic acid;
3-[3-Cyano-1-(3,5-di-tert-butyl-4-hydroxy-phenyl)-prop-1-ene-2-sulfonyl]-benzoic acid;
3-(3,5-Di-tert-butyl-4-hydroxy-phenyl)-2-[2-(morpholine-4-carbonylbbenzenesulfonyl]-acrylonitrile;
3-(3,5-Di-tert-butyl-4-hydroxy-phenyl)-2-[3-(morpholine-4-carbonyl)-benzenesulfonyl]-acrylonitrile;
3-(3,5-Di-tert-butyl-4-hydroxy-phenyl)-2-(4-methoxy-benzenesulfonyl)-acrylonitrile;
3-(3,5-Di-tert-butyl-4-hydroxy-phenyl)-2-(3-methoxy-benzenesulfonyl)-acrylonitrile;
3-(3,5-Di-tert-butyl-4-hydroxy-phenyl)-2-(3,4-dimethoxy)-benzenesulfonyl)-acrylonitrile;
3-(3,5-Di-tert-butyl-4-hydroxy-phenyl)-2-[4-(2,3-dihydro-furan-2-yl)-benzenesulfonyl]-acrylonitrile;
3-{4-[2-Cyano-1-(3,5-di-tert-butyl-4-hydroxy-phenylyethenesulfonyl)-phenyl)-2-methyl-acrylic acid methyl ester;
3-(3,5-Di-tert-butyl-4-hydroxy-phenyl)-2-(4-styryl-benzenesulfonyl)-acrylonitrile;
3-(3,5-Di-tert-butyl-4-hydroxy-phenyl)-2-[4-(2-isobutoxy-vinyl)-benzenesulfonyl]-acrylonitrile;
2-[4-(2-Butoxy-vinyl)-benzenesulfonyl]-3-(3,5-di-tert-butyl-4-hydroxy-phenyl)-acrylonitrile;
3-(3,5-Di-tert-butyl-4-hydroxy-phenyl)-2-(4-phenylethynyl-benzenesulfonyl)-acrylonitrile;
3-(3,5-Di-tert-butyl-4-hydroxy-phenyl)-2-(2-ethoxy-ethanesulfonyl)-acrylonitrile;
3-(3,5-Di-tert-butyl-4-hydroxy-phenyl)-2-[2-(2-ethoxy-ethoxy)-ethanesulfonyl]-acrylonitrile;
2-(4-Chloro-benzenesulfonyl)-3-(3,5-di-sec-butyl-4-hydroxy-phenyl)-acrylonitrile;
(E)-2-(3-Bromo-benzenesulfonyl)-3-(3,5-di-tert-butyl-4-hydroxy-phenyl)-acrylonitrile;
3-(3,5-Di-tert-butyl-4-hydroxy-phenyl)-2-(hex-5-ene-1-sulfonyl)acrylonitrile;
(E)-2-(4-Iodo-benzenesulfonyl)-3-(3,5-di-tert-butyl-4-hydroxy-phenyl)-acrylonitrile:
(E)-2-Cyclopentanesulfonyl-3-(3,5-di-tert-butyl-4-hydroxy-phenyl)-acrylonitrile;
(E)-3-(3,5-Di-tert-butyl-4-hydroxy-phenyl)-2-(nonane-1-sulfonyl)-acrylonitrile;
(E)-3-(3,5-Di-tert-butyl-4-hydroxy-phenyl)-2-(pentane-1-sulfonyl)-acrylonitrile;
(E)-3-(3,5-Di-tert-butyl-4-hydroxy-phenyl)-2-(heptane-1-sulfonyl)-acrylonitrile;
(E)-2-Cyclohexanesulfony)-3-(3,5-di-tert-buty)-4-hydroxy-phenyl-acrylonitrile;
(E-3-{4-[2-Cyano-1-(3,5-di-tert-butyl-4-hydroxy-phenyl)-ethenesulfonyl]-phenyl}-propionic acid;
(E)-3-(3,5-Di-tert-butyl-4-hydroxy-phenyl)-2-(4-methyl-2-oxo-2H-chromene-7-sulfonyl)-acrylonitrile;
(E)-3-(3,5-Di-tert-butyl-4-hydroxy-phenyl)-2-(4-trifluoromethoxy-phenylmethanesulfonyl)-acrylonitrile;
(E)-2-Cyclohexylmethanesulfonyl-3-(3,5-di-tert-butyl-4-hydroxy-phenyl)-acrylonitrile;
(E)-2-(Butane-1-sulfonyl)-3-(3,5-di-tert-butyl-4-hydroxy-phenyl)-acrylonitrile;
(E)-2-(Propane-1-sulfonyl)-3-(3,5-di-tert-butyl-4-hydroxy-phenyl)-acrylonitrile;
(E)-2-(Ethane-1-sulfonyl)-3-(3,5-di-tert-butyl-4-hydroxy-phenyl)-acrylonitrile;
(E)-2-Cyclopropylmethanesulfonyl-3-(3,5-di-tert-butyl-4-hydroxy-phenyl)-acrylonitrile;
(E)-2-Cycloheptanesulfonyl-3-(3,5-di-tert-butyl-4-hydroxy-phenyl)-acrylonitrile;
(E)-2-Cyclobutylmethanesulfonyl-3-(3,5-di-tert-butyl-4-hydroxy-phenyl)-acrylonitrile;
(E)-3-(3,5-Di-tert-buty)-4-hydroxy-phenyl)-2-(tricyclo[2.2.1.0^{2,6}]heptane-3-sulfonyl)-acrylonitrile;
(E)-2-Cyclooctanesulfonyl-3-(3,5-di-tert-butyl-4-hydroxy-phenyl)-acrylonitrile;
(E)-3-(3-tert-Butyl-4-hydroxy-5-methyl-phenyl)-2-(4-trifluoromethoxy-benzenesulfonyl)-acrylonitrile;
(E)-3-(3-tert-Butyl-4-hydroxy-5-methyl-phenyl)-2-(3-trifluoromethoxy-benzenesulfonyl)-acrylonitrile;
(E)-3-(3,5-Di-tert-butyl-4-hydroxy-phenyl)-2-(5-methyl-hexane-1-sulfonyl)-acrylonitrile;
(E)-2-(2-Cyclohexyl-ethanesulfonyl)-3-(3,5-di-tert-butyi-4-hydroxy-phenyl)-acrylonitrile;
(E)-3-(3,5-Di-tert-butyl-4-hydroxy-phenyl)-2-(4-methyl-pentane-1-sulfonyl)-acrylonitrile;
(E)-3-(3-tert-Butyl-4-hydroxy-5-methyl-phenyl)-2-(hexane-1-sulfonyl)-acrylonitrile;
(E)-3-(4-Hydroxy-3,5-diisopropyl-phenyl)-2-(4-trifluoromethoxy-benzenesulfonyl)-acrylonitrile;
(E)-3-(4-Hydroxy-3,5-diisopropyl-phenyl)-2-(3-trifluoromethoxy-benzenesulfonyl)-acrylonitrile;
(E)-2-(4-Chloro-benzenesulfonyl)-3-(4-hydroxy-3,5-dimethyl-phenyl)-acrylonitrile;
(E)-2-(4-Chloro-benzenesulfonyl)-3-(4-hydroxy-3,5-dibromo-phenyl)-acrylonitrile:
(E)-2-(4-Chloro-benzenesulfonyl)-3-(4-hydroxy-3,5-dimethoxy-phenyl)-acrylonitrile;
(E)-2-(4-Chloro-benzenesulfonyl)-3-(4-hydroxy-3-iodo-5-methoxy-phenyl)-acrylonitrile;
(E)-2-(Hexane-1-sulfonyl)-3-(4-hydroxy-3,5-diisopropyl-phenyl)-acrylonitrile;
(E)-3-(3,5-Di-tert-butyl-4-hydroxy-phenyl)-2-(2-methoxy-ethanesulfonyl)-acrylonitrile;
(E)-3-(3,5-Di-tert-butyl-4-hydroxy-phenyl)-2-(pent-4-ene-1-sulfonyl)-acrylonitrile;
(E)-3-(3,5-Di-tert-butyl-4-hydroxy-phenyl)-2-(piperidine-1-sulfonyl)-acrylonitrile;
(E)-3-(3,5-Di-tert-butyl-4-hydroxy-pheny)-2-(azepane-1-sulfonyl)-acrylonitrile;
(E)-3-(3,5-Di-tert-butyl-4-hydroxy-phenyl)-2-(pyrrolidine-1-sulfonyl)-acrylonitrile; and
(E)-3-(3,5-Di-tert-butyl-4-hydroxy-phenyl)-2-(morpholine-4-sulfonyl)-acrylonitrile.

50. Use of a compound as defined in any one of claims 1 to 49 in the manufacture of a medicament for the treatment of obesity, type 2 diabetes, dyslipidemia, hypertension or gallbladder diseases; prevention of weight gain; or maintenance of weight loss.

## Patentansprüche

1. Verbindung der Formel I 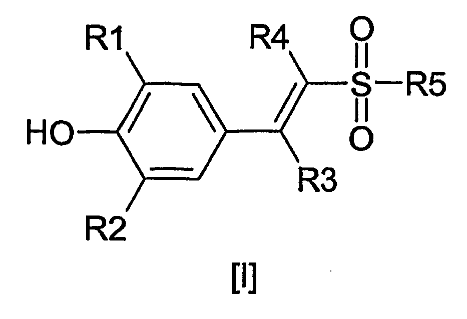
wobei R1 und R2 unabhängig für Wasserstoff, Nitro, Cyano, Halogen, Alkyl, Alkenyl, Alkinyl, Aryl, Heteroaryl, Halogenalkyl, Alkoxy, Alkylamino, -C(O)OR6, -S(O)₂OR6, -S(O)ₙR6, -OC(O)R6, -NHC(O)R6 oder -N(C(O)R6)₂ stehen;
R3 für Wasserstoff, Nitro, Cyano, Halogen, Alkyl, Alkenyl, Alkinyl, Alkoxy, Alkylamino, -C(O)OR6, -S(O)₂OR6, -S(O)ₙR6, -OC(O)R6, -NHC(O)R6 oder -N(C(O)R6)₂ steht;
R4 für Nitro, Cyano, Halogen, Halogenalkyl, -C(O)R6, -C(O)OR6, -C(O)N(R6)₂, -S(O)₂OR6, -S(O)ₙR6, -S(O)₂N(R6)₂, -P(O)(OR6)₂ oder -B(OR6)₂ steht;
R6 für Wasserstoff oder Alkyl, Aryl oder Heteroaryl steht, die alle gegebenenfalls mit einem oder mehreren Substituenten substituiert sein können, die aus Hydroxy, Halogen, Nitro und Cyano ausgewählt sind;
n für 0, 1 oder 2 steht;
R5 für Alkyl, Alkenyl, Alkinyl, Cycloalkyl, Aryl, Heteroaryl, Heterocyclyl steht, die alle gegebenenfalls mit einem oder mehreren Substituenten substituiert sein können, die aus Alkyl, Alkenyl, Alkinyl, Phenyl, Heteroaryl, Heterocyclyl, Halogen, Hydroxy, Cyano, Nitro, Carboxyl, Halogenalkyl, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -NR7-C(O)R7, NR7-S(O)ₙR7 und -(CH₂)ₚ-N(R7)(R8) ausgewählt sind, wobei das Alkyl, Alkenyl, Alkinyl, Phenyl, Heteroaryl oder Heterocyclyl gegebenenfalls mit einem oder mehreren Substituenten substituiert sind, die aus Alkyl, Halogen, Halogenalkyl, Hydroxyalkyl, Cyano, Nitro, -O-R13, -S(O)ₙR11, -O-C(O)R11, -C(O)-O-R11, -C(O)-R11, -C(O)-N(R11)(R12), -N(R11)(R12), -(CH₂)ₚ-N(R11)-C(O)-R12, -B(OR11)(OR12), -(CH₂)ₚ-O-R11 und -(CH₂)ₚ-N(R11)(R12) ausgewählt sind;
R7 und R8 unabhängig für Wasserstoff, Halogenalkyl, Hydroxyalkyl, Alkyl, Alkenyl, Alkinyl, Cycloalkyl, Heterocyclyl oder Phenyl stehen, wobei das Phenyl oder Heterocyclyl gegebenenfalls mit einem oder mehreren Substituenten substituiert sind, die aus Alkyl, Halogen, Halogenalkyl, Hydroxy, Hydroxyalkyl, Cyano, Nitro, -N(R9)(R10) und -(CH₂)ₚ-N(R9)(R10) ausgewählt sind; oder R7 und R8, wenn sie an einen Stickstoff gebunden sind, zusammen mit dem Stickstoff einen 5- bis 8-gliedrigen Heterocyclus bilden, der weiterhin mit Alkyl substituiert sein kann;
R9 und R10 unabhängig für Wasserstoff, Alkyl, Alkenyl, Alkinyl, Halogenalkyl, Hydroxyalkyl oder Cycloalkyl stehen; oder R4 und R5 zusammen mit den Atomen, an die sie angelagert sind, einen 5-, 6-, 7- oder 8-gliedrigen Ring darstellen, der gesättigt sein kann oder zum Teil oder vollständig ungesättigt sein kann, und wobei der Ring gegebenenfalls mit einem oder mehreren Substituenten substituiert ist, die aus Alkyl, Halogen, Hydroxy, Cyano und Nitro ausgewählt sind;
jedes R11 und R12 unabhängig für Wasserstoff, Halogenalkyl, Hydroxyalkyl, Alkyl, Alkenyl, Alkinyl, Cycloalkyl oder Phenyl steht;
R13 für Halogenalkyl, Hydroxyalkyl, Alkyl, Alkenyl, Alkinyl oder Cycloalkyl steht;
p für 0, 1 oder 2 steht;
oder ein pharmazeutisch unbedenkliches Salz, Solvat oder Hydrat davon;
gegebenenfalls in Kombination mit einer anderen therapeutisch wirksamen Verbindung; zur Verwendung in der Behandlung von Obesität, Typ-2-Diabetes, Dyslipidämie; Hypertonie oder Gallenblasenerkrankungen; Verhinderung von Gewichtszunahme oder Aufrechterhaltung von Gewichtsabnahme.

2. Verbindung nach Anspruch 1, wobei R1 und R2 in der Verbindung der Formel I unabhängig aus Wasserstoff, Alkyl, Alkoxy, Aryl, Heteroaryl, Halogen, Nitro, -C(O)OR6 und -S(O)₂OR6 ausgewählt sind.

3. Verbindung nach Anspruch 2, wobei R1 und R2 in der Verbindung der Formel I unabhängig für Alkyl, Halogen oder Nitro stehen.

4. Verbindung nach Anspruch 3, wobei R1 und R2 in der Verbindung der Formel I unabhängig für C₁₋₆-Alkyl stehen.

5. Verbindung nach Anspruch 2, wobei R1 und R2 in der Verbindung der Formel I beide für Methyl, Isopropyl, sek.-Butyl, tert.-Butyl, Methoxy oder Brom stehen.

6. Verbindung nach einem der Ansprüche 1 - 5, wobei R3 in der Verbindung der Formel I für Wasserstoff, Alkyl, Alkenyl, Alkinyl, Alkoxy oder Alkylamino steht.

7. Verbindung nach einem der Ansprüche 1 - 6, wobei R3 in der Verbindung der Formel I C₁₋₄-Alkyl ist.

8. Verbindung nach einem der Ansprüche 1 - 6, wobei R3 in der Verbindung der Formel I Methyl ist.

9. Verbindung nach einem der Ansprüche 1 - 8, wobei R4 in der Verbindung der Formel I für Nitro, Cyano, -C(O)R6, -C(O)OR6, -C(O)N(R6)₂, -S(O)₂OR6, -S(O)₂N(R6)₂ oder -S(O)ₙR6 steht, wobei n für 1 oder 2 steht.

10. Verbindung nach Anspruch 9, wobei R4 in der Verbindung der Formel I für Nitro steht.

11. Verbindung nach Anspruch 9, wobei R4 in der Verbindung der Formel I für Cyano steht.

12. Verbindung nach Anspruch 9, wobei R4 in der Verbindung der Formel I für -C(O)R6 steht.

13. Verbindung nach Anspruch 9, wobei R4 in der Verbindung der Formel I für -C(O)OR6 steht.

14. Verbindung nach Anspruch 9, wobei R4 in der Verbindung der Formel I für -C(O)N(R6)₂ steht.

15. Verbindung nach Anspruch 9, wobei R4 in der Verbindung der Formel I für -S(O)₂OR6 steht.

16. Verbindung nach Anspruch 9, wobei R4 in der Verbindung der Formel I für -S(O)ₙR6 steht.

17. Verbindung nach Anspruch 9, wobei R4 in der Verbindung der Formel I für -S(O)₂N(R6)₂ steht.

18. Verbindung nach einem der Ansprüche 1 - 8, wobei R4 und R5 in der Verbindung der Formel I zusammen mit den Atomen, an die sie angelagert sind, einen 5- oder 6-gliedrigen Ring darstellen, der gesättigt oder zum Teil oder vollständig ungesättigt sein kann, und wobei der Ring gegebenenfalls mit einem oder mehreren Substituenten substituiert ist, die aus Alkyl, Halogen, Hydroxy, Cyano und Nitro ausgewählt sind.

19. Verbindung nach Anspruch 18, wobei R4 und R5 in der Verbindung der Formel I zusammen mit den Atomen, an die sie angelagert sind, einen Ring darstellen, der aus [1,3]Dithiolan-1,1,3,3-tetraoxid, 1,1-Dioxotetrahydro-1-thiophen-3-on, 1,1-Dioxothiazolidin-4-on, 1,1-Dioxothiomorpholin-3-ontetrahydrothiopyran-1,1-dioxid und Tetrahydrothiophen-1,1-dioxid ausgewählt ist, die alle gegebenenfalls mit einem oder mehreren Substituenten substituiert sein können, die aus Alkyl, Halogen, Hydroxy, Cyano und Nitro ausgewählt sind.

20. Verbindung nach einem der Ansprüche 1 - 17, wobei R5 in der Verbindung der Formel I für Alkyl, Cycloalkyl, Alkenyl, Aryl, Heteroaryl oder Heterocyclyl steht, die alle gegebenenfalls mit einem oder mehreren Substituenten substituiert sein können, die aus Alkyl, Halogen, Hydroxy, Cyano, Nitro, Halogenalkyl, -O-R7, -S(O)ₙR7, -O-C(O)R7, -C(O)-O-R7, -C(O) -R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7 und NR7-S(O)ₙ-R7 ausgewählt sind.

21. Verbindung nach Anspruch 20, wobei R5 in der Verbindung der Formel I für C₁₋₁₂-Alkyl steht, das gegebenenfalls mit -OR7, -C(O)R7 oder Cycloalkyl substituiert sein kann.

22. Verbindung nach Anspruch 21, wobei R5 in der Verbindung der Formel I für Methyl, Propyl, 2-Propyl, Butyl, Pentyl, Hexyl, Octyl oder Nonyl steht.

23. Verbindung nach Anspruch 21 oder 22, wobei das Cycloalkyl Cyclopropyl, Cyclobutyl oder Cyclohexyl ist.

24. Verbindung nach einem der Ansprüche 21 - 23, wobei R7 in der Verbindung der Formel I für Wasserstoff oder Ethyl steht.

25. Verbindung nach Anspruch 20, wobei R5 für C₂₋₈-Alkenyl steht.

26. Verbindung nach Anspruch 25, wobei R5 für Pent-4-enyl oder Hex-5-enyl steht.

27. Verbindung nach Anspruch 20, wobei R5 für Cycloalkyl steht, das gegebenenfalls mit C₁₋₆-Alkyl substituiert sein kann.

28. Verbindung nach Anspruch 27, wobei R5 für Cyclopentyl, Cyclohexyl, Cycloheptyl, Cyclooctyl oder Tricyclo[2.2.1.0^{2,6}]heptyl steht.

29. Verbindung nach Anspruch 20, wobei R5 für Aryl, Phenyl oder Naphthyl steht, die alle gegebenenfalls mit einem oder mehreren Substituenten substituiert sein können, die aus Alkyl, Alkenyl, Alkinyl, Heteroaryl, Heterocyclyl, Halogen, Hydroxy, Cyano, Nitro, Halogenalkyl, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7 und NR7-S(O)ₙ-R7 ausgewählt sind.

30. Verbindung nach Anspruch 29, wobei R5 für Phenyl steht, das mit Chlor, Brom oder Iod substituiert ist.

31. Verbindung nach Anspruch 29, wobei R5 für Phenyl steht, das mit C₁₋₆-Alkyl, C₂₋₆-Alkenyl oder C₂₋₆-Alkinyl substituiert ist, die alle gegebenenfalls weiterhin mit -C(O)R7, -OR7 oder Phenyl substituiert sein können.

32. Verbindung nach Anspruch 31, wobei R7 für Wasserstoff oder C₁₋₆-Alkyl steht.

33. Verbindung nach Anspruch 29, wobei R5 für Phenyl steht, das mit Heterocyclyl substituiert ist.

34. Verbindung nach Anspruch 29, wobei R5 für Phenyl steht, das mit -OR7, -C(O)OR7 oder -S(O)₂R7 substituiert ist.

35. Verbindung nach Anspruch 34, wobei R7 für Wasserstoff, C₁₋₄-Alkyl oder C₁₋₄-Halogenalkyl steht.

36. Verbindung nach Anspruch 29, wobei R5 für Phenyl steht, das mit -C(O)-N(R7)(R8) substituiert ist.

37. Verbindung nach Anspruch 36, wobei R7 und R8 unabhängig für C₁₋₄-Alkyl oder C₁₋₄-Hydroxyalkyl stehen oder R7 und R8 zusammen mit dem Stickstoff, an den sie gebunden sind, einen 6-gliedrigen Heterocyclus bilden, der N und gegebenenfalls O umfasst, wobei der Heterocyclus gegebenenfalls weiterhin mit C₁₋₄-Alkyl substituiert sein kann.

38. Verbindung nach Anspruch 20, wobei R5 für ein Heteroaryl steht, das aus Pyridyl, Furanyl und Imidazolyl ausgewählt ist, die gegebenenfalls mit einem Substituenten substituiert sind, der aus Alkyl, Halogen, Hydroxy, Cyano, Nitro, Halogenalkyl, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7 und NR7-S(O)ₙ-R7 ausgewählt ist.

39. Verbindung nach Anspruch 38, wobei das Heteroaryl mit einem Substituenten substituiert ist, der aus Fluor, Chlor, Methyl, Hydroxy, Cyano, Nitro, -C(O)-O-R7 und -C(O)-N(R7)(R8) ausgewählt ist.

40. Verbindung nach Anspruch 20, wobei R5 für ein Heterocyclyl steht, das aus Piperidinyl, Morpholinyl, Pyrrolidinyl und Azepanyl ausgewählt ist, die gegebenenfalls mit einem Substituenten substituiert sind, der aus Alkyl, Halogen, Hydroxy, Cyano, Nitro, Halogenalkyl, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), -NR7-C(O)R7 und NR7-S(O)ₙ-R7 ausgewählt ist.

41. Verbindung nach Anspruch 40, wobei das Heterocyclyl mit einem Substituenten substituiert ist, der aus Fluor, Chlor, Hydroxy, Cyano, Nitro, -C(O)-O-R7 und -C(O)-N(R7)(R8) ausgewählt ist.

42. Verbindung nach einem der Ansprüche 1 - 41, wobei R9 und R10 in der Verbindung der Formel I unabhängig für Wasserstoff, Alkyl, Halogenalkyl oder Hydroxyalkyl stehen.

43. Verbindung nach einem der Ansprüche 1 - 42, wobei R11 und R12 unabhängig für Wasserstoff, C₁₋₆-Alkyl, C₁₋₆-Halogenalkyl oder C₁₋₆-Hydroxyalkyl stehen.

44. Verbindung nach Anspruch 43, wobei R11 und R12 unabhängig für Wasserstoff, Methyl, Ethyl, Trifluormethyl, Hydroxymethyl oder 2-Hydroxyethyl stehen.

45. Verbindung nach einem der Ansprüche 1 - 44, wobei R13 für C₁₋₆-Alkyl, C₁₋₆-Halogenalkyl oder C₁₋₆-Hydroxyalkyl steht.

46. Verbindung nach Anspruch 45, wobei R13 für Methyl, Ethyl, Trifluormethyl, Hydroxymethyl oder 2-Hydroxyethyl steht.

47. Verbindung nach einem der Ansprüche 1 - 46, wobei n in der Verbindung der Formel 1 2 ist.

48. Verbindung nach einem der Ansprüche 1 - 47, wobei p in der Verbindung der Formel I 1 ist.

49. Verbindung nach Anspruch 1, wobei die Verbindung der Formel I aus den folgenden ausgewählt ist:
(E)-3-(3,5-Di-tert.-butyl-4-hydroxyphenyl)-2-methansulfonylacrylnitril;
(E)-2-(4-Chlorbenzolsulfonyl)-3-(3,5-di-tert.-butyl-4-hydroxyphenyl)acrylnitril;
(E)-2-(4-Chlorbenzolsulfonyl)-3-(4-hydroxy-3-nitrophenyl)acrylnitril;
(E)-3-(4-Hydroxy-3-nitrophenyl)-2-methansulfonylacrylnitril;
(E)-3-(3,5-Di-tert.-butyl-4-hydroxyphenyl)-2-(1-methyl-1H-imidazol-2-sulfonyl)acrylnitril;
(E/Z)-2-(4-Chlorbenzolsulfonyl)-3-(3,5-di-tert.-butyl-4-hydroxyphenyl)but-2-ennitril;
(E)-4-[3-Cyano-1-(3,5-di-tert.-butyl-4-hydroxyphenyl)prop-1-en-2-sulfonyl]benzoesäure;
(E)-3-(3,5-Di-tert.-butyl-4-hydroxyphenyl)-2-(4-nitrobenzolsulfonyl)acrylnitril;
(E)-3-(3,5-Di-tert.-butyl-4-hydroxyphenyl)-2-(4-trifluormethoxybenzolsulfonyl)acrylnitril;
(E)-3-(3-tert.-Butyl-4-hydroxy-5-nitrophenyl)-2-(4-chlorbenzolsulfonyl)acrylnitril;
(E)-4-[3-Cyano-1-(3,5-di-tert.-butyl-4-hydroxyphenyl)prop-1-en-2-sulfonyl]-N,N-dimethylbenzamid;
(E)-4-[3-Cyano-1-(3,5-di-tert.-butyl-4-hydroxyphenyl)prop-1-en-2-sulfonyl]-N,N-bis-(2-hydroxypropyl)benzamid;
(E)-3-(3,5-Di-tert.-butyl-4-hydroxyphenyl)-2-[4-(4-methylpiperazin-1-carbonyl)benzolsulfonyl]acrylnitril;
(E)-4-[3-Cyano-1-(3,5-di-tert.-butyl-4-hydroxyphenyl)prop-1-en-2-sulfonyl]-N,N-(2-hydroxyethyl)benzamid;
(E)-3-(3,5-Di-tert.-butyl-4-hydroxyphenyl)-2-[4-(2,6-dimethylmorpholin-4-carbonyl)benzolsulfonyl]acrylnitril;
(E)-3-(3,5-Di-tert.-butyl-4-hydroxyphenyl)-2-[4-(morpholin-4-carbonyl)benzolsulfonyl]acrylnitril;
(E)-2-(4-Aminobenzolsulfonyl)-3-(3,5-di-tert.-butyl-4-hydroxyphenyl)acrylnitril;
(E)-2-[2-Cyano-1-(3,5-di-tert.-butyl-4-hydroxyphenyl)ethensulfonyl]-3-(3,5-di-tert.-butyl-4-hydroxyphenyl)acrylnitril;
(E)-2-(4-Chlorphenylmethansulfonyl)-3-(3,5-di-tert.-butyl-4-hydroxyphenyl)acrylnitril;
(E)-3-(3,5-Di-tert.-butyl-4-hydroxyphenyl)-2-(propan-2-sulfonyl)acrylnitril;
4-[3-Cyano-1-(3,5-di-tert.-butyl-4-hydroxyphenyl)prop-1-en-2-sulfonyl]-N,N-bis-(2-hydroxyethyl)benzamid;
3-(3,5-Di-tert.-butyl-4-hydroxyphenyl)-2-[4-(3,5-dimethylmorpholin-4-carbonyl)benzolsulfonyl]acrylnitril; 3-(3,5-Di-tert.-butyl-4-hydroxyphenyl)-2-(2,5-dichlorbenzolsulfonyl)acrylnitril;
2-(4-tert.-Butylbenzolsulfonyl)-3-(3,5-di-tert.-butyl-4-hydroxyphenyl)acrylnitril;
3-(3,5-Di-tert.-butyl-4-hydroxyphenyl)-2-(naphthalin-1-sulfonyl)acrylnitril;
3-(3,5-Di-tert.-butyl-4-hydroxyphenyl)-2-(furan-2-ylmethansulfonyl)acrylnitril;
3-(3,5-Di-tert.-butyl-4-hydroxyphenyl)-2-(2,4-dichlorbenzolsulfonyl)acrylnitril;
3-(3,5-Di-tert.-butyl-4-hydroxyphenyl)-2-(toluol-4-sulfonyl)acrylnitril;
3-(3,5-Di-tert.-butyl-4-hydroxyphenyl)-2-(4-isopropylbenzolsulfonyl)acrylnitril;
2-(3,5-Di-tert.-butyl-4-hydroxybenzolsulfonyl)-3-(3,5-di-tert.-butyl-4-hydroxyphenyl)acrylnitril;
3-(3,5-Di-tert.-butyl-4-hydroxyphenyl)-2-(hexan-1-sulfonyl)acrylnitril;
2-(4-Brombenzolsulfonyl)-3-(3,5-di-tert.-butyl-4-hydroxyphenyl)acrylnitril;
3-(3,5-Di-tert.-butyl-4-hydroxyphenyl)-2-(4-methansulfonylbenzolsulfonyl)acrylnitril;
3-(3,5-Di-tert.-butyl-4-hydroxyphenyl)-2-(3-trifluormethoxybenzolsulfonyl)acrylnitril;
3-(3,5-Di-tert.-butyl-4-hydroxyphenyl)-2-(2-trifluormethoxybenzolsulfonyl)acrylnitril;
2-(5-Chlorpyridin-2-sulfonyl)-3-(3,5-di-tert.-butyl-4-hydroxyphenyl)acrylnitril;
2-[3-Cyano-1-(3,5-di-tert.-butyl-4-hydroxyphenyl)prop-1-en-2-sulfonyl]benzoesäure;
3-[3-Cyano-1-(3,5-di-tert.-butyl-4-hydroxyphenyl)prop-1-en-2-sulfonyl]benzoesäure;
3-(3,5-Di-tert.-butyl-4-hydroxyphenyl)-2-[2-(morpholin-4-carbonyl)benzolsulfonyl]acrylnitril;
3-(3,5-Di-tert.-butyl-4-hydroxyphenyl)-2-[3-(morpholin-4-carbonyl)benzolsulfonyl]acrylnitril;
3-(3,5-Di-tert.-butyl-4-hydroxyphenyl)-2-(4-methoxybenzolsulfonyl)acrylnitril;
3-(3,5-Di-tert.-butyl-4-hydroxyphenyl)-2-(3-methoxybenzolsulfonyl)acrylnitril;
3-(3,5-Di-tert.-butyl-4-hydroxyphenyl)-2-(3,4-dimethoxybenzolsulfonyl)acrylnitril;
3-(3,5-Di-tert.-butyl-4-hydroxyphenyl)-2-[4-(2,3-dihydrofuran-2-yl)benzolsulfonyl]acrylnitril;
3-{4-[2-Cyano-1-(3,5-di-tert.-butyl-4-hydroxyphenyl)ethensulfonyl]phenyl}-2-methylacrylsäuremethylester;
3-(3,5-Di-tert.-butyl-4-hydroxyphenyl)-2-(4-styrylbenzolsulfonyl)acrylnitril;
3-(3,5-Di-tert.-butyl-4-hydroxyphenyl)-2-[4-(2-isobutoxyvinyl)benzolsulfonyl]acrylnitril;
2-[4-(2-Butoxyvinyl)benzolsulfonyl]-3-(3,5-di-tert.-butyl-4-hydroxyphenyl)acrylnitril;
3-(3,5-Di-tert.-butyl-4-hydroxyphenyl)-2-(4-phenylethinylbenzolsulfonyl)acrylnitril;
3-(3,5-Di-tert.-butyl-4-hydroxyphenyl)-2-(2-ethoxyethansulfonyl)acrylnitril;
3-(3,5-Di-tert.-butyl-4-hydroxyphenyl)-2-[2-(2-ethoxyethoxy)ethansulfonyl]acrylnitril;
2-(4-Chlorbenzolsulfonyl)-3-(3,5-di-sek.-butyl-4-hydroxyphenyl)acrylnitril;
(E)-2-(3-Brombenzolsulfonyl)-3-(3,5-di-tert.-butyl-4-hydroxyphenyl)acrylnitril;
3-(3,5-Di-tert.-butyl-4-hydroxyphenyl)-2-(hex-5-en-1-sulfonyl)acrylnitril;
(E)-2-(4-Iodbenzolsulfonyl)-3-(3,5-di-tert.-butyl-4-hydroxyphenyl)acrylnitril;
(E)-2-Cyclopentansulfonyl-3-(3,5-di-tert.-butyl-4-hydroxyphenyl)acrylnitril;
(E)-3-(3,5-Di-tert.-butyl-4-hydroxyphenyl)-2-(nonan-1-sulfonyl)acrylnitril;
(E)-3-(3,5-Di-tert.-butyl-4-hydroxyphenyl)-2-(pentan-1-sulfonyl)acrylnitril;
(E)-3-(3,5-Di-tert.-butyl-4-hydroxyphenyl)-2-(heptan-1-sulfonyl)acrylnitril;
(E)-2-Cyclohexansulfonyl-3-(3,5-di-tert.-butyl-4-hydroxyphenyl)acrylnitril;
(E)-3-{4-[2-Cyano-1-(3,5-di-tert.-butyl-4-hydroxyphenyl)ethensulfonyl]phenyl}propionsäure;
(E)-3-(3,5-Di-tert.-butyl-4-hydroxyphenyl)-2-(4-methyl-2-oxo-2H-chromen-7-sulfonyl)acrylnitril;
(E)-3-(3,5-Di-tert.-butyl-4-hydroxyphenyl)-2-(4-trifluormethoxyphenylmethansulfonyl)acrylnitril;
(E)-2-Cyclohexylmethansulfonyl-3-(3,5-di-tert.-butyl-4-hydroxyphenyl)acrylnitril;
(E)-2-(Butan-1-sulfonyl)-3-(3,5-di-tert.-butyl-4-hydroxyphenyl)acrylnitril;
(E)-2-(Propan-1-sulfonyl)-3-(3,5-di-tert.-butyl-4-hydroxyphenyl)acrylnitril;
(E)-2-(Ethan-1-sulfonyl)-3-(3,5-di-tert.-butyl-4-hydroxyphenyl)acrylnitril;
(E)-2-Cyclopropylmethansulfonyl-3-(3,5-di-tert.-butyl-4-hydroxyphenyl)acrylnitril;
(E)-2-Cycloheptansulfonyl-3-(3,5-di-tert.-butyl-4-hydroxyphenyl)acrylnitril;
(E)-2-Cyclobutylmethansulfonyl-3-(3,5-di-tert.-butyl-4-hydroxyphenyl)acrylnitril;
(E)-3-(3,5-Di-tert.-butyl-4-hydroxyphenyl)-2-(tricyclo[2.2.1.0^{2,6}] heptan-3-sulfonyl) acrylnitril;
(E)-2-Cyclooctansulfonyl-3-(3,5-di-tert.-butyl-4-hydroxyphenyl)acrylnitril;
(E)-3-(3-tert.-Butyl-4-hydroxy-5-methylphenyl)-2-(4-trifluormethoxybenzolsulfonyl)acrylnitril;
(E)-3-(3-tert.-Butyl-4-hydroxy-5-methylphenyl)-2-(3-trifluormethoxybenzolsulfonyl)acrylnitril;
(E)-3-(3,5-Di-tert.-butyl-4-hydroxyphenyl)-2-(5-methylhexan-1-sulfonyl)acrylnitril;
(E)-2-(2-Cyclohexylethansulfonyl)-3-(3,5-di-tert.-butyl-4-hydroxyphenyl)acrylnitril;
(E)-3-(3,5-Di-tert.-butyl-4-hydroxyphenyl)-2-(4-methylpentan-1-sulfonyl)acrylnitril;
(E)-3-(3-tert.-Butyl-4-hydroxy-5-methylphenyl)-2-(hexan-1-sulfonyl)acrylnitril;
(E)-3-(4-Hydroxy-3,5-diisopropylphenyl)-2-(4-trifluormethoxybenzolsulfonyl)acrylnitril;
(E)-3-(4-Hydroxy-3,5-diisopropylphenyl)-2-(3-trifluormethoxybenzolsulfonyl)acrylnitril;
(E)-2-(4-Chlorbenzolsulfonyl)-3-(4-hydroxy-3,5-dimethylphenyl)acrylnitril;
(E)-2-(4-Chlorbenzolsulfonyl)-3-(4-hydroxy-3,5-dibromphenyl)acrylnitril;
(E)-2-(4-Chlorbenzolsulfonyl)-3-(4-hydroxy-3,5-dimethoxyphenyl)acrylnitril;
(E)-2-(4-Chlorbenzolsulfonyl)-3-(4-hydroxy-3-iod-5-methoxyphenyl)acrylnitril;
(E)-2-(Hexan-1-sulfonyl)-3-(4-hydroxy-3,5-diisopropylphenyl)acrylnitril;
(E)-3-(3,5-Di-tert.-butyl-4-hydroxyphenyl)-2-(2-methoxyethansulfonyl)acrylnitril;
(E)-3-(3,5-Di-tert.-butyl-4-hydroxyphenyl)-2-(pent-4-en-1-sulfonyl)acrylnitril;
(E)-3-(3,5-Di-tert.-butyl-4-hydroxyphenyl)-2-(piperidin-1-sulfonyl)acrylnitril;
(E)-3-(3,5-Di-tert.-butyl-4-hydroxyphenyl)-2-(azepan-1-sulfonyl)acrylnitril;
(E)-3-(3,5-Di-tert.-butyl-4-hydroxyphenyl)-2-(pyrroldin-1-sulfonyl)acrylnitril und
(E)-3-(3,5-Di-tert.-butyl-4-hydroxyphenyl)-2-(morpholin-4-sulfonyl)acrylnitril.

50. Verwendung einer wie in einem der Ansprüche 1 bis 49 definierten Verbindung bei der Herstellung eines Arzneimittels zur Behandlung von Obesität, Typ-2-Diabetes, Dyslipidämie, Hypertonie oder Gallenblasenerkrankungen; Verhinderung von Gewichtszunahme oder Aufrechterhaltung von Gewichtsabnahme.

## Revendications

1. Composé de formule I 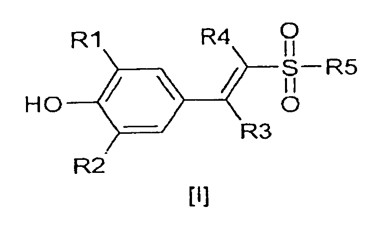
dans laquelle R1 et R2 représentent indépendamment hydrogène, nitro, cyano, halogène, alkyle, alcényle, alcynyle, aryle, hétéroaryle, haloalkyle, alkoxy, alkylamino, -C(O)OR6, -S(O)₂OR6, -S(O)ₙR6, -OC(O)R6, -NHC(O)R6 OU -N(C(O)R6)₂ ;
R3 représente hydrogène, nitro, cyano, halogène, alkyle, alcényle, alcynyle, alkoxy, alkylamino, -C(O)OR6, -S(O)₂OR6, -S(O)ₙR6, -OC(O)R6, -NHC(O)R6 ou -N(C(O)R6)₂ ;
R4 représente nitro, cyano, halogène, haloalkyle, -C(O)R6, -C(O)OR6, -C(O)N(R6)₂, -S(O)₂OR6, -S(O)ₙR6, -S(O)₂N(R6)₂, -P(O)(OR6)₂ ou -B(OR6)₂ ;
R6 représente hydrogène ou alkyle, aryle ou hétéroaryle, qui peuvent tous être éventuellement substitués par un ou plusieurs substituants sélectionnés parmi hydroxy, halogène, nitro et cyano ;
n représente 0, 1 ou 2 ;
R5 représente alkyle, alcényle, alcynyle, cycloalkyle, aryle, hétéroaryle, hétérocyclyle, qui peuvent tous être éventuellement substitués par un ou plusieurs substituants sélectionnés parmi alkyle, alcényle, alcynyle, phényle, hétéroaryle, hétérocyclyle, halogène, hydroxy, cyano, nitro, carboxyle, haloalkyle, -O-R7, -S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -NR7-C(O)R7, NR7-S(O)ₙR7 et -(CH₂)ₚ-N(R7)(R8), lesdits alkyle, alcényle, alcynyle, phényle, hétéroaryle ou hétérocyclyle étant éventuellement substitués par un ou plusieurs substituants sélectionnés parmi alkyle, halogène, haloalkyle, hydroxyalkyle, cyano, nitro, O-R13, -S(O)ₙR11, -O-C(O)R11, -C(O)O-R11, -C(O)-R11, -C(O)-N(R11)(R12), -N(R11)(R12), -(CH₂)ₚ-N(R11)-C(O)-R12, -B(OR11)(OR12), (CH₂)ₚ-O-R11, et -(CH₂)ₚ-N(R11)(R12) ;
R7 et R8 représentent indépendamment hydrogène, haloalkyle, hydroxyalkyle, alkyle, alcényle, alcynyle, cycloalkyle, hétérocyclyle ou phényle, lesdits phényle ou hétérocyclyle étant éventuellement substitués par un ou plusieurs substituants sélectionnés parmi alkyle, halogène, haloalkyle, hydroxy, hydroxy-alkyle, cyano, nitro, -N(R9) (R10) et -(CH₂)ₚ-N(R9) (R10) ; ou R7 et R8 lorsqu'ils sont liés à un azote forment avec ledit azote un hétérocycle à 5 à 8 chaînons qui peut être davantage substitué par un alkyle ;
R9 et R10 représentent indépendamment hydrogène, alkyle, alcényle, alcynyle, haloalkyle, hydroxyalkyle, ou cycloalkyle ;
ou R4 et R5 avec les atomes auxquels ils sont attachés constituent un noyau à 5, 6, 7 ou 8 chaînons, qui peut être saturé ou peut être partiellement ou totalement insaturé, et dans lequel ledit noyau est éventuellement substitué par un ou plusieurs substituants sélectionnés parmi alkyle, halogène, hydroxy, cyano et nitro ;
chaque R11 et R12 représente indépendamment hydrogène, haloalkyle, hydroxyalkyle, alkyle, alcényle, alcynyle, cycloalkyle ou phényle ;
R13 représente haloalkyle, hydroxyalkyle, alkyle, alcényle, alcynyle ou cycloalkyle ;
p représente 0, 1 ou 2 ;
ou un sel, un solvate ou un hydrate de ceux-ci pharmaceutiquement acceptable ;
éventuellement en combinaison avec un autre composé thérapeutiquement actif ; à utiliser dans le traitement de l'obésité, du diabète de type 2, de la dyslipidémie ; de l'hypertension ou des maladies de la vésicule biliaire ; la prévention de la prise de poids ; ou le maintien de la perte de poids.

2. Composé selon la revendication 1, dans lequel R1 et R2 dans ledit composé de formule I sont indépendamment sélectionnés parmi hydrogène, alkyle, alkoxy, aryle, hétéroaryle, halogène, nitro, -C(O)OR6 et -S(O)₂OR6.

3. Composé selon la revendication 2, dans lequel R1 et R2 dans ledit composé de formule I représentent indépendamment alkyle, halogène ou nitro.

4. Composé selon la revendication 3, dans lequel R1 et R2 dans ledit composé de formule I représentent indépendamment alkyle en C₁-C₆.

5. Composé selon la revendication 2, dans lequel R1 et R2 dans ledit composé de formule I représentent tous les deux méthyle, isopropyle, sec-butyle, tert-butyle, méthoxy ou bromo.

6. Composé selon l'une quelconque des revendications 1 à 5, dans lequel R3 dans ledit composé de formule I représente hydrogène, alkyle, alcényle, alcynyle, alkoxy ou alkylamino.

7. Composé selon l'une quelconque des revendications 1 à 6, dans lequel R3 dans ledit composé de formule I est alkyle en C₁-C₄.

8. Composé selon l'une quelconque des revendications 1 à 6, dans lequel R3 dans ledit composé de formule I est méthyle.

9. Composé selon l'une quelconque des revendications 1 à 8, dans lequel R4 dans ledit composé de formule I représente nitro, cyano, -C(O)R6, -C(O)OR6, -C(O)N(R6)₂, -S(O)₂OR6, -S(O)₂N(R6)₂ ou -S(O)ₙR6, dans lequel n représente 1 ou 2.

10. Composé selon la revendication 9, dans lequel R4 dans ledit composé de formule I représente nitro.

11. Composé selon la revendication 9, dans lequel R4 dans ledit composé de formule I représente cyano.

12. Composé selon la revendication 9, dans lequel R4 dans ledit composé de formule I représente -C(O)R6.

13. Composé selon la revendication 9, dans lequel R4 dans ledit composé de formule I représente -C(O)OR6.

14. Composé selon la revendication 9, dans lequel R4 dans ledit composé de formule I représente -C(O)N(R6)₂.

15. Composé selon la revendication 9, dans lequel R4 dans ledit composé de formule I représente -S(O)₂OR6.

16. Composé selon la revendication 9, dans lequel R4 dans ledit composé de formule I représente -S(O)ₙR6.

17. Composé selon la revendication 9, dans lequel R4 dans ledit composé de formule I représente -S(O)₂N(R6)₂.

18. Composé selon l'une quelconque des revendications 1 à 8, dans lequel R4 et R5 dans ledit composé de formule I constituent avec les atomes auxquels ils sont attachés un noyau à 5 ou 6 chaînons qui peut être saturé, ou partiellement ou totalement insaturé, et dans lequel ledit noyau est éventuellement substitué par un ou plusieurs substituants sélectionnés parmi alkyle, halogène, hydroxy, cyano et nitro.

19. Composé selon la revendication 18, dans lequel R4 et R5 dans ledit composé de formule I constituent avec les atomes auxquels ils sont attachés un noyau sélectionné parmi [1,3]dithiolane, 1,1,3,3-tétraoxyde, 1,1-dioxo-tétrahydro-1-thiophèn-3-one, 1,1-dioxo-thiazolidine-4-one, 1,1-dioxo-thiomorpholine-3-one, tétrahydrothiopyran-1,1-dioxyde et tétrahydrothiophèn-1,1-dioxyde, qui tous peuvent être éventuellement substitués par un ou plusieurs substituants sélectionnés parmi alkyle, halogène, hydroxy, cyano et nitro.

20. Composé selon l'une quelconque des revendications 1 à 17, dans lequel R5 dans ledit composé de formule I représente alkyle, cycloalkyle, alcényle, aryle, hétéroaryle ou hétérocyclyle, qui tous peuvent être éventuellement substitués par un ou plusieurs substituants sélectionnés parmi alkyle, halogène, hydroxy, cyano, nitro, haloalkyle, -O-R7, S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), NR7-C(O)R7 et -NR7-S(O)ₙ-R7.

21. Composé selon la revendication 20, dans lequel R5 dans ledit composé de formule I représente alkyle en C₁-C₁₂ qui peut être éventuellement substitué par -OR7, -C(O)R7 ou cycloalkyle.

22. Composé selon la revendication 21, dans lequel R5 dans ledit composé de formule I représente méthyle, propyle, 2-propyle, butyle, pentyle, hexyle, octyle ou nonyle.

23. Composé selon la revendication 21 ou la revendication 22, dans lequel ledit cycloalkyle est cyclopropyle, cyclobutyle ou cyclohexyle.

24. Composé selon l'une quelconque des revendications 21 à 23, dans lequel R7 dans ledit composé de formule I représente hydrogène ou éthyle.

25. Composé selon la revendication 20, dans lequel R5 représente alcényle en C₂-C₈.

26. Composé selon la revendication 25, dans lequel R5 représente pent-4-ényle ou hex-5-ényle.

27. Composé selon la revendication 20, dans lequel R5 représente cycloalkyle, qui peut être éventuellement substitué par alkyle en C₁-C₆.

28. Composé selon la revendication 27, dans lequel R5 représente cyclopentyle, cyclohexyle, cycloheptyle, cyclooctyle ou tricyclo[2.2.1.0^{2,6}]heptyle.

29. Composé selon la revendication 20, dans lequel R5 représente aryle, phényle ou naphtyle, qui tous peuvent être éventuellement substitués par un ou plusieurs substituants sélectionnés parmi alkyle, alcényle, alcynyle, hétéroaryle, hétérocyclyle, halogène, hydroxy, cyano, nitro, haloalkyle, -O-R7, S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR₇)(OR₈), -(CH₂)ₚ-O-R7, -(CH₂)ₚ-N(R7)(R8), NR7-C(O)R7 et -NR7-S(O)ₙ-R7.

30. Composé selon la revendication 29, dans lequel R5 représente phényle substitué par chloro, bromo ou iodo.

31. Composé selon la revendication 29, dans lequel R5 représente phényle substitué par alkyle en C₁-C₆, alcényle en C₂-C₆ ou alcynyle en C₂-C₆, qui tous peuvent être éventuellement davantage substitués par -C(O)R7, -OR7 ou phényle.

32. Composé selon la revendication 31, dans lequel R7 représente hydrogène ou alkyle en C₁-C₆.

33. Composé selon la revendication 29, dans lequel R5 représente phényle substitué par hétérocyclyle.

34. Composé selon la revendication 29, dans lequel R5 représente phényle substitué par -OR7, -C(O)OR7 ou -S(O)₂R7.

35. Composé selon la revendication 34, dans lequel R7 représente hydrogène, alkyle en C₁-C₄ ou haloalkyle en C₁-C₄.

36. Composé selon la revendication 29, dans lequel R5 représente phényle substitué par -C(O)-N(R7)(R8).

37. Composé selon la revendication 36, dans lequel R7 et R8 représentent indépendamment alkyle en C₁-C₄ ou hydroxyalkyle en C₁-C₄, ou R7 et R8 forment avec l'azote auquel ils sont liés un hétérocycle à 6 chaînons comprenant N et éventuellement O, dans lequel ledit hétérocycle peut être éventuellement davantage substitué par alkyle en C₁-C₄.

38. Composé selon la revendication 20, dans lequel R5 représente un hétéroaryle sélectionné parmi pyridyle, furanyle et imidazolyle, éventuellement substitué par un substituant sélectionné parmi alkyle, halogène, hydroxy, cyano, nitro, haloalkyle, -O-R7, S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH₂)ₚ-O-R7, -(CH₂)ₚ-N(R7)(R8), NR7-C(O)R7 et -NR7-S(O)ₙ-R7.

39. Composé selon la revendication 38, dans lequel ledit hétéroaryle est substitué par un substituant sélectionné parmi fluoro, chloro, méthyle, hydroxy, cyano, nitro, -C (O) -O-R7 et -C (O) -N (R7) (R8) .

40. Composé selon la revendication 20, dans lequel R5 représente un hétérocyclyle sélectionné parmi pipéridinyle, morpholinyle, pyrrolidinyle et azépanyle, éventuellement substitué par un substituant sélectionné parmi alkyle, halogène, hydroxy, cyano, nitro, haloalkyle, -O-R7, S(O)ₙR7, -O-C(O)R7, -C(O)-O-R7, -C(O)-R7, -C(O)-N(R7)(R8), -N(R7)(R8), -(CH₂)ₚ-N(R8)-C(O)-R7, -B(OR7)(OR8), -(CH2)ₚ-OR7, -(CH₂)ₚ-N(R7)(R8), NR7-C(O)R7 et -NR7-S(O)ₙ-R7.

41. Composé selon la revendication 40, dans lequel ledit hétérocyclyle est substitué par un substituant sélectionné parmi fluoro, chloro, hydroxy, cyano, nitro, -C(O)-O-R7 et -C(O)-N(R7)(R8).

42. Composé selon l'une quelconque des revendications 1 à 41, dans lequel R9 et R10 dans ledit composé de formule I représentent indépendamment hydrogène, alkyle, haloalkyle ou hydroxyalkyle.

43. Composé selon l'une quelconque des revendications 1 à 42, dans lequel R11 et R12 représentent indépendamment hydrogène, alkyle en C₁-C₆, haloalkyle en C₁-C₆ ou hydroxyalkyle en C₁-C₆.

44. Composé selon la revendication 43, dans lequel R11 et R12 représentent indépendamment hydrogène, méthyle, éthyle, trifluorométhyle, hydroxyméthyle ou 2-hydroxyéthyle.

45. Composé selon l'une quelconque des revendications 1 à 44, dans lequel R13 représente alkyle en C₁-C₆, haloalkyle en C₁-C₆ ou hydroxyalkyle en C₁-C₆.

46. Composé selon la revendication 45, dans lequel R13 représente méthyle, éthyle, trifluorométhyle, hydroxyméthyle ou 2-hydroxyéthyle.

47. Composé selon l'une quelconque des revendications 1 à 46, dans lequel n dans ledit composé de formule I est 2.

48. Composé selon l'une quelconque des revendications 1 à 47, dans lequel p dans ledit composé de formule I est 1.

49. Composé selon la revendication 1, dans lequel ledit composé de formule I est sélectionné parmi :
(E)-3-(3,5-di-tert-butyl-4-hydroxy-phényl)-2-méthanesulfonyl-acrylonitrile ;
(E)-2-(4-chloro-benzènesulfonyl)-3-(3,5-di-tert-butyl-4-hydroxy-phényl)-acrylonitrile ;
(E)-2-(4-chloro-benzènesulfonyl)-3-(4-hydroxy-3-nitro-phényl)-acrylonitrile ;
(E)-3-(4-hydroxy-3-nitro-phényl)-2-méthanesulfonyl-acrylonitrile ;
(E)-3-(3,5-di-tert-butyl-4-hydroxy-phényl)-2-(1-méthyl-1H-imidazole-2-sulfonyl)-acrylonitrile ;
(EIZ)-2-(4-chloro-benzènesulfonyl)-3-(3,5-di-tert-butyl-4-hydroxy-phényl)-but-2-ènenitrile ;
acide benzoïque de (E)-4-[3-cyano-1-(3,5-di-tert-butyl-4-hydroxy-phényl)-prop-1-ène-2-sulfonyle] ;
(E)-3-(3,5-di-tert-butyl-4-hydroxy-phényl)-2-(4-nitro-benzènesulfonyl)-acrylonitrile ;
(E)-3-(3,5-di-tert-butyl-4-hydroxy-phényl)-2-(4-trifluorométhoxy-benzènesulfonyl)-acrylonitrile ;
(E)-3-(3-tert-butyl-4-hydroxy-5-nitro-phényl)-2-(4-chloro-benzènesulfonyl)-acrylonitrile ;
(E)-4-[3-cyano-1-(3,5-di-tert-butyl-4-hydroxy-phényl)-prop-1-ène-2-sulfonyl]-N,N-diméthylbenzamide ;
(E)-4-[3-cyano-1-(3,5-di-tert-butyl-4-hydroxy-phényl)-prop-1-ène-2-sulfonyl]-N,N-bis-(2-hydroxy-propyl)-benzamide ;
(E)-3-(3,5-di-tert-butyl-4-hydroxy-phényl)-2-[4-(4-méthyl-pipérazine-1-carbonyl)-benzènesulfonyl]-acrylonitrile ;
(E)-4-[3-cyano-1-(3,5-di-tert-butyl-4-hydroxy-phényl)-prop-1-ène-2-sulfonyl]-N-(2-hydroxy-éthyl)-benzamide ;
(E)-3-(3,5-di-tert-butyl-4-hydroxy-phényl)-2-[4-(2,6-diméthyl-morpholine-4-carbonyl)-benzènesulfonyl]-acrylonitrile ;
(E)-3-(3,5-di-tert-butyl-4-hydroxy-phényl)-2-[4-(morpholine-4-carbonyl)-benzènesulfonyl]-acrylonitrile ;
(E)-2-(4-amino-benzènesulfonyl)-3-(3,5-di-tert-butyl-4-hydroxy-phényl)-acrylonitrile ;
(E)-2-[2-cyano-1-(3,5-di-tert-butyl-4-hydroxy-phényl]-éthènesulfonyl]-3-(3,5-di-tert-butyl-4-hydroxy-phényl)-acrylonitrile ;
(E)-2-(4-chloro-phénylméthanesulfonyl)-3-(3,5-di-tert-butyl-4-hydroxy-phényl)-acrylonitrile ;
(E)-3-(3,5-di-tert-butyl-4-hydroxy-phényl)-2-(propane-2-sulfonyl)-acrylonitrile ;
4-[3-cyano-1-(3,5-di-tert-butyl-4-hydroxy-phényl)-prop-1-ène-2-sulfonyl]-N,N-bis-(2-hydroxyéthyl)-benzamide ;
3-(3,5-di-tert-butyl-4-hydroxy-phényl)-2-[4-(3,5-diméthyl-morpholine-4-carbonyl)-benzènesulfonyl]-acrylonitrile ;
3-(3,5-di-tert-butyl-4-hydroxy-phényl)-2-(2,5-dichloro-benzènesulfonyl)-acrylonitrile ;
2-(4-tert-butyl-benzènesulfonyl)-3-(3,5-di-tert-butyl-4-hydroxy-phényl)-acrylonitrile ;
3-(3,5-di-tert-butyl-4-hydroxy-phényl)-2-(naphtalène-1-sulfonyl)-acrylonitrile ;
3-(3,5-di-tert-butyl-4-hydroxy-phényl)-2-(furan-2-ylméthanesulfonyl)-acrylonitrile ;
3-(3,5-di-tert-butyl-4-hydroxy-phényl)-2-(2,4-dichloro-benzènesulfonyl)-acrylonitrile ;
3-(3,5-di-tert-butyl-4-hydroxy-phényl)-2-(toluène-4-sulfonyl)-acrylonitrile ;
3-(3,5-di-tert-butyl-4-hydroxy-phényl)-2-(4-isopropyl-benzènesulfonyl)-acrylonitrile ;
2-(3,5-di-tert-butyl-4-hydroxy-benzènesulfonyl)-3-(3,5-di-tert-butyl-4-hydroxy-phényl)-acrylonitrile ;
3-(3,5-di-tert-butyl-4-hydroxy-phényl)-2-(hexane-1-sulfonyl)-acrylonitrile ;
2-(4-bromo-benzènesulfonyl)-3-(3,5-di-tert-butyl-4-hydroxy-phényl)-acrylonitrile ;
3-(3,5-di-tert-butyl-4-hydroxy-phényl)-2-(4-méthanesulfonyl-benzènesulfonyl)-acrylonitrile ;
3-(3,5-di-tert-butyl-4-hydroxy-phényl)-2-(3-trifluorométhoxy-benzènesulfonyl)-acrylonitrile ;
3-(3,5-di-tert-butyl-4-hydroxy-phényl)-2-(2-trifluorométhoxy-benzènesulfonyl)-acrylonitrile ;
2-(5-chloro-pyridine-2-sulfonyl)-3-(3,5-di-tert-butyl-4-hydroxy-phényl)-acrylonitrile ;
acide benzoïque de 2-[3-cyano-1-(3,5-di-tert-butyl-4-hydroxy-phényl)-prop-1-ène-2-sulfonyle] ;
acide benzoïque de 3-[3-cyano-1-(3,5-di-tert-butyl-4-hydroxy-phényl)-prop-1-ène-2-sulfonyle] ;
3-(3,5-di-tert-butyl-4-hydroxy-phényl)-2-[2-(morpholine-4-carbonyl)-benzènesulfonyl]-acrylonitrile ;
3-(3,5-di-tert-butyl-4-hydroxy-phényl)-2-[3-(morpholine-4-carbonyl)-benzènesulfonyl]-acrylonitrile ;
3-(3,5-di-tert-butyl-4-hydroxy-phényl)-2-(4-méthoxy-benzènesulfonyl)-acrylonitrile ;
3-(3,5-di-tert-butyl-4-hydroxy-phényl)-2-(3-méthoxy-benzènesulfonyl)-acrylonitrile ;
3-(3,5-di-tert-butyl-4-hydroxy-phényl)-2-(3,4-diméthoxy-benzènesulfonyl)-acrylonitrile ;
3-(3,5-di-tert-butyl-4-hydroxy-phényl)-2-[4-(2,3-dihydro-furan-2-yl)-benzènesulfonyl]-acrylonitrile ;
ester méthylique d'acide acrylique de 3-{4-[2-cyano-1-(3,5-di-tert-butyl-4-hydroxy-phényl)-éthènesulfonyl]-phényl}-2-méthyle ;
3-(3,5-di-tert-butyl-4-hydroxy-phényl)-2-(4-styryl-benzènesulfonyl)-acrylonitrile ;
3-(3,5-di-tert-butyl-4-hydroxy-phényl)-2-[4-(2-isobutoxy-vinyl)-benzènesulfonyl]-acrylonitrile ;
2-[4-(2-butoxy-vinyl)-benzènesulfonyl]-3-(3,5-di-tert-butyl-4-hydroxy-phényl)-acrylonitrile ;
3-(3,5-di-tert-butyl-4-hydroxy-phényl)-2-(4-phényléthynyl-benzènesulfonyl)-acrylonitrile ;
3-(3,5-di-tert-butyl-4-hydroxy-phényl)-2-(2-éthoxy-éthanesulfonyl)-acrylonitrile ;
3-(3,5-di-tert-butyl-4-hydroxy-phényl)-2-[2-(2-éthoxy-éthoxy)-éthanesulfonyl]-acrylonitrile ;
2-(4-chloro-benzènesulfonyl)3-(3,5-di-tert-butyl-4-hydroxy-phényl)-acrylonitrile ;
(E)-2-(3-bromo-benzènesulfonyl)-3-(3,5-di-tert-butyl-4-hydroxy-phényl)-acrylonitrile ;
3-(3,5-di-tert-butyl-4-hydroxy-phényl)-2-(hex-5-ène-1-sulfonyl)-acrylonitrile ;
(E)-2-(4-iodo-benzènesulfonyl)-3-(3,5-di-tert-butyl-4-hydroxy-phényl)-acrylonitrile ;
(E)-2-cyclopentanesulfonyl-3-(3,5-di-tert-butyl-4-hydroxy-phényl)-acrylonitrile ;
(E)-3-(3,5-di-tert-butyl-4-hydroxy-phényl)-2-(nonane-1-sulfonyl)-acrylonitrile ;
(E)-3-(3,5-di-tert-butyl-4-hydroxy-phényl)-2-(pentane-1-sulfonyl)-acrylonitrile ;
(E)-3-(3,5-di-tert-butyl-4-hydroxy-phényl)-2-(heptane-1-sulfonyl)-acrylonitrile ;
(E)-2-cyclohexanesulfonyl-3-(3,5-di-tert-butyl-4-hydroxy-phényl)-acrylonitrile ;
acide propionique de (E)-3-{4-[2-cyano-1-(3,5-di-tert-butyl-4-hydroxy-phényl)-éthènesulfonyl]-phényle} ;
(E)-3-(3,5-di-tert-butyl-4-hydroxy-phényl)-2-(4-méthyl-2-oxo-2H-chromène-7-sulfonyl)-acrylonitrile ;
(E)-3-(3,5-di-tert-butyl-4-hydroxy-phényl)-2-(trifluorométhoxy-phénylméthanesulfonyl)-acrylonitrile ;
(E)-2-cyclohexylméthanesulfonyl-3-(3,5-di-tert-butyl-hydroxy-phényl)-acrylonitrile ;
(E)-2-(butane-1-sulfonyl)-3-(3,5-di-tert-butyl-4-hydroxy-phényl)-acrylonitrile ;
(E)-2-(propane-1-sulfonyl)-3-(3,5-di-tert-butyl-4-hydroxy-phényl)-acrylonitrile ;
(E)-2-(éthane-1-sulfonyl)-3-(3,5-di-tert-butyl-4-hydroxy-phényl)-acrylonitrile ;
(E)-2-cyclopropylméthanesulfonyl-3-(3,5-di-tert-butyl-4-hydroxy-phényl)-acrylonitrile ;
(E)-2-cycloheptanesulfonyl-3-(3,5-di-tert-butyl-4-hydroxy-phényl)-acrylonitrile ;
(E)-2-cyclobutylméthanesulfonyl-3-(3,5-di-tert-butyl-4-hydroxy-phényl)-acrylonitrile ;
(E)-3-(3,5-di-tert-butyl-4-hydroxy-phényl)-2-(tricyclo[2.2.1.0^{2,6}]heptane-3-sulfonyl)-acrylonitrile ;
(E)-2-cyclooctanesulfonyl-3-(3,5-di-tert-butyl-4-hydroxy-phényl)-acrylonitrile ;
(E)-3-(3-tert-butyl-4-hydroxy-5-méthyl-phényl)-2-(4-trifluorométhoxy-benzènesulfonyl)-acrylonitrile ;
(E)-3-(3-tert-butyl-4-hydroxy-5-méthyl-phényl)-2-(3-trifluorométhoxy-benzènesulfonyl)-acrylonitrile ;
(E)-3-(3,5-di-tert-butyl-4-hydroxy-phényl)-2-(5-méthyl-hexane-1-sulfonyl)-acrylonitrile ;
(E)-2-(2-cyclohexyl-éthanesulfonyl)-3-(3,5-di-tert-butyl-4-hydroxy-phényl)-acrylonitrile ;
(E)-3-(3,5-di-tert-butyl-4-hydroxy-phényl)-2-(4-méthyl-pentane-1-sulfonyl)-acrylonitrile ;
(E)-3-(3-tert-butyl-4-hydroxy-5-méthyl-phényl)-2-(hexane-1-sulfonyl)-acrylonitrile ;
(E)-3-(4-hydroxy-3,5-diisopropyl-phényl)-2-(4-trifluorométhoxy-benzènesulfonyl)-acrylonitrile ;
(E)-3-(4-hydroxy-3,5-diisopropyl-phényl)-2-(3-trifluorométhoxy-benzènesulfonyl)-acrylonitrile ;
(E)-2-(4-chloro-benzènesulfonyl)-3-(4-hydroxy-3,5-diméthyl-phényl)-acrylonitrile ;
(E)-2-(4-chloro-benzènesulfonyl)-3-(4-hydroxy-3,5-dibromo-phényl)-acrylonitrile ;
(E)-2-(4-chloro-benzènesulfonyl)-3-(4-hydroxy-3,5-diméthoxy-phényl)-acrylonitrile ;
(E)-2-(4-chloro-benzènesulfonyl)-3-(4-hydroxy-3-iodo-5-méthoxy-phényl)-acrylonitrile ;
(E)-2-(hexane-1-sulfonyl)-3-(4-hydroxy-3,5-diisopropyl-phényl)-acrylonitrile ;
(E)-3-(3,5-di-tert-butyl-4-hydroxy-phényl)-2-(2-méthoxy-éthanesulfonyl)-acrylonitrile ;
(E)-3-(3,5-di-tert-butyl-4-hydroxy-phényl)-2-(pent-4-ène-1-sulfonyl)-acrylonitrile ;
(E)-3-(3,5-di-tert-butyl-4-hydroxy-phényl)-2-(pipéridine-1-sulfonyl)-acrylonitrile ;
(E)-3-(3,5-di-tert-butyl-4-hydroxy-phényl)-2-(azépane-1-sulfonyl)-acrylonitrile ;
(E)-3-(3,5-di-tert-butyl-4-hydroxy-phényl)-2-(pyrrolidine-1-sulfonyl)-acrylonitrile ; et
(E)-3-(3,5-di-tert-butyl-4-hydroxy-phényl)-2-(morpholine-4-sulfonyl)-acrylonitrile.

50. Utilisation d'un composé tel que défini dans l'une quelconque des revendications 1 à 49 dans la fabrication d'un médicament pour le traitement de l'obésité, du diabète de type 2, de la dyslipidémie, de l'hypertension ou des maladies de la vésicule biliaire ; la prévention de la prise de poids ; ou le maintien de la perte de poids.
